# EUROPEAN PATENT SPECIFICATION

(11) **EP 3 796 979 B1**
(45) Date of publication and mention of the grant of the patent: **04.06.2025**
(21) Application number: 19723328.1
(22) Date of filing: 23.04.2019
(51) Int. Cl.: A61K 31/713, A61P 25/00, A61P 27/00, A61P 21/00, A61P 19/00, A61K 45/06

(54) **MIR-181 INHIBITORS AND USES THEREOF**
MIR-181-INHIBITOREN UND VERWENDUNGEN DAVON
INHIBITEURS DE MIR-181 ET LEURS UTILISATIONS

(30) Priority: 20.04.2018 EP 18168602
(43) Date of publication of application: 31.03.2021
(73) Proprietor: Fondazione Telethon ETS, 00185 Roma (IT)
(72) Inventor: BANFI, Sandro, 80078 Pozzuoli (NA) (IT); FRANCO, Brunella, 80078 Pozzuoli (NA) (IT); INDRIERI, Alessia, 80078 Pozzuoli (NA) (IT); CARRELLA, Sabrina, 80078 Pozzuoli (NA) (IT)
(74) Representative: Manfredi, Irene
(86) International application number: PCT/EP2019/060348
(87) International publication number: WO 2019/202162

(56) References cited:
- WO-A1-2008/126693
- WO-A1-2013/055865
- SREENIVAS AVULA ET AL: "Treatment of Mitochondrial Disorders", CURRENT TREATMENT OPTIONS IN NEUROLOGY, vol. 16, no. 6, 4 April 2014 (2014-04-04), XP055145083, ISSN: 1092-8480, DOI: 10.1007/s11940-014-0292-7
- SABRINA CARRELLA ET AL: "miR-181a/b control the assembly of visual circuitry by regulating retinal axon specification and growth : miR-181a/b Control the Assembly of Visual Circuitry", DEVELOPMENTAL NEUROBIOLOGY, vol. 75, no. 11, 11 June 2015 (2015-06-11), pages 1252 - 1267, XP055600192, ISSN: 1932-8451, DOI: 10.1002/dneu.22282
- FILIPE DUARTE ET AL: "The Role of microRNAs in Mitochondria: Small Players Acting Wide", GENES, vol. 5, no. 4, 26 September 2014 (2014-09-26), pages 865 - 886, XP055600202, DOI: 10.3390/genes5040865
- ALESSIA INDRIERI ET AL: "miR-181a/b downregulation exerts a protective action on mitochondrial disease models", EMBO MOLECULAR MEDICINE (ONLINE), vol. 11, no. 5, 1 May 2019 (2019-05-01), DE, pages e8734, XP055600185, ISSN: 1757-4684, DOI: 10.15252/emmm.201708734

## Description

### TECHNICAL FIELD

The present invention relates to agents capable to inhibit miR-181, for use in treatment and/or prevention of mitochondrial disorders including mitochondrial diseases with eye and/or brain involvement and neurodegenerative conditions affecting eye and/or brain associated with mitochondrial dysfunction, as set out in the appended set of claims.

### BACKGROUND

Mitochondria are key players in different cellular processes, and their dysfunction contributes to the pathogenesis of a variety of human disorders, either directly, e.g., in the case of mitochondrial diseases (MD), or indirectly, e.g. in the case of common neurodegenerative disorders, including Parkinson's Disease (PD) or Alzheimer's Disease (AD) where mitochondrial dysfunctions have been described as a central causal factor in the pathogenesis (ref: PMID:23567191).

Primary Mitochondrial Diseases (PMDs) are a heterogeneous group of disorders caused by mutations in mitochondrial- or nuclear- encoded genes involved in oxidative phosphorylation (OXPHOS). The clinical manifestations of PMDs range from single tissue/organ lesions to syndromic multi-organ conditions with prominent involvement of the central nervous system (CNS), which is particularly sensitive to mitochondrial dysfunction. PMDs include: Leigh syndrome, Leber Hereditary Optic Neuropathy (LHON), Mitochondrial Myopathy, Encephalopathy, Lactic Acidosis and Stroke Syndrome (MELAS), Kjer's optic neuropathy, Neuropathy ataxia and retinitis pigmentosa (NARP), Autosomal dominant optic atrophy, Kearns-Sayre syndrome, Myoclonus Epilepsy with Ragged-Red Fibers (MERRF) syndrome. Secondary Mitochondrial Diseases (SMDs) are a group of disorders caused by mutations in nuclear genes directly affecting mitochondrial function, including Friedreich's ataxia, Hereditary Spastic paraplegia, Charcot-Marie- Tooth disease.

Although each individual disorder is rare, collectively the total prevalence of adult MDs is estimated to be of approximately 1 in 4300 representing one of the most prevalent groups of inherited neurological diseases (Gorman et al, 2015).

Neurodegenerative disorders affecting the central nervous system and/or the eye are often associated with mitochondrial dysfunction; said disorders may be of sporadic or familiar nature: e.g. sporadic age-related neurodegenerative diseases include Parkinson's Disease (PD), Alzheimer's Disease (AD), Age-related Macular degeneration (AMD), Diabetic Retinopathy, Glaucoma.

Familiar neurodegenerative diseases include familiar PD, AD, Huntington's disease, Retinitis Pigmentosa (RP), Stargardt's disease.

Mitochondrial diseases (both PMDs and SMDs) are biochemically and genetically heterogeneous and their complexity has so far prevented the development of effective treatments. For the majority of patients, therapy is limited to either preventing or treating complications, such as diabetes, cardiac involvement, and epilepsy. Available treatments are either supportive (not addressing the primary defects) or focused against specific aspects of mitochondrial dysfunction. Therefore, no effective therapy is available to date and treating patients with established mitochondrial disease remains a major challenge (Lightowlers et al, 2015; Viscomi et al, 2015). Recently, the specific modulation of either mitochondrial biogenesis/dynamics or mitochondrial clearance/quality control has been tested as possible therapeutic strategy both in *in vitro* and *in vivo* MD models (Cerutti et al, 2014; Civiletto et al, 2018; Civiletto et al, 2015; Johnson et al, 2013; Viscomi et al, 2011). In spite of some promising data, the latter approaches yielded discordant results and failed to be effective across different MD models [reviewed in (Lightowlers et al, 2015; Viscomi et al, 2015)]. Therefore, there is a need for more effective modulation of mitochondrial pathways as therapeutic strategies for MDs.

No effective therapies are available for neurodegenerative disorders such as PD or AD. AD is the most common cause of dementia, accounting for 60-80% of cases, whereas the prevalence of PD in industrialized countries is generally estimated at 1% in people over 60 years of age representing an important burden for health systems considering that to date treatment options are mostly symptomatic (Barnes & Yaffe, 2011; de Lau & Breteler, 2006).

A small molecule for the treatment of mitochondrial disorders, in particular LHON, is disclosed in Sreenivas Avula et al (Current Treatment Options in Neurology, vol. 16, no. 6, 2014).

MicroRNAs (miRNAs) are fundamental fine regulators of gene expression and represent promising therapeutic tools due to their capability of simultaneously modulating multiple molecular pathways involved in disease pathogenesis and progression. Modulation of miRNAs has been applied with therapeutic purposes to different disorders and has reached preclinical and clinical stages in specific instances (Broderick & Zamore, 2011; Christopher et al, 2016; Janssen et al, 2013; Ling et al, 2013). miRNAs play a key role in neuron survival and accumulating evidence indicates that alterations of miRNA-regulated gene networks increase the risk of neurodegenerative disorders (Hebert & De Strooper, 2009). The miR-181 family members are produced by three independent primary transcripts localized to three separate chromosomes. Each transcript produces two precursor sequences for a total of six precursor sequences. Each precursor produces two miRNA strands: the guide strand or 5p, and the passenger strand or 3p, for a total of ten mature miRNA forms: miR-181a-5p, miR-181a-1-3p, miR-181a-2-3p, miR-181b-5p, miR-181b-1-3p, miR-181b-2-3p miR-181c-5p, miR-181c-3p, miR-181d-5p and miR-181d-3p. The 5p mature sequences are those displaying the most abundant expression levels in mammalian tissues (Karali et al, 2016) and contain the same 5' "seed" sequence suggesting a significant degree of functional redundancy (Henao-Mejia et al, 2013; Ji et al, 2009). As used herein, the six mature 5p miRNAs will be defined as: miR-181a-1, miR-181a-2, miR-181b-1, miR-181b-2, miR-181c, and miR-181d. The mature forms of miR-181a-1 and miR-181a-2, as well as miR-181b-1 and miR-181b-2 are identical in sequence; miR-181c mature form has one nucleotide difference compared to miR-181a, whilst miR-181d mature form has one nucleotide difference compared to miR-181b. miR-181a and miR-181b (miR-181a/b) are highly conserved in vertebrates and highly expressed in different regions of the brain and retina (Boudreau et al, 2014; Karali et al, 2016) and were recently reported to target genes involved in mitochondrial-dependent cell death (Hutchison et al, 2013; Ouyang et al, 2012; Rodriguez-Blazquez et al, 2015) and autophagy (Cheng et al, 2016; He et al, 2013; Tekirdag et al, 2013).

WO 2013/055865 mentions miR-181a among numerous targets of antagomirs for the treatment of Amyotrophic lateral sclerosis (ALS), a disease involving motor neurons, not attributable to mitochondrial diseases with eye and/or brain involvement.

Filipe Duarte et al. (GENES, vol. 5, no. 4, 2014) propose miR-181a/c as a therapeutic target for cardiovascular diseases.

Sabrina Carrella et al. (DEVELOPMENTAL NEUROBIOLOGY, vol. 75, no. 11, 2015) show that knock-down of mir-181a and mir-181b using morpholino oligonucleotides, which however results in neuritogenesis defects in medaka fish.

### SUMMARY OF THE INVENTION

The present invention relates to miR-181 inhibitors for therapy and/or prevention of mitochondrial disorders including mitochondrial diseases with eye and/or brain involvement and neurodegeneration affecting eye and/or brain associated with mitochondrial dysfunction, as set out in the appended set of claims.

The present inventors have surprisingly found that miR-181, in particular miR-181 a and b are involved in the global regulation of mitochondrial function by controlling genes implicated in mitochondrial biogenesis, functionality, and antioxidant response in the CNS; furthermore, the inventors have found that miR-181a/b modulate the expression of mitochondrial-dependent programmed cell death and autophagy/mitophagy-related genes, simultaneously and finely modulating mitochondrial pathways in the CNS.

The inventors have surprisingly demonstrated that inhibition of miR-181 exerts a neuroprotective effect in MDs and in neurodegeneration associated to mitochondrial dysfunction, of particular high relevance especially for genetically heterogeneous conditions such as MDs, PD and RP for which a gene-independent therapeutic strategy is highly desirable. The present inventors have found that miR-181a/b modulate mitochondrial turnover via a fine coordination of biogenesis and clearance. The inventors have further found that inhibition of miR-181 exerts a beneficial effect on several pathologies characterized by mitochondrial dysfunction, particularly by protecting from mitochondrial mediated cell death and neurodegeneration in diseases such as PMDs including Leigh syndrome, Leber Hereditary optic Neuropathy (LHON), Mitochondrial Myopathy, Encephalopathy, Lactic Acidosis and Stroke Syndrome (MELAS), Myoclonic Epilepsy associated with Ragged-Red fibers (MERRF), Progressive External Ophthalmoplegia (PEO), Pearson's syndrome, Maternally inherited Myopathy and cardiomyopathy (MIMyCA), Kjer's optic neuropathy, Neuropathy ataxia and retinitis pigmentosa (NARP), Autosomal dominant optic atrophy, Kearns-Sayre syndrome, Myoclonus Epilepsy with Ragged-Red Fibers (MERRF) syndrome; SMDs including Friedreich's ataxia, Hereditary Spastic paraplegia, Charcot-Marie- Tooth disease; Neurodegenerative disorders affecting the central nervous system and/or the eye associated with mitochondrial dysfunction: including sporadic age-related neurodegenerative diseases, e.g. Parkinson's Disease (PD), Alzheimer's Disease (AD), Age-related Macular degeneration (AMD), Diabetic Retinopathy, Glaucoma; familiar neurodegenerative diseases including familiar PD, AD, Huntington's disease, Retinitis Pigmentosa (RP), Stargardt's disease.

Then, the present invention provides an inhibitor of miR-181 for use in the treatment and/or prevention of a mitochondrial disorder, selected from the group consisting of: Leigh syndrome, Leber Hereditary optic Neuropathy (LHON), Mitochondrial Myopathy, Encephalopathy, Lactic Acidosis and Stroke Syndrome (MELAS), Myoclonic Epilepsy associated with Ragged-Red fibers (MERRF), Progressive External Ophthalmoplegia (PEO), Pearson's syndrome, Maternally inherited Myopathy and cardiomyopathy (MIMyCA), Kjer's optic neuropathy, Neuropathy ataxia and retinitis pigmentosa (NARP), Autosomal dominant optic atrophy, Kearns-Sayre syndrome, Myoclonus Epilepsy with Ragged-Red Fibers (MERRF) syndrome, Friedreich's ataxia, Hereditary Spastic paraplegia, Charcot-Marie- Tooth disease, sporadic age-related neurodegenerative disease, Parkinson's Disease (PD), Alzheimer's Disease (AD), Age-related Macular degeneration (AMD), Diabetic Retinopathy, Glaucoma, familiar neurodegenerative disease, familiar PD, AD, Huntington's disease, Retinitis Pigmentosa (RP), Stargardt's disease, said inhibitor of miR-181 being an inhibitory nucleic acid comprising at least one sequence complementary to miR-181, wherein said inhibitor comprises a sequence complementary to ACAUUCA (SEQ ID NO. 5).

In the present invention a mitochondrial disorder and a mitochondrial disease is used equally. According to this aspect of the invention, a treatment and/or prevention of a mitochondrial disorder can be effective to mitigate at least one symptom of said disorder.

When treating the underlying cause of the disorder, it is believed that management of symptoms can likewise be achieved. By management of symptoms, it is intended that the severity of symptoms can be maintained (i.e., worsening or advancement of symptoms is controlled) or, more preferably, the severity of symptoms can be reduced either in whole or in part.

As used herein, "treatment" (and grammatical variations thereof such as "treat" or "treating") or "prevention" (and grammatical variations thereof such as "prevent" or "preventing") refers to clinical intervention in an attempt to alter the natural course of the individual being treated, and can be performed either for prophylaxis or during the course of clinical pathology or disorder. Desirable effects of treatment include, but are not limited to, preventing occurrence or recurrence of disease or disorder, alleviation of symptoms, diminishment of any direct or indirect pathological consequences of the disease or disorder, preventing relapse, decreasing the rate of disease/disorder progression, amelioration or palliation of the disease/disorder state, and remission or improved prognosis. In some embodiments, the inhibitor of the invention is used to delay development of a disease or disorder or to slow the progression of a disease or disorder.

Any references to methods of treatment by therapy or surgery or *in vivo* diagnosis methods in this description are to be interpreted as references to compounds, pharmaceutical compositions and medicaments of the present invention for use in those methods.

Said inhibitor is an inhibitory nucleic acid agent.

Further inhibitors, not falling within the subject matter for which protection is sought, are a small molecule or a genome editing agent. An editing agent is an agent which can mediate targeted gene disruption or repair, wherein said agent is an engineered nuclease, for instance a Zn Finger nuclease, a transcription activator-like effector nuclease, or a clustered regularly interspaced short palindromic repeats system. Preferably the inhibitory nucleic acid agent is selected from the group consisting of: siRNA, an antisense oligonucleotide (e.g. an antagomir, an LNA), a miRNA sponge, and/or a ribozyme as defined herein.

The inhibitor of miR-181 comprises at least one sequence complementary to miR-181. The inhibitor of miR-181 comprises at least one sequence complementary to ACAUUCA (SEQ ID NO. 5). Preferably said inhibitor reduces the function and/or activity of miR-181.

The function and activity of miR-181 may be measured by any known method in the art, in particular as described herein.

More preferably said inhibitor has at least one property selected from the group consisting of: inhibiting the miRNA activity, preferably inhibiting mRNA target binding, increasing mRNA levels of at least one specific miR-181 target, ameliorating mitochondrial function, increasing the number of mitochondria, reducing oxidative stress and/or cell death, ameliorating cell survival. The above properties may be measured by any known method in the art, in particular as described herein.

Preferably said miR-181 is a miR-181 precursor or a mature miR-181, preferably said miR-181 is selected from the group consisting of miR-181a-1, miR-181a-2, miR-181b-1, miR-181b-2, miR-181c, and miR-181d or a combination thereof.

Preferably the inhibitor of miR-181 comprises a sequence complementary to ACAUUCA (SEQ ID NO. 5) and has at least 80% sequence identity with SEQ ID No. 1 or SEQ ID No. 2 or SEQ ID No. 3 or SEQ ID No. 4. Preferably it has at least 85 %, 90 %, 95 %, 99 % identity with SEQ ID No. 1 or SEQ ID No. 2 or SEQ ID No. 3 or SEQ ID No. 4.

Still preferably said inhibitor is from 18 to 21 nucleotide long.

In a preferred embodiment said inhibitor of miR is a miRNA sponge with the formula:

(X1sX2sX3)n₁S2(X1sX2sX3)n₂

wherein
X1, X2 and X3 are independently chosen from
   - an MBS sequence complementary to miR-181a and/or miR-181c, preferably modified from nucleotide 9 to nucleotide 12 to create a bulge;
   - an MBS sequence complementary to miR-181b and/or miR-181d, preferably modified from nucleotide 9 to nucleotide 12 to create a bulge;
X3 may be present or absent;
s is a Spacer sequence of 4 to 6 nucleotides, preferably 4 nucleotides;
S2 is a central Spacer sequence of 4 to 6 nucleotides, preferably 6 nucleotides, preferably corresponding to a restriction enzyme site, preferably a site for the Xbal restriction enzyme;
n₁ and n₂ is a number independently selected from 3, 6 and 24, preferably between 3, 6
and 12, most preferably n is 3, n₁ and n₂ may be identical or different.

In a preferred embodiment said miRNA sponge has the formula:

(AsB)n₁S2(AsB)n₂

wherein:
A is an MBS sequence complementary to miR-181a and/or miR-181c, preferably modified from nucleotide 9 to nucleotide 12 to create a bulge;
B is an MBS sequence complementary to miR-181b and/or miR-181d, preferably modified from nucleotide 9 to nucleotide 12 to create a bulge;
s is a Spacer sequence of 4 to 6 nucleotides, preferably 4 nucleotides;
S2 is a central Spacer sequence of 4 to 6 nucleotides, preferably 6 nucleotides, preferably corresponding to a site for a restriction enzyme, preferably the Xba I restriction enzyme n₁ and n₂ is a number independently selected from 3,6 and 24, preferably between 3, 6 and 12, most preferably n is 3, n₁ and n₂ may be identical or different.

Preferably X1, X2, X3, A is 3' TTGTAAGTn1n2n3n2ACAGCCACTCA 5' (SEQ ID NO. 113)
wherein according to IUPAC nomenclature:

| | |
|---|---|
| n1= | A or C or G |
| n2= | A or C or T |
| n3= | A or G or T |

or wherein X1, X2, X3, B is 3' TTGTAAGTn'1n'1n'2n'3ACAGCCACCCA 5' (SEQ ID NO. 114)
wherein according to IUPAC nomenclature

| | |
|---|---|
| n'1= | C or G or T |
| n'2= | A or G or T |
| n'3= | A or C or T; |

or wherein n₁ = 3 and n₂ = 3;
or wherein s consists of 4 nucleotides, preferably s= tagc;
or wherein S2 consists of 6 nucleotides, preferably S2 = Tctaga.

Preferably the sequence of the inhibitor further comprises one or more locked nucleic acid (LNA) nucleotides and one or more non-locked nucleotides, wherein at least one of the non-locked nucleotides comprises a chemical modification, preferably the locked nucleic acid (LNA) nucleotides has a 2' to 4' methylene bridge, preferably the chemical modification is a 2' O-alkyl or 2' halo modification.

Still preferably said inhibitor of miR-181 is used with at least one additional agent.

More preferably said at least one additional agent is an inhibitor of miR-181 as defined above and in the present invention.

Then the use refers to a combination of one or more inhibitor of miR-181, i.e one or more siRNA, an antisense oligonucleotide (e.g. an antagomir, an LNA), a miRNA sponge, and/or a ribozyme that can be the same or different.

In a preferred embodiment said at least one additional agent is selected from the group consisting of an agent that improves mitochondrial function, selected from: an agent that increases mitochondrial biogenesis (i.e. an agent that activates a pathway controlling mitochondrial biogenesis, leading to increased oxidative phosphorylation and/or improvement in the defective activities of the respiratory chain complexes, for instance a NAD+ precursor (e.g. among others, a nicotinamide riboside), an AMPK agonist (e.g. AICAR), a Poly ADP ribose polymerase (PARP) inhibitor (e.g. among others, Olaparib (AZD-2281)), Resveratrol, metformin, retinoic acid, an agent that modulates autophagy, for instance an activator of autophagy, e.g. a mTOR inhibitor (for instance, rapamycin ), an agent that inhibits apoptosis, A ROS scavenger , e.g. antioxidant compounds, including lipoic acid, vitamins C and E, and CoQ, an agent that shifts heteroplasmy, a scavenger of toxic compounds , N-acetylcysteine (NAC), metronidazole, a supplementation of nucleotides, a permeability transition pore inhibitor, e.g. cyclosporin A or a derivative thereof, an agent that replaces the missing factor, e.g. an enzyme replacement therapy wherein the enzyme that is deficient or absent in the body is replaced, or gene therapy e.g. the therapeutic delivery of nucleic acid into a patient's cells as a drug to treat a disease.

Preferred additional agents of the invention are agents for treating a disease of the invention, for instance agents for treatment of LHON disease: Idebenone, a synthetic analogue of ubiquinone, acts by improving mitochondrial oxidative metabolism in the brain and reducing oxidative stress (Lyseng-Williamson, 2016; Sanchez et al, 2016). Hydroxocobalamin, a synthetic, injectable form of Vitamin B12, a recombinant AAV vector serotype 2 containing the human wild-type mitochondrial ND4 gene, a recombinant AAV vector containing the human wild-type mitochondrial ND1 gene, elamipretide, a mitochondrial membrane permeability transition pore opening blocker.

Preferably said inhibitor of miR-181 and said at least one additional agent are administered sequentially or simultaneously.

The present invention also provides a pharmaceutical composition comprising the inhibitor of miR-181 as defined in any one of previous embodiments, or a pharmaceutically acceptable salt thereof, and a pharmaceutically acceptable carrier or diluent for use in the treatment and/or prevention of a mitochondrial disorder.

Preferably the composition further comprises at least one additional agent, preferably said at least one additional agent is an inhibitor of miR-181 as defined above, preferably said at least one additional agent is selected from the group consisting of: an agent that increases mitochondrial biogenesis, an agent that modulates autophagy, an agent that inhibits apoptosis, a ROS scavenger, an agent that shifts heteroplasmy, a scavenger of toxic compounds, a supplementation of nucleotides, an agent that replaces the missing gene.

The present disclosure further provides a method of treating a mitochondrial disorder in a subject in need thereof, comprising administering to the subject at least an inhibitor of miR-181 as defined above or the pharmaceutical composition as defined above.

The invention also provides a vector comprising at least one inhibitor of miR-181 as defined above and a promoter sequence, preferably the vector is a viral vector, preferably and adeno-associated viral vector for use in the treatment and/or prevention of a mitochondrial disorder. Preferably the mitochondrial disease is a primary or a secondary mitochondrial disease.

Preferably the mitochondrial disease is selected from the group consisting of: a mitochondrial disease with eye and/or brain involvement, a neurodegeneration affecting eye and/or brain said neurodegeneration being associated with mitochondrial dysfunction.

The mitochondrial disease is selected from the group consisting of: Leigh syndrome, Leber Hereditary optic Neuropathy (LHON), Mitochondrial Myopathy, Encephalopathy, Lactic Acidosis and Stroke Syndrome (MELAS), Myoclonic Epilepsy associated with Ragged-Red fibers (MERRF), Progressive External Ophthalmoplegia (PEO), Pearson's syndrome, Maternally inherited Myopathy and cardiomyopathy (MIMyCA), Kjer's optic neuropathy, Neuropathy ataxia and retinitis pigmentosa (NARP), Autosomal dominant optic atrophy, Kearns-Sayre syndrome, Myoclonus Epilepsy with Ragged-Red Fibers (MERRF) syndrome, Friedreich's ataxia, Hereditary Spastic paraplegia, Charcot-Marie- Tooth disease, sporadic age-related neurodegenerative disease, Parkinson's Disease (PD), Alzheimer's Disease (AD), Age-related Macular degeneration (AMD), Diabetic Retinopathy, Glaucoma, familiar neurodegenerative disease, familiar PD, AD, Huntington's disease, Retinitis Pigmentosa (RP), Stargardt's disease.

The invention will be described by means of non-limiting examples in reference to the following figures.

### Brief Description of the Drawings

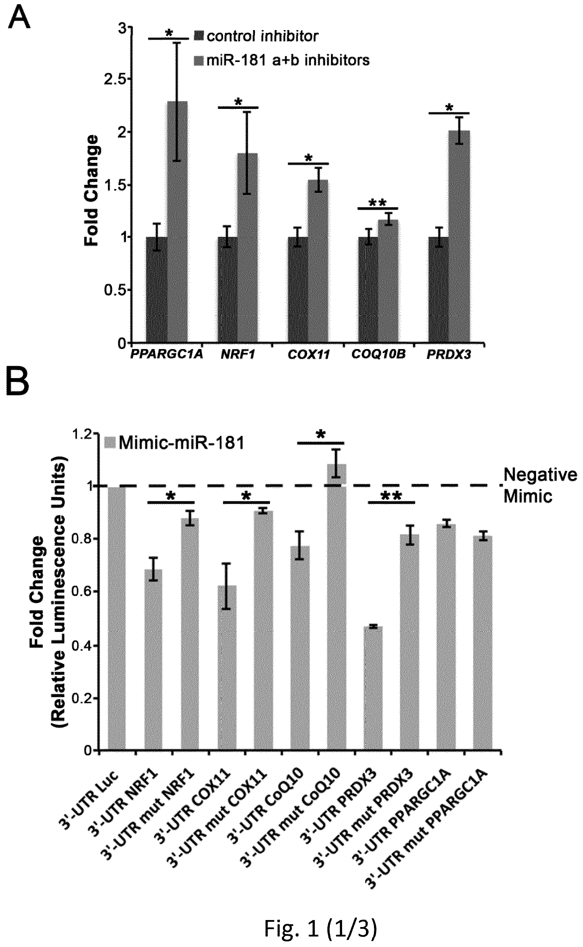

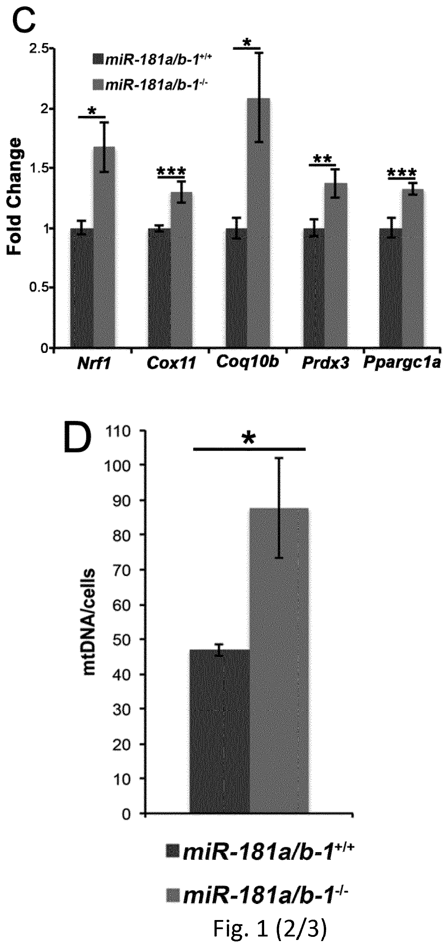

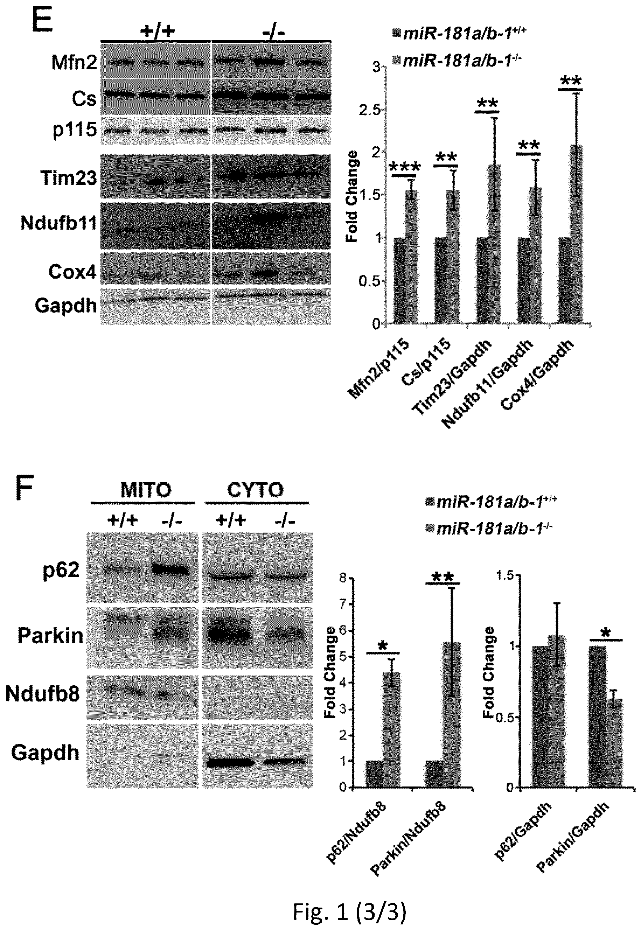

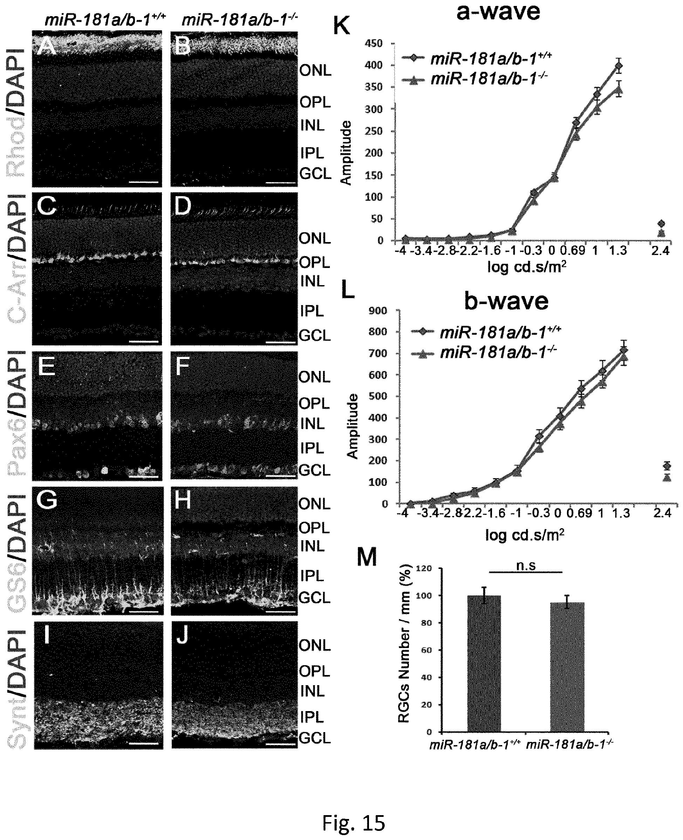

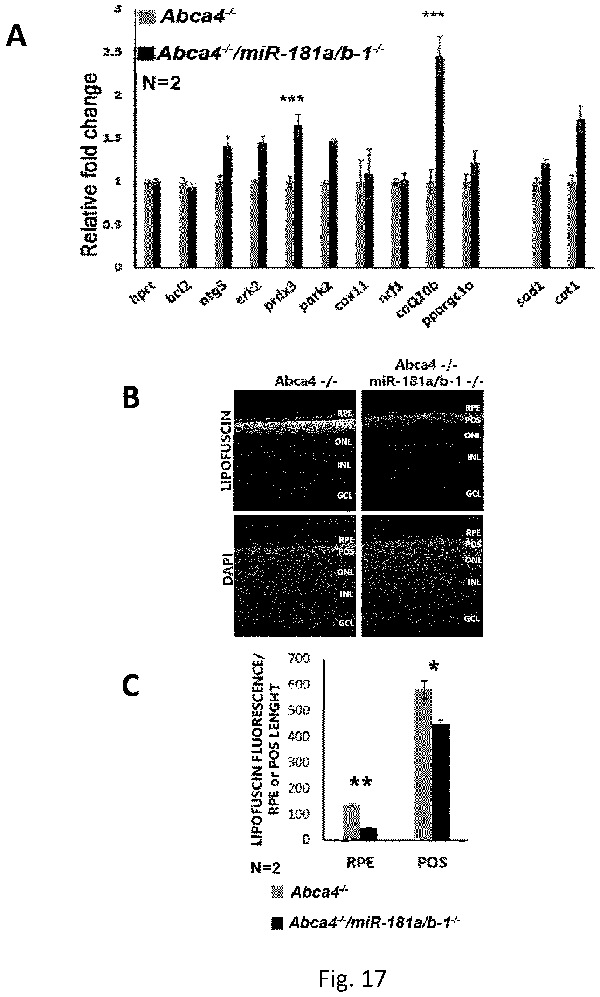

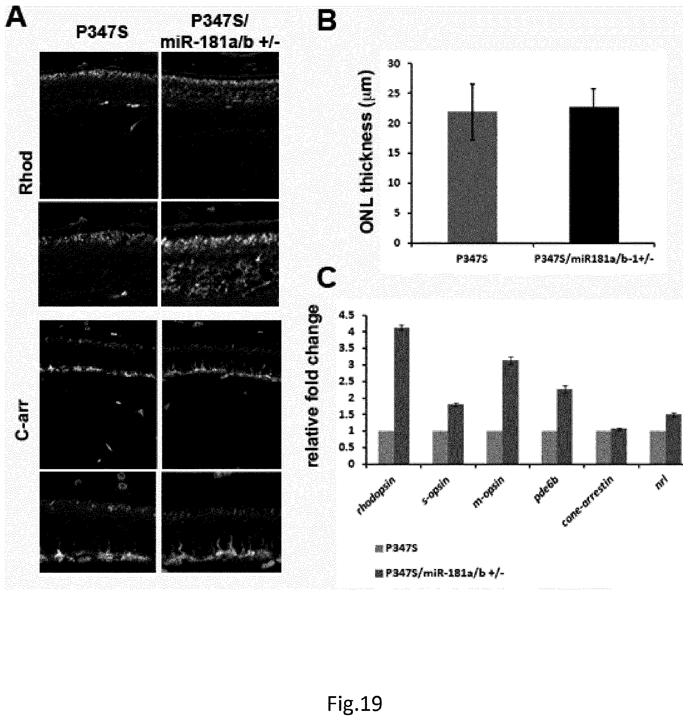

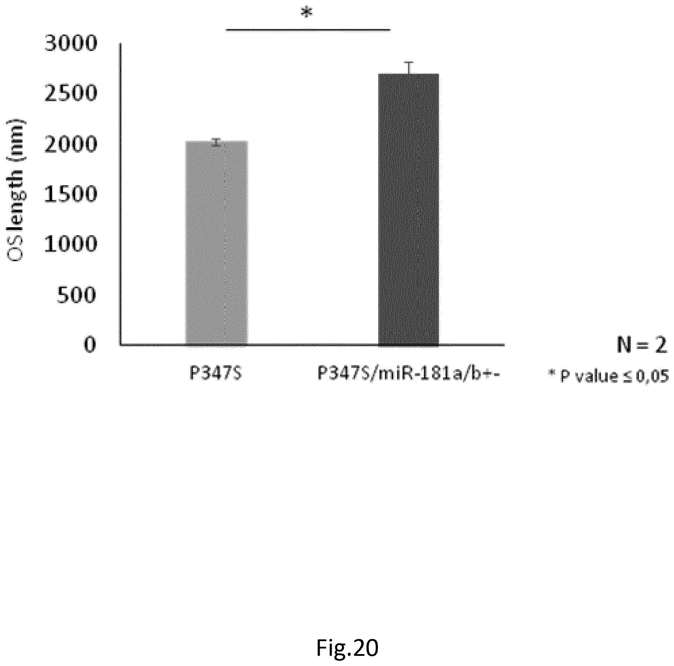

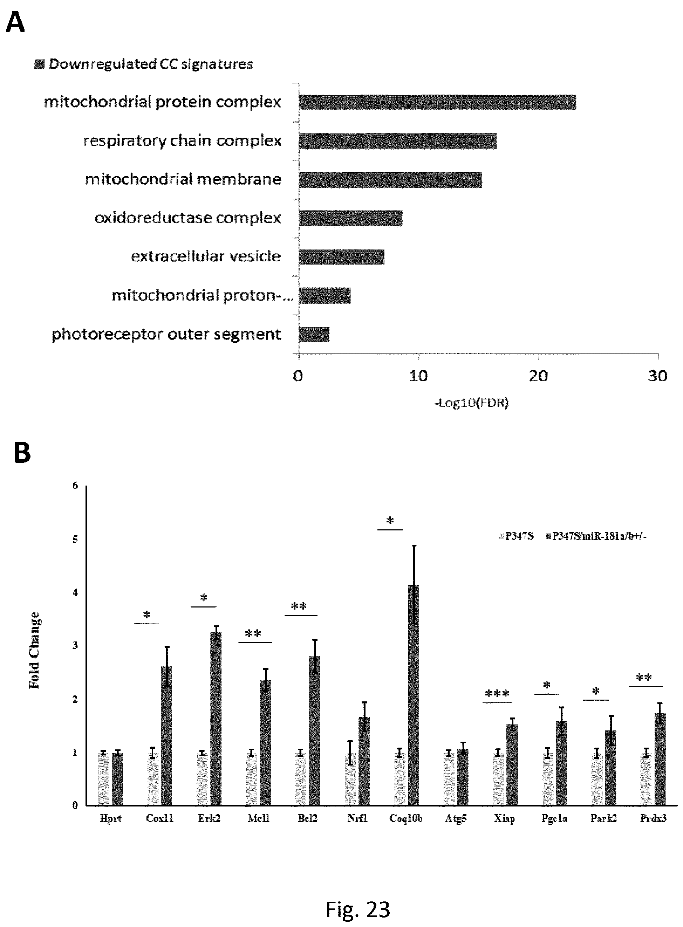

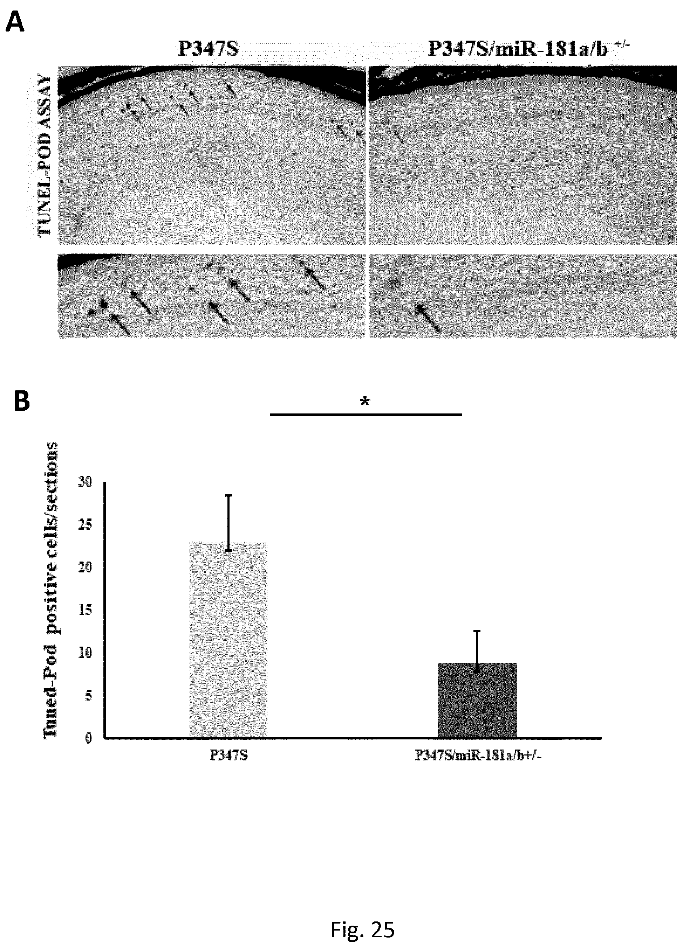

**Figure 1****. miR-181a/b modulates mitochondrial biogenesis and mitophagy.**
   **A)** qRT-PCR confirms that miR-181a/b silencing leads to upregulation of miR-181a/b predicted targets in SH-SY5Y cells. N=3 independent experiments.
   **B)** miR-181-mimic transfection specifically inhibits Luciferase activity of constructs containing WT 3'-UTR predicted target sequences. Point mutations (mut) in miR-181a/b binding sites abolish Luciferase repression. Data are normalized to negative mimic transfection (dashed line). N=6 independent experiments.
   **C)** qRT-PCR reveals upregulation of miR-181a/b targets in the eyes of *miR-181a*/*b-1*^{*-*/*-*} vs. *miR-181a*/*b-1*^{*+*/*+*} animals. n≥5 animals/genotype.
   ***D)*** *miR-181a*/*b-1*^{*-*/*-*} mice show increased mtDNA content vs. *miR-181a*/*b-1*^{*+*/*+*} mice. N=4 animals/genotype.
   **E)** WB analysis (left panel) reveals increased levels (quantified in the right panel) of mitochondrial proteins in the eyes of *miR-181a*/*b-1*^{*-*/*-*} (-/-) vs. *miR-181a*/*b-1*^{*+*/*+*} (+/+) mice. Data are normalized to either p115 or Gapdh. N=3 animals/genotype. Please note that all compared bands from +/+ and -/- samples are from the same blots which were cropped and shown split for the sake of clarity of data presentation.
   **F)** WB analysis on mitochondrial and cytosolic fractions (left panel) show increased levels of p62 and Parkin (quantified in the right panel) in mitochondrial fraction from the eye of -/- vs. +/+ mice. Data are normalized to Ndufb8 or Gapdh for mitochondrial and cytosolic fractions, respectively. N=3 animals/genotype.
   *p<0.05, **p<0.01, ***p<0.001 one-tailed (in **A-C** and in **E)** and two-tailed Student's t-test (in **D).**

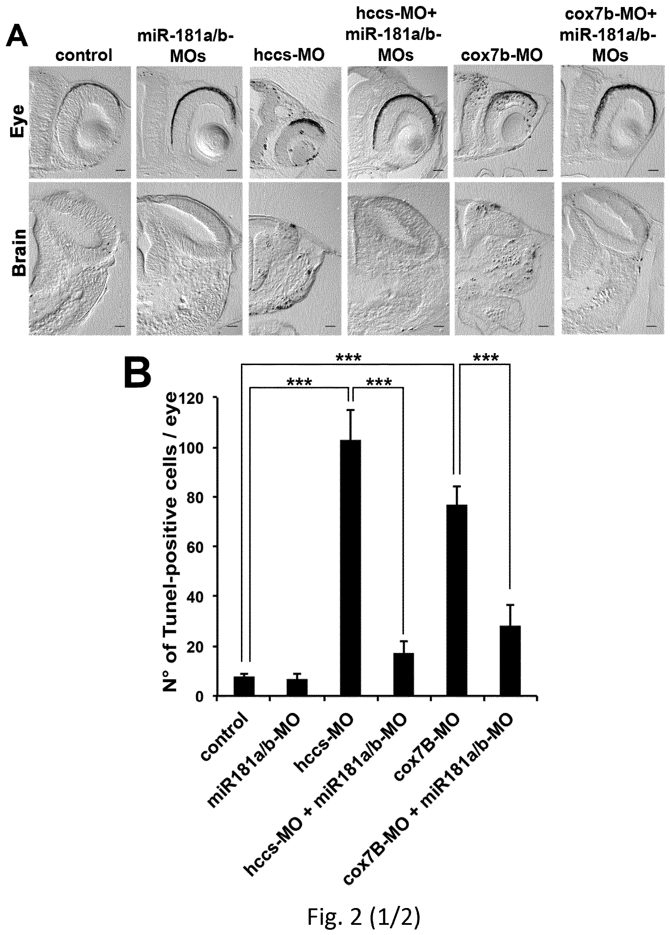

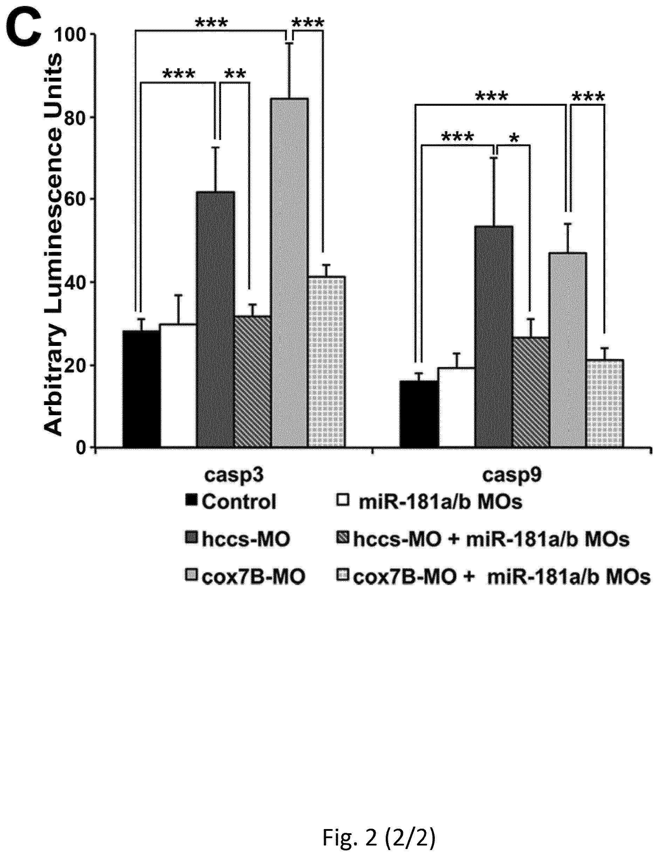

**Figure 2****. miR-181a/b inhibition counteracts cell death in Microphthalmia with Linear Skin defects (MLS) medakafish models.**
   **A)** TUNEL assays on retinal and brain sections of st30 medakafish control and MO-injected embryos reveal a decrease in the number of apoptotic cells in both h*ccs*-MO/miR-181a/b-MO-and *cox7b*-MO/miR-181a/b-MO-injected compared to *hccs-MO-* and cox7b-MO-injected embryos respectively. Scale bars are 20µm.
   **B)** TUNEL-positive cells/eye. n≥5 retinae for each model.
   **C)** Caspase assays show restored levels of caspase-3 and caspase-9 activities in hccs-MO/miR-181a/b-MO- and *cox7b*-MO/miR-181a/b-MO-injected embryos compared to *hccs-MO-* and cox7b-MO-injected embryos respectively. n≥5 embryos for each model. *p<0.05, **p<0.01, ***p<0.001. Analysis of Deviance for Negative Binomial Generalized Linear Model (in **B);** One-way ANOVA with post-hoc Tukey analysis (in C); Error bars are SEM.

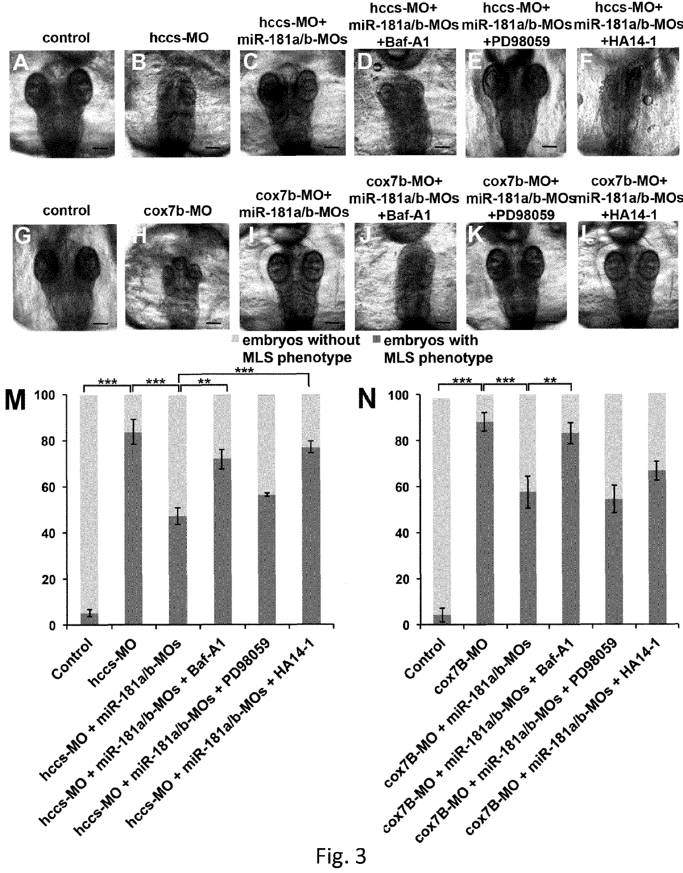

**Figure 3****. miR-181a/b downregulation ameliorates the phenotype of MLS medakafish models.**
   **(A-I)** Representative images of st30 medaka embryos injected with *hccs-MO* **(B)** and *cox7b-MO* **(H)** alone or co-injected with miR-181a/b-MOs **(C, I)**. Co-injection of miR-181a/b-MOs rescues microphthalmia and microcephaly in both *hccs-MO* and *cox7b-MO* embryos.
   **(D-F, J-L)** *hccs*-MO/miR-181a/b-MO- and *cox7b*-MO/miR-181a/b-MO-injected embryos were treated with Baf-A1, PD98059 or HA14-1. Baf-A1 treatment counteracts the protective effect of miR-181a/b downregulation in both *hccs*-MO/miR-181a/b-MO and *cox7b*-MO/miR-181a/b-MO embryos **(D, J).** PD98059 treatment does not interfere with the modulation of the MLS phenotype mediated by miR-181a/b downregulation **(E, K).** HA14-1 treatment counteracts the effect of miR-181a/b downregulation in the *hccs*-MO/miR-181a/b-MO model but not in the *cox7b*-MO/miR-181a/b-MO embryos **(F, L).** Scale bars are 100µm.
   **(M-N)** Percentage of embryos with or without MLS phenotype in the different conditions illustrated in (A-L) in *hccs-MO* (M) and *cox7b-MO* (N) embryos. Light gray: embryos without MLS phenotype, Dark gray: embryos with MLS phenotype. n≥300 embryos/conditions, **p<0,01; ***p<0,001, Analysis of Deviance for Generalized Linear Model; Error bars are SEM.

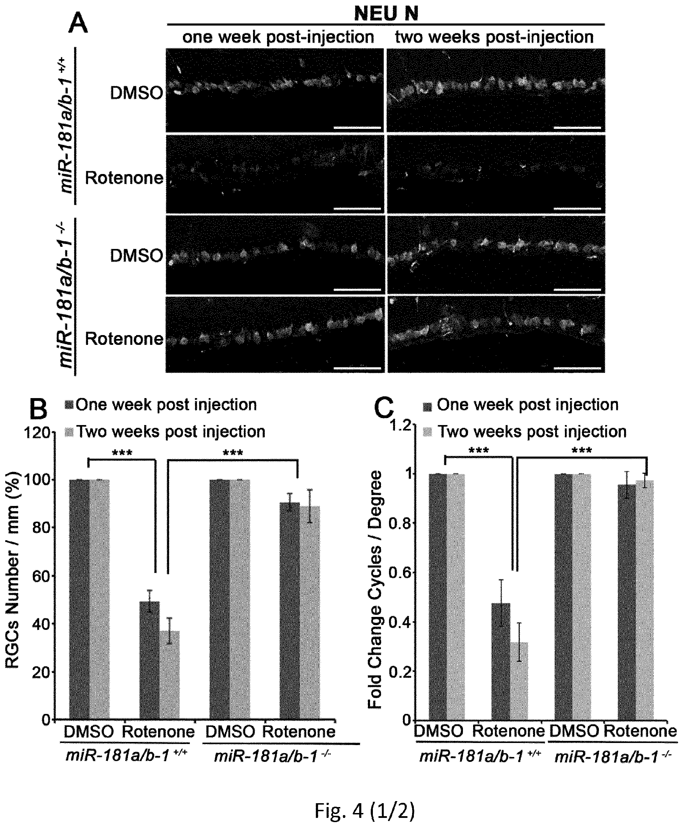

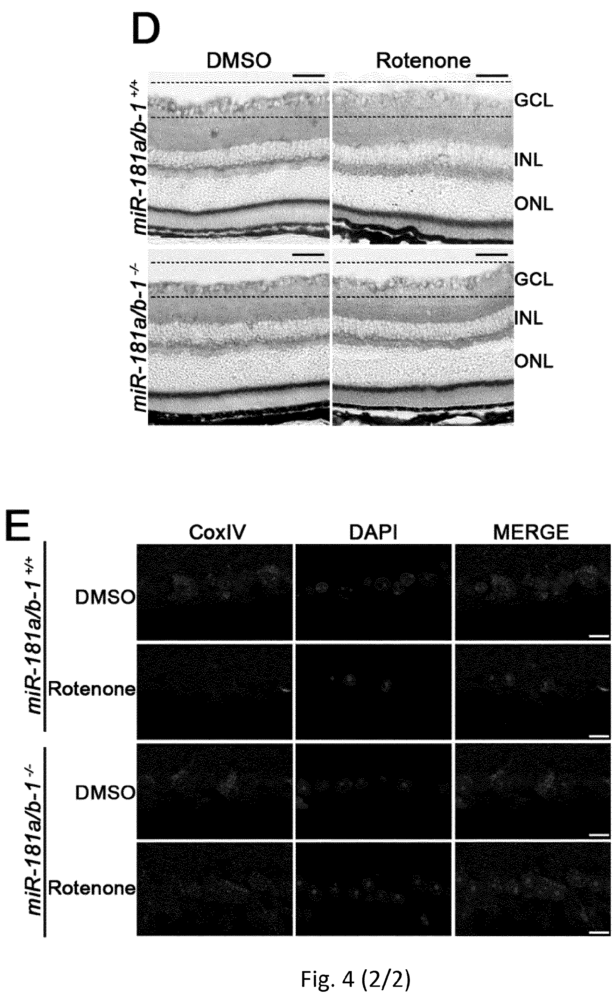

**Figure 4****. Inactivation of *miR-181a*/*b-1* protects RGCs from cell death in a rotenone-induced mouse model of LHON syndrome.**
   **A)** Immunofluorescence analysis with anti-NeuN antibody in the retina of *miR-181a*/*b-1*^{*+*/*+*} and *miR-181a*/*b-1*^{*-*/*-*} mice intravitreally injected with Rotenone or DMSO. RGCs are preserved in *miR-181a*/*b-1*^{*-*/*-*} rotenone-injected mice compared to controls at both one and two weeks after injection. Scale bars are 50µm.
   **B)** Number of RGCs/mm (indicated as %) at one and two weeks post-injection. N=10.
   **C)** Graphical representation of the results of the optokinetic tracking assays reported as fold change of cycles/degree. Visual acuity is preserved in *miR-181a*/*b-1*^{*-*/*-*} rotenone-injected mice with respect to controls at both one and two weeks after injection. N=10.
      *** p<0,001 One-way ANOVA with post-hoc Tukey analysis (in B, C); Error bars are SEM.
   **D)** NADH dehydrogenase histochemical reaction on retinal sections of *miR-181a*/*b-1*^{*+*/*+*} and *miR-181a*/*b-1*^{*-*/*-*} mice intravitreally injected with Rotenone or DMSO. At one week post-injection, NADH dehydrogenase activity is lost in RGCs (GCL, areas within dashed lines) of *miR-181a*/*b-1*^{*+*/*+*} Rotenone-injected eyes, while it is preserved in those of *miR-181a*/*b-1*^{*-*/*-*} Rotenone-injected eyes. GCL, Ganglion Cells Layer ONL; INL, Inner Nuclear Layer; Outer Nuclear Layer. Scale bars are 50µm.
   **E)** Immunofluorescence analysis, with an anti-CoxIV antibody, in the retina of *miR-181a*/*b-1*^{*+*/*+*} and *miR-181a*/*b-1*^{*-*/*-*} mice, injected with either Rotenone or DMSO, showed preserved mitochondria in *miR-181a*/*b-1*^{*-*/*-*} rotenone-injected eyes. Scale bars are 10µm.

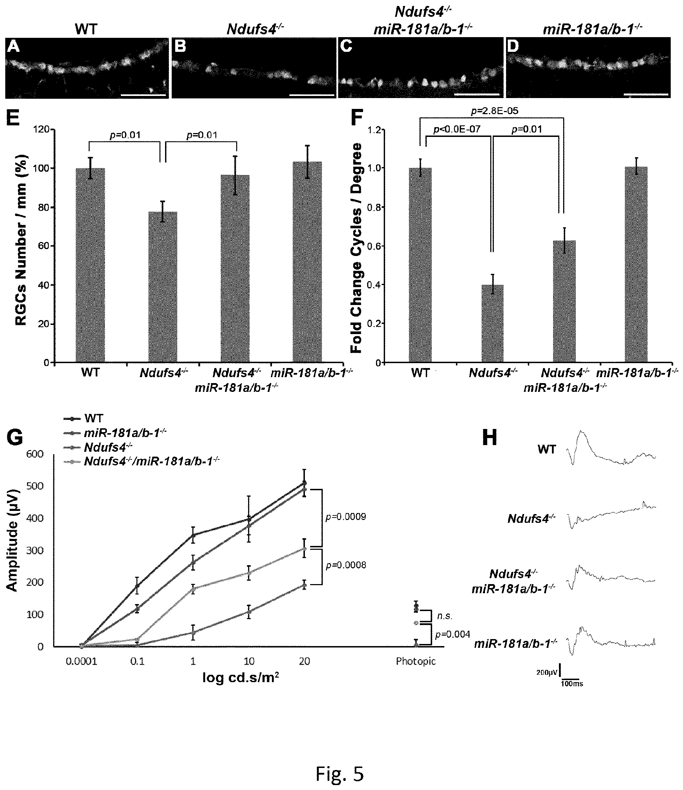

**Figure 5****. miR-181a/b depletion protects RGCs and ameliorates visual function in *Ndufs4*^{*-*/*-*}mice.**
   **A-D)** Immunofluorescence analysis with anti-NeuN antibody in the retina of *WT, Ndufs4*^{*-*/}*⁻, Ndufs4*^{*-*/*-*}/*miR-181a*/*b-1*^{*-*/*-*} and *miR-181a*/*b-1*^{*-*/*-*} mice. RGCs are preserved in *Ndufs4*^{*-*/*-*}/*miR-181a*/*b-1*^{*-*/*-*} with respect to *Ndufs4*^{*-*/*-*} mice. Scale bars are 50µm. **E)** Number of RGCs/mm (%). N=5. **F)** Graphical representation of the results of the optokinetic tracking assays reported as fold change of cycles/degree. Visual acuity is preserved in *Ndufs4*^{*-*/*-*}/*miR-181a*/*b-1*^{*-*/*-*} with respect to *Ndufs4*^{*-*/*-*} mice. N≥8. **G, H)** Electroretinographic analysis reveals amelioration of b-wave patterns in *Ndufs4*^{*-*/*-*}/*miR-181a*/*b-1*^{*-*/*-*} with respect to *Ndufs4*^{*-*/*-*}mice*.* N≥5.
   p-values were calculated by one-way ANOVA with post-hoc Tukey analysis in **E-F,** and by Two-ANOVA Repetaed Measures with post-hoc analysis in **G.** p-values in **G** refers to 20 log cd.s/m² and Photopic points. p-values for the other points are reported in the Appendix Table S1; Error bars are SEM.

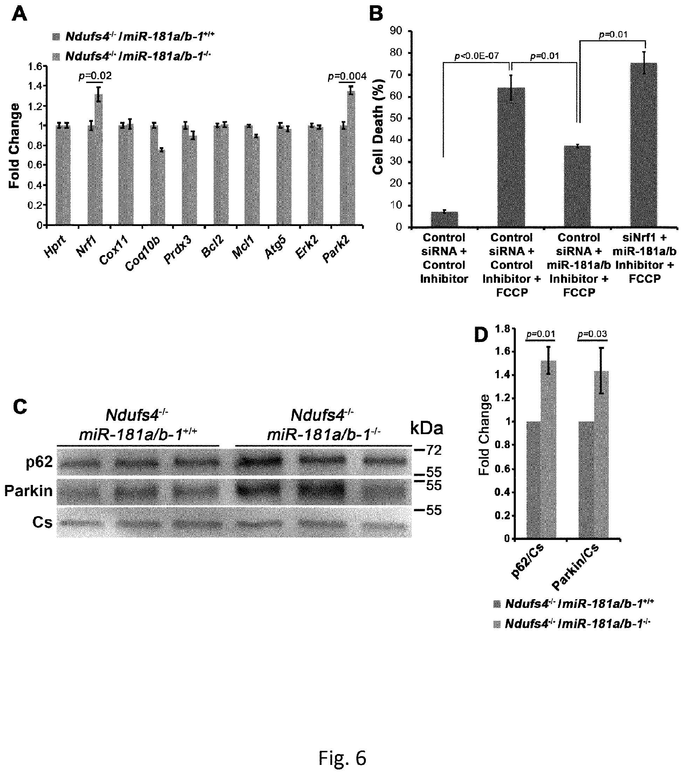

**Figure 6****. miR-181a/b depletion increases *Nrf1* and *Park2* transcript levels and enhances mitophagy in *Ndufs4*^{*-*/*-*} retina.**
   **A)** q-RT-PCR reveals upregulation of the miR-181a/b targets *Nrf1* and *Park2* in the eyes of *Ndufs4*^{*-*/*-*}/*miR-181a*/*b-1^{-l-}* vs. *Ndufs4*^{*-*/*-*} animals. N=4 animals/genotype. **B)** Cell death analysis shows that *NRF1* downregulation abolish the miR-181a/b-mediated protection in SH-SY5Y cells treated with FCCP. N=4. **C)** WB analysis on mitochondrial fractions shows enhanced increase levels of p62 and Parkin (quantified in **D)** in mitochondrial fraction from the eye of *Ndufs4*^{*-*/*-*}/*miR-181a*/*b-1^{-l-}* vs. *Ndufs4*^{*-*/*-*} mice. Data are normalized to Cs. N=3 animals/genotype. p-values were calculated by one-way ANOVA with post-hoc analysis in **B** and one-tailed Student's t-test in **D.** Error bars are SEM.

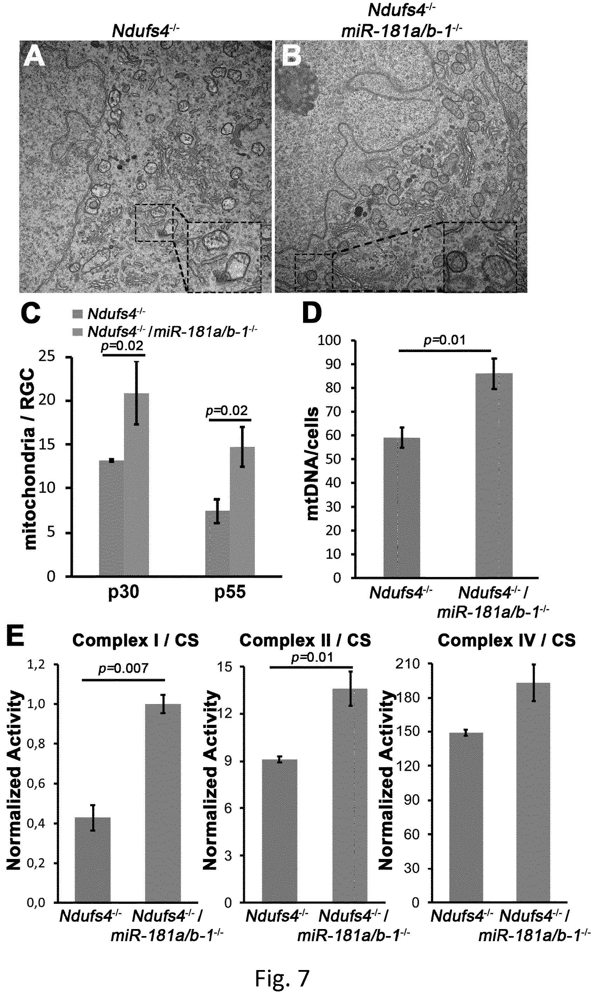

**Figure 7****. miR-181a/b depletion enhances mitochondria biogenesis *Ndufs4*^{*-*/*-*} mice.**
   **A-C)** Electron microscopy analysis shows amelioration of mitochondria morphology and increase of mitochondrial number in *Ndufs4*^{*-*/*-*}/*miR-181a*/*b-1^{-l-}* vs. *Ndufs4⁻¹⁻* mice. Scale bars are 1µm. The quantitative increase of mitochondria is reported in C as number of mitochondria/RGC, at p30 and p55. N≥2 animals/genotype. **D)** *Ndufs4*^{*-*/*-*}/*miR-181a*/*b-1^{-l-}* mice show increased mtDNA content vs. *Ndufs4*^{*-*/*-*} mice as measured by q-PCR. N≥4 animals/genotype. **E)** Biochemical activity of MRC complexes I, II and IV normalized by the percentage of citrate synthase (CS) activity in *Ndufs4*^{*-*/*-*}/*miR-181a*/*b-1^{-l-}* vs. *Ndufs4*^{*-*/*-*} mice. N≥3 animals/genotype. p-values were calculated by two tailed Student's t-test; Error bars are SEM.

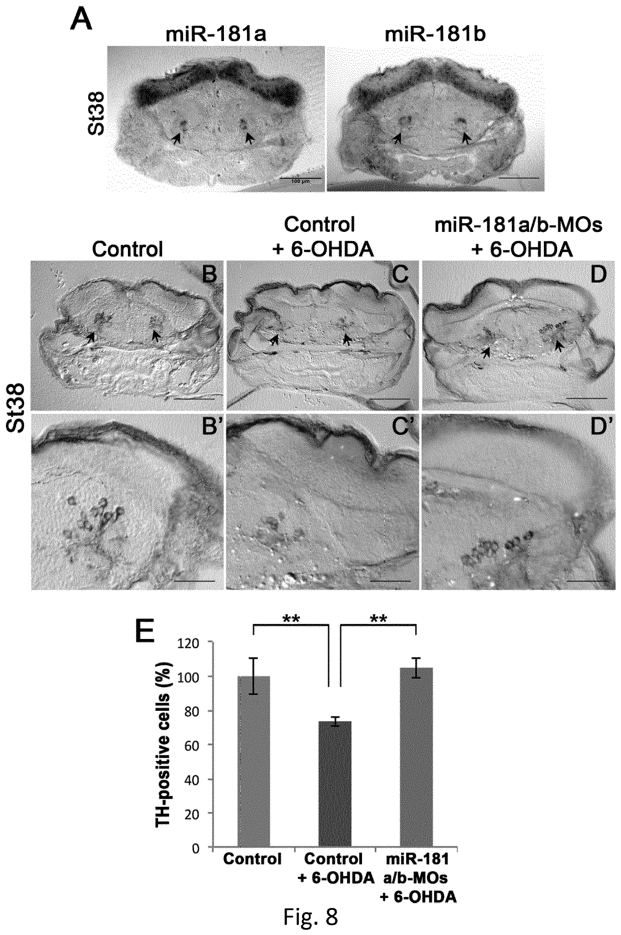

**Figure 8****. miR-181a/b inhibition protects DA neurons against 6-OHDA-induced cell death in medakafish.**
   **A)** RNA ISH in medaka brain shows strong expression of miR-181a and miR-181b in DA neurons localized in the *locus coeruleus* (arrows). Scale bars are 100µm.
   **(B-D)** anti-TH immunohistochemistry staining on the brain of st38 controls (DMSO-treated) (B), of 6-OHDA-treated controls (C) and of 6-OHDA-treated miR-181a/b-MOs-injected (D) medakafish to visualize DA neurons. 6-OHDA treatment leads to a reduction of TH-positive cells (arrows) in controls but not in miR-181a/b-injected fish. Scale bars are 100µm.
   **(B'-D')** Higher magnifications of B-D. Scale bars are 40µm.
   **E)** Number of TH-positive *cells*/*locus coeruleus* (indicated as %). N=6, **p<0.01 One-way ANOVA with post-hoc Tukey analysis; Error bars are SEM.

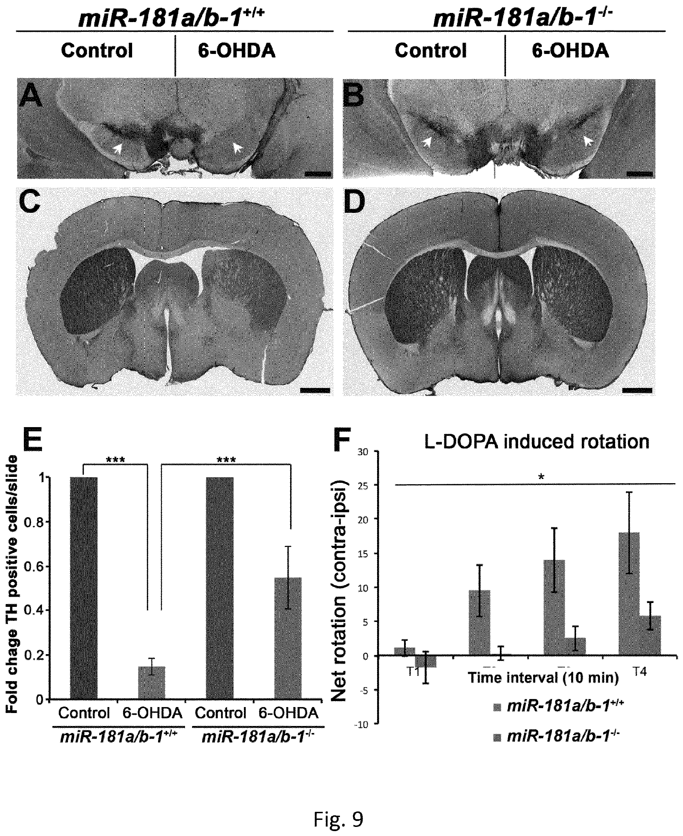

**Figure 9****. Inactivation of *miR-181a*/*b-1* ameliorates the phenotype of a 6-OHDA-induced PD mouse model.**
   **(A-C)** anti-TH immunohistochemistry staining on *miR-181a*/*b-1*^{*+*/*+*} and *miR-181a*/*b-1*^{*-*/*-*} mouse brains unilaterally injected with 6-OHDA in the substantia nigra (SN).
   **(A, B)** 6-OHDA injection leads to TH-positive cells reduction in the SN with respect to the control saline-injected side in *miR-181a*/*b-1*^{*+*/*+*} mice **(A),** whereas in *miR-181a*/*b-1*^{*-*/*-*} animals, DA neurons (arrows) are preserved from 6-OHDA-induced death. **(B).**
   **(C, D)** Decrease of TH staining in striatal sections of *miR-181a*/*b-1*^{*+*/*+*} mice in the 6-OHDA-injected side with respect to the control saline-injected side **(C),** as opposed to no differences between the 6-OHDA-injected side and the control side in *miR-181a*/*b-1*^{*-*/*-*} mice **(D).** Scale bars are 1mm.
   **E)** Number of TH-positive cells/section in the SN reported as fold change, with the control hemisphere corresponding to 1 for each mouse genotype. N=6.
   **F)** L-DOPA-induced rotations test measures behavioral and motor defects in *miR-181a*/*b-1*^{*+*/}*⁺ 6-*OHDA-injected mice. *miR-181a*/*b-1*^{*-*/*-*} 6-OHDA-injected are less sensitive to the rotation-induced effects of L-DOPA. Net contralateral rotations (contralateral-ipsilateral rotations) were analyzed for each time intervals (T1=0-600s, T2=600-1200s, T3=1200-1800s, T4=1800-2400s). N=8. *p<0.05, *** p<0.001 One-way ANOVA with post-hoc Tukey analysis in E, genotype effect two-way ANOVA for repeated measures in F; Error bars are SEM.

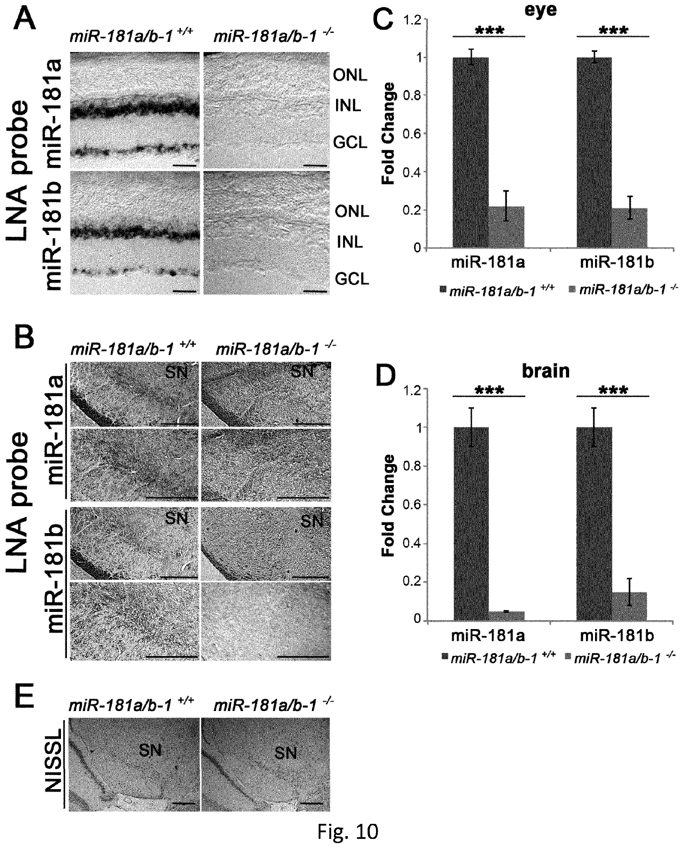

**Figure 10****. The miR-181a/b-1 cluster accounts for most of miR-181a and miR-181b expression in mouse brain and eye.**
   **A, B)** RNA in-situ hybridization with miR-181a and miR-181b probes in retina (A) and substantia nigra (SN) (B) sections of miR-181a/b-1+/+ and miR-181a/b-1^{-/-} mice. Scale bars are 50µm in (A) and 1mm in (B).
   **C, D)** Expression analysis of miR-181a and miR-181b in miR-181a/b-1+/+ and miR-181a/b-1-/- eyes and brain by Taqman assays. Targeted deletion of the miR-181a/b cluster-1 leads to a striking reduction of the expression levels of the mature forms of miR-181a and miR-181b in both tissues. n=3 *** p<0.005 one-tailed Student's t-test; Error bars are SEM.
   **E)** NISSL staining of brain sections from miR-181a/b-1+/+ and miR-181a/b-1-/- mice shows no obvious morphological differences in miR-181a/b-1-/- mice compared to controls. Scale bars are 1mm. ONL, Outer Nuclear Layer; INL, Inner Nuclear Layer; GCL, Ganglion Cells Layer.

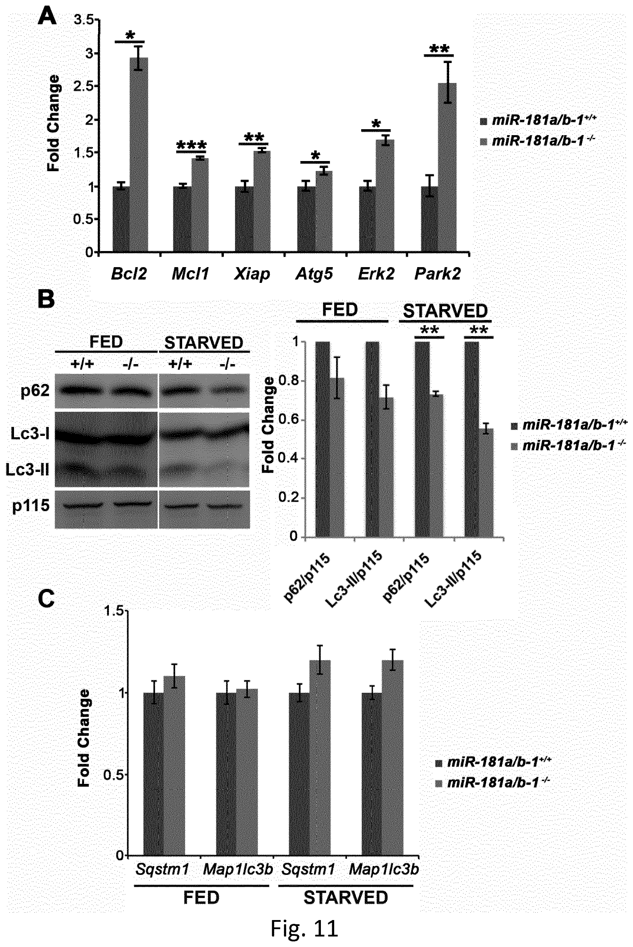

**Figure 11****. miR-181a/b-1 deletion leads to increased autophagic flux.**
   **A)** qRT-PCR analysis reveals increased levels of miR-181a/b targets Bcl2, Mcl1, Xiap, Atg5, Erk2 and Park2 in the eyes of miR-181a/b-1-/- vs. miR-181a/b-1+/+ animals. n≥5 animals/genotype.
   **B)** WB analysis (left panel) of Lc3-I/Lc3-II and p62 on protein extracts from eyes of animal in fed and starved conditions reveals decreased levels (quantified in the right panel) of both proteins in miR-181a/b-1-/- (-/-) vs. miR-181a/b-1+/+ (+/+) mice. n=2 mice for each genotype and condition. Error bars are SEM.
   **C)** qRT-PCR analysis reveals no changes of the Sqstm1 (p62) and Map1lc3b (LC3) transcript levels between miR- 181a/b-1-/- and miR-181a/b-1+/+ mouse eyes in both fed and starved conditions. These data indicate that the decreased levels of the autophagy markers Lc3-II and p62 observed by WB analysis are due to increased autophagic flux in miR-181a/b-1-/- eyes. n=3 for each genotype and condition. **p<0.01 ***p<0.001 one-tailed Student's t-test.
**Figure 12****. miR-181a/b silencing protects SH-SY5Y cells from FCCP-induced cell death.**
   Cell death analysis shows that miR-181a/b silencing (100nM) protects SH-SY5Y cells from FCCP treatment. n=3 independent experiments. **p<0.01, two-tailed Student's t-test. Error bars are SEM.

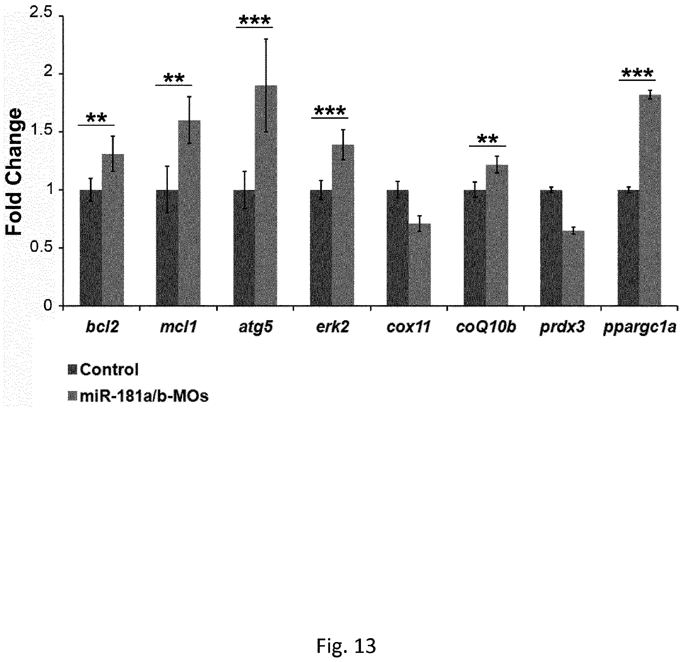

**Figure 13****. miR-181a/b knockdown in medaka leads to upregulation of genes involved in mitochondrial function and mitophagy.**
   qRT-PCR carried out on total RNA extracted from whole miR-181a/b-MOs medaka embryos to analyze the transcript levels of the miR-181a/b targets involved in mitochondrial-dependent cell death (bcl2, mcl1), mitophagy (atg5, erk2), and mitochondrial biogenesis and function (cox11, coq10b, prdx3) and of the indirect target ppargc1a. n=3 **<0.05; ***<0.005 one-tailed Student's t-test; Error bars are SEM.

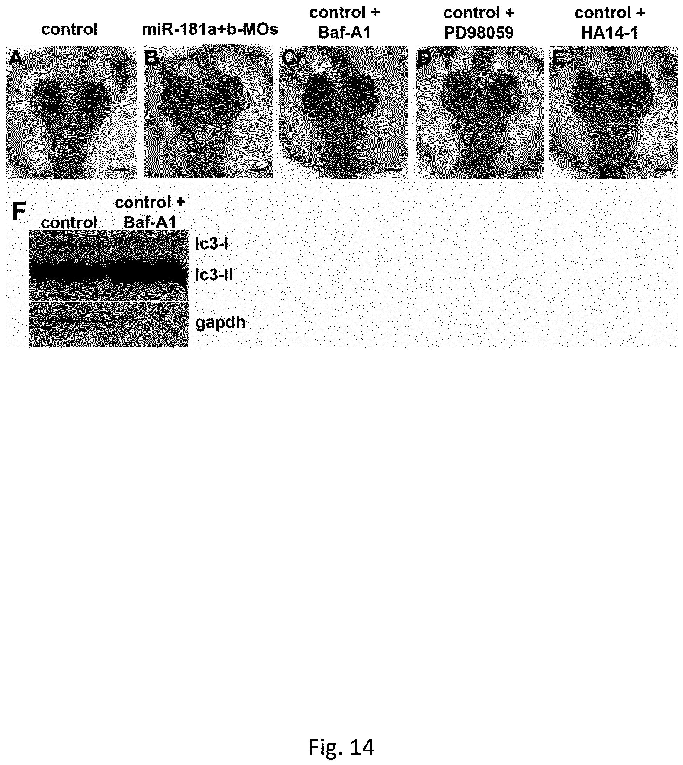

**Figure 14****. Absence of abnormal phenotypes following MO-mediated silencing of miR-181a/b and drug administration.**
   **(A, B)** Medaka embryos injected with miR-181a/b-MOs only (B) show no phenotypic differences compared to control embryos (A).
   **(C-E)** Bafilomycin A (Baf-A1) (C), PD98059 (D) and HA14-1 (E) do not induce any morphological alterations in control embryos at the concentrations used in this study. Scale bars are 100µm.
   **(F)** The concentration of Bafilomycin A used for the study blocks autophagy as demonstrated by Ic3-I/II Wb analysis
**Figure 15****. miR-181a/b-1 deletion does not cause significant alterations of retina morphology and function in mouse.**
   **(A-J)** Immunofluorescence staining of different retinal markers [(A, B) Rhodopsin (Rhod) for rods; (C, D) Cone-arrestin (C-Arr) for cones; (E, F) Pax6 for RGCs and amacrine cells; (G, H) GS6 for Muller cells; (I, J) Synthaxin (Synt) for synaptic structures in the inner plexiform layer] reveals no abnormalities in cell layer organization in the retina of miR-181a/b-1-/- with respect to control mice. Scale bars are 50µm.
   **K, L)** Electroretinographic analysis reveals no difference in a-wave (K) and b-wave (L) patterns between miR-181a/b-1+/+ and miR-181a/b-1-/- mice, indicating that miR-181a/b ablation does not cause retinal dysfunction. n=10 for each genotype.
   **M)** Graphical representation of the number of RGCs/mm (indicated as %) shows no significant differences between miR-181a/b-1+/+ and miR-181a/b-1-/- mice. n=10 for each genotype. ONL, Outer Nuclear Layer; OPL, Outer Plexiform Layer; INL, Inner Nuclear Layer; IPL, Inner Plexiform Layer; GCL, Ganglion Cells Layer.

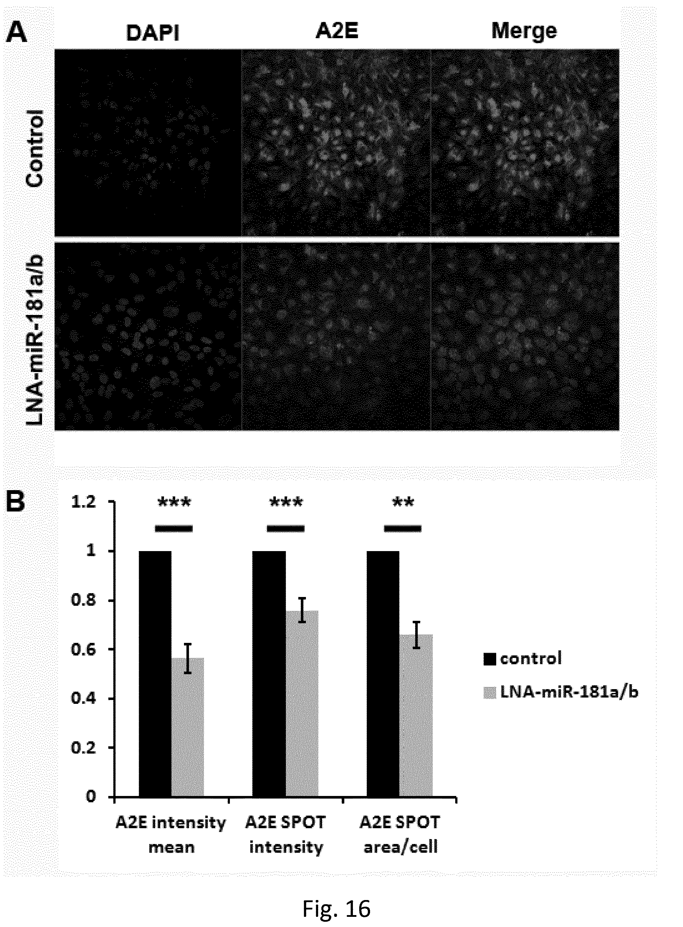

**Figure 16****. miR-181a/b inhibition reduces accumulation of lipofuscin (A2E) in an *in vitro* model of AMD.**
   **A)** Confocal images of ARPE-19 cells transfected with control LNA and LNA-miR-181a/b and then treated with A2E. Autofluorescence analysis indicate the amount of A2E accumulation and indicate that miR-181a/b inhibition reduces A2E accumulation compared to control cells. **B)** Graphical representation of quantification of A2E autofluorescence intensity, A2E SPOT intensity and A2E SPOT area per cell in LNA-miR-181a/b transfected cells compared to control cells reported as fold change. Student t test p value ***<0.001, **<0.005
**Figure 17****. miR-181a/b depletion reduces accumulation of lipofuscin (A2E) in *Abca4*^{*-*/*-*} mouse.**
   **A)** qRT-PCR analysis of miR-181a/b targets, *sod1* and *cat1* transcripts in *Abca4*^{*-*/*-*}/*miR-181a*/*b-*1^{-/-} RPE. **B)** Representative images of Lipofuscin accumulation in RPE and POS in *Abca4*^{*-*/*-*} and *Abca4*^{*-*/*-*}/*miR-181a*/*b-1*^{*-*/*-*} retina sections. **C)** Lipofuscin autofluorescence quantification in RPE and POS. Error bars are SEM. *p<0.05, **p<0.01, ***p<0.001.

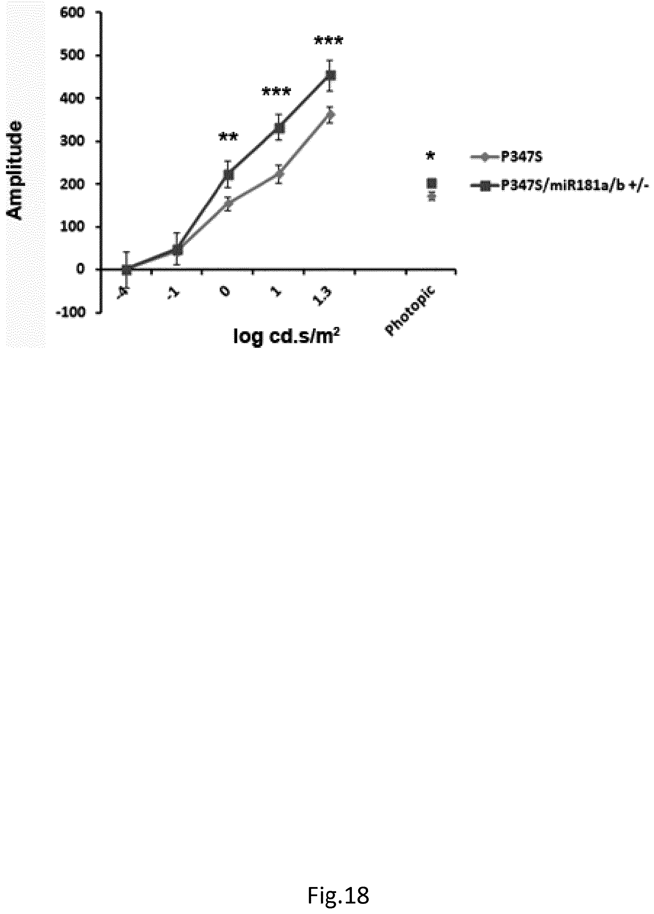

**Figure 18****. miR-181a/b reduction ameliorates visual functionality in a mouse model of Retinitis Pigmentosa.**
   ERG responses from P347S and P347S/miR-181a/b^{+/-} mice at p30 highlight an amelioration in miR-181a/b heterozygously depleted animals. N=16, ** p<0,01; ***p<0,001. [Constant luminance (cd·s/m2), Photopic= 2.4 log cd·s/m2]
**Figure 19****. miR-181a/b reduction ameliorates photoreceptor marker expression in** a **mouse model of Retinitis Pigmentosa.**
   **A)** Immunofluorescence images of P347S and and P347S/miR-181a/b^{+/-} retinas at p30 stained with two different retinal markers (green), Rhodopsin and Cone-arrestin, and DAPI (blue). **B)** ONL thickness analysis at p30 between P347S and P347S/miR-181a/b^{+/-}. **C)** qRT-PCR analysis reveals increased levels of the main PRs marker *(Rhodopsin, S-opsin, M-opsin, Pde6b,* and *Cone Arrestin)* in P347S/miR-181a/b^{+/-} respect to P347S mice retina at p30. N=3; error bars are SEM
**Figure 20****. miR-181a/b reduction ameliorates photoreceptor Outer Segment length.** P347S/miR-181a/b^{+/-} photoreceptors showed an in increase in Outer Segment (OS) length respect to P347S animals. * p<0.05, Student's t-test. Error bars are SEM

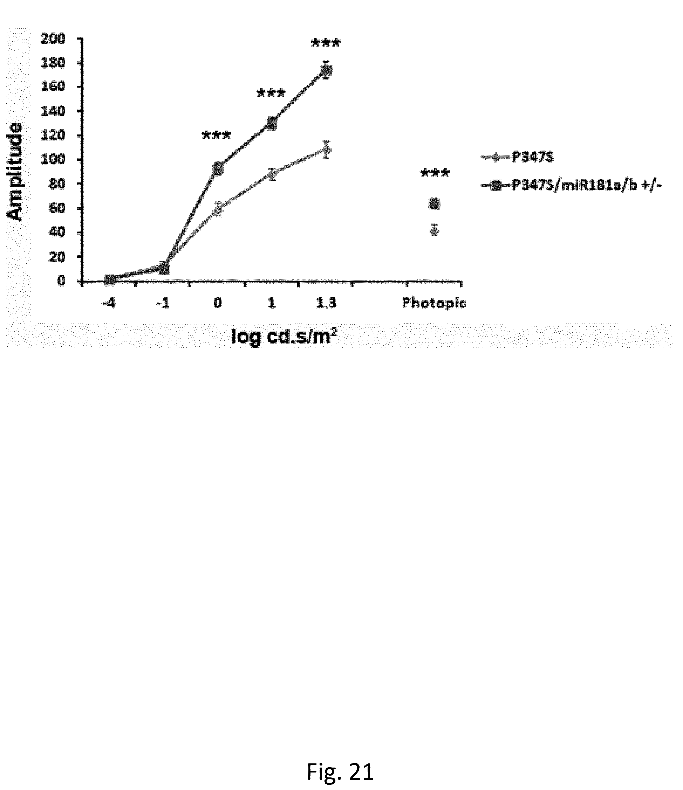

**Figure 21****. miR-181a/b reduction ameliorates visual functionality in a mouse model of Retinitis Pigmentosa at late time point.**
   ERG responses from P347s and P347S/miR-181a/b^{+/-} mice at p90 highlight an amelioration in miR-181a/b heterozygously depleted animals. N=24, ***p<0,001.

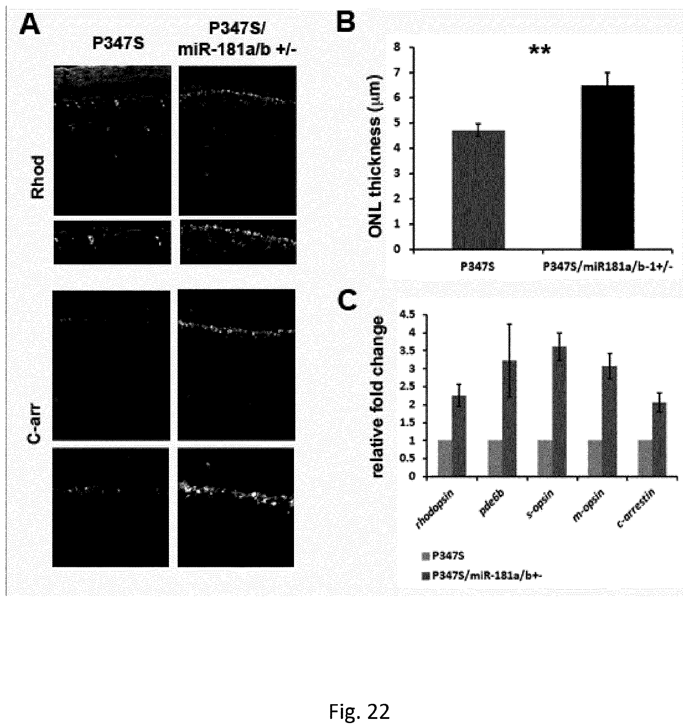

**Figure 22****. miR-181a/b reduction ameliorates photoreceptor marker expression and survival in a mouse model of Retintis Pigmentosa.**
   **A)** Immunofluorescence images of P347S and and P347S/miR-181a/b^{+/-} retinas at p90 stained with two different retinal markers (green), Rhodopsin and Cone-arrestin, and DAPI (blue). **B)** ONL thickness analysis at p90 between P347S and P347S/miR-181a/b⁺⁻ , highlight a significant increase of ONL thickness. **C)** qRT-PCR analysis reveals increased levels of the main PRs marker *(Rhodopsin, S-opsin, M-opsin, Pde6b,* and *Cone Arrestin)* in P347S/miR-181a/b^{+/-} compared to P347S mice retina at p30. N=3; error bars are SEM
**Figure 23. A****)** Downregulated terms from transcriptomic analysis of P347S vs. WT eyes. **B)** qRT-PCR analysis of several miR-181a/b targets (Hprt, Cox11, Erk2, Bcl2, Nrf1, Coq10b, Atg5, Xiap, Pgc1a, Park2, Prdx3) in P347S/181a/b^{+/-} eye compared to the P347S at p30; * p<0.05;** p<0.01, one tailed Student's t-test. Error bars are SEM.

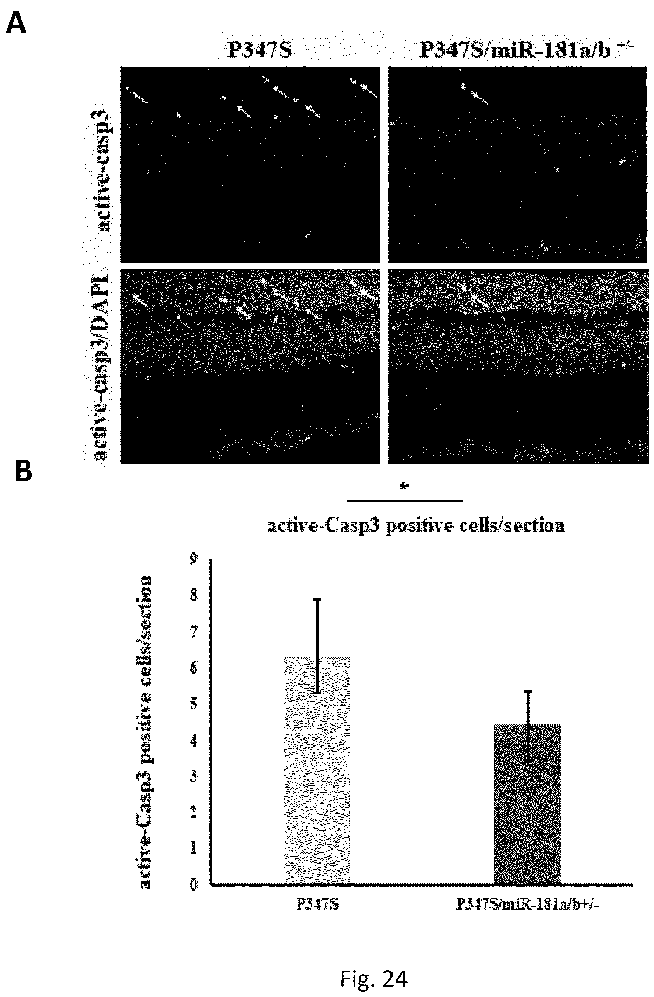

**Figure 24****. miR-181a/b reduction decreases activated Caspase 3 in P347S retina.**
   **A)** Immunofluorescence images on frozen retinal sections from P347S and P347S/miR-181a/b^{+/-}at p30, stained with an anti-active-Caspase-3 antibody(green) and DAPI (blue). The assay reveals a lower number of apoptotic cells in P347S/miR-181a/b^{+/-} vs P347S retinas. **B)** Count of anti-active-Caspase-3-positive cells performed manually. N = 5; * p < 0.05, Student's t-test. Error bars are SEM
**Figure 25****. miR-181a/b reduction decreases TUNEL staining in P347S retina. A)** TUNEL-POD staining of P347S and P347S/miR-181a/b^{+/-} of frozen retinas at p30. Brown spots indicate DNA double-strand damage or single-strand-damage. The assay reveals a reduced number of apoptotic cells in P347S/miR-181a/b^{+/-} with respect to P347S/miR-181a/b^{+/+} mice. **B)** Count of TUNEL-POD- positive cells performed manually. N = 3; * p < 0.05, Student's t-test. Error bars are SEM

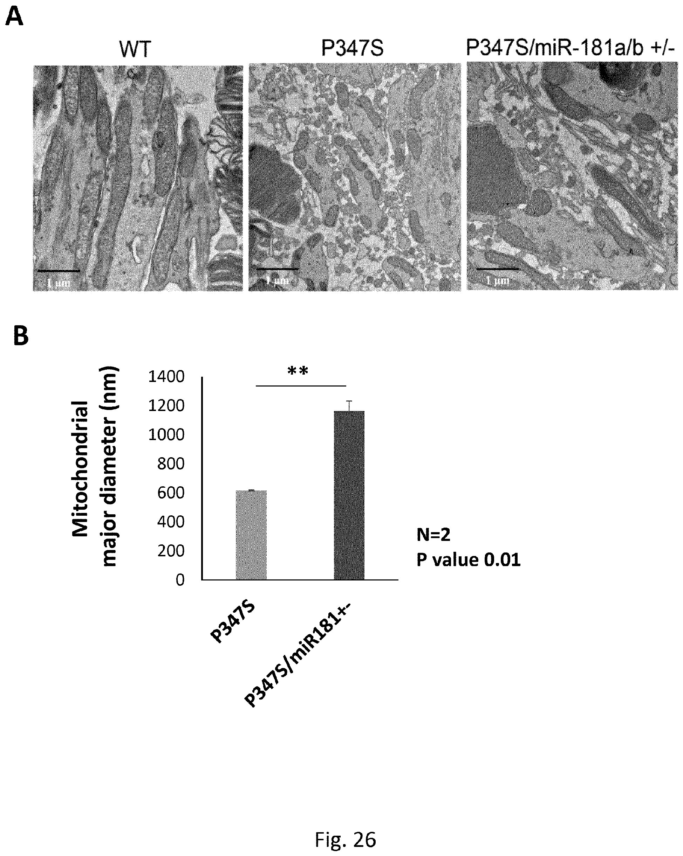

**Figure 26****. miR-181a/b reduction ameliorates mitochondria morphology in P347S photoreceptors. A)** Electron microscopy (EM) images of retina of WT, P347S and P347S/mir-181a/b^{+/-} animals at p30; n=2 animals/genotype. The EM images of PRs mitochondria of WT, P347S and P347S/miR-181a/b^{+/-} at p30 showed an amelioration of mitochondria phenotype in miR-181a/b downregulated animals. **B)** quantification of mitochondrial major diameter length. N= 2; ** p=0.01, Student's t-test. Error bars are SEM

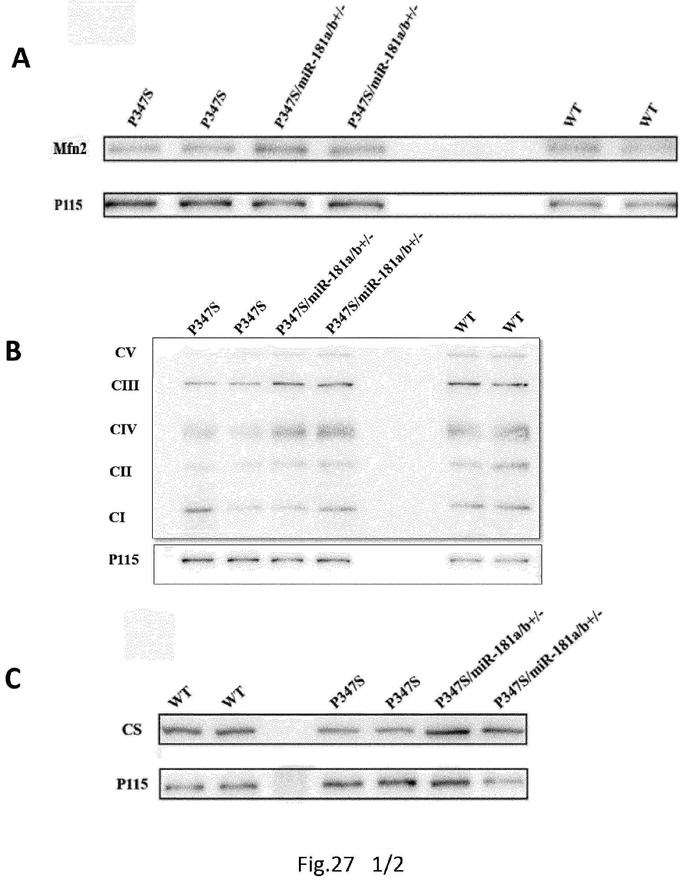

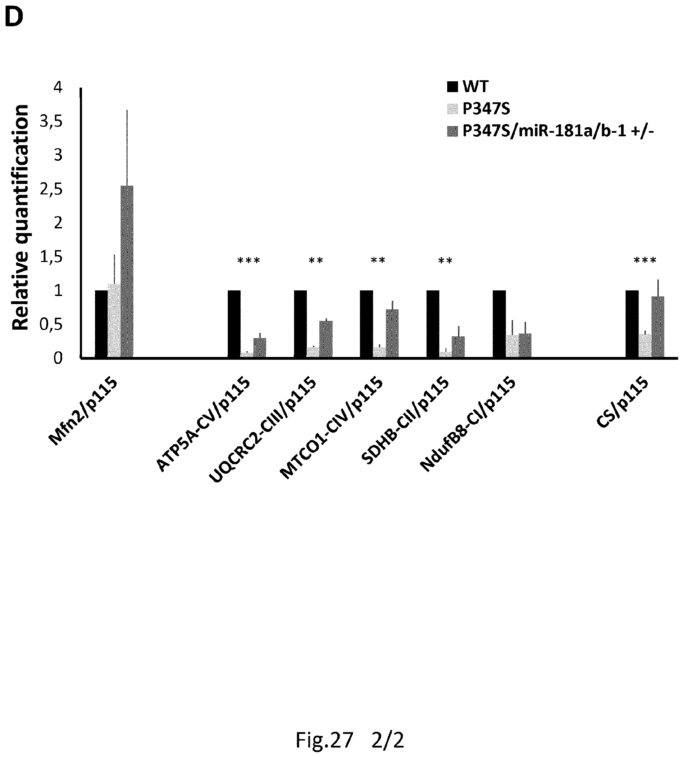

**Figure 27****. miR-181a/b reduction ameliorates mitochondria markers expression in P347S eyes. A)** Protein levels of Mfn2 detected by WB analysis in protein extracts from eyes of Wild Type, P347S and P347S/miR-181a/b^{+/-} animals at p 30; Quantification in D), data are normalized to p115. n=2 animals/genotype. Error bars are SEM. **B)** Protein levels of CI subunit NDUFB8, CII SDHB, CIII UQCRC2, CIV MTC01, CV ATP5A detected by WB analysis in protein extracts from eyes of Wild Type, P347S and P347S/miR-181a/b^{+/-} animals at p 30. Quantifications of protein levels in D) Data are normalized to p115. n=2 animals/genotype. Error bars are SEM. **C)** Protein levels of Citrate Synthase detected by WB analysis in protein extracts from eyes of Wild Type, P347S and P347S/miR-181a/b^{+/-} animals at p 30; the quantifications of protein levels are also shown in D), Data are normalized to p115. n=4 animals/genotype. **D)** Graphical representation of protein quantification of WB shown in A, B and C. Data are normalized to p115. * p<0.05;** p<0.01, *** p < 0.001 two tailed Student's t-test. Error bars are SEM.

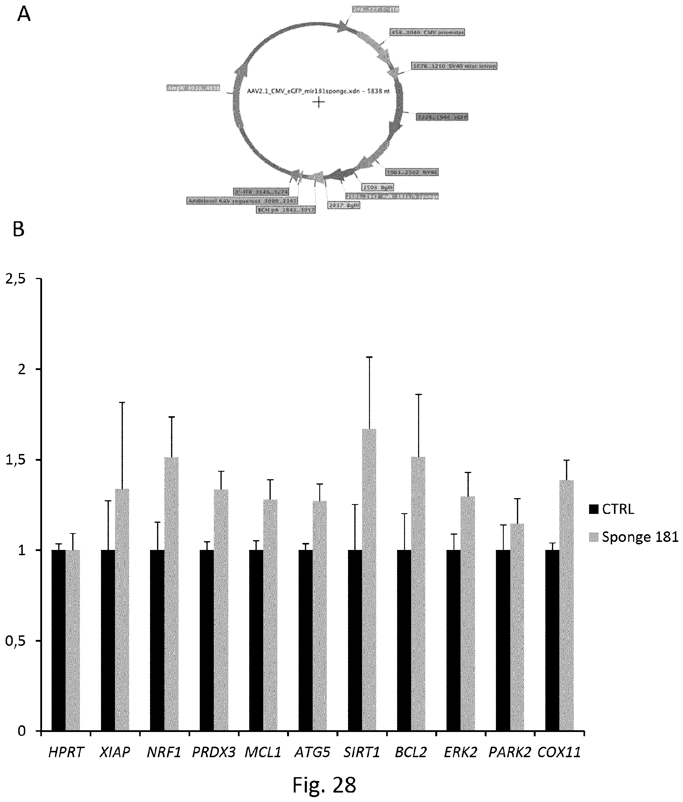

**Figure 28****. *In vitro* experimental validation of miR-181a/b sponge efficiency.**
   **A)** AAV2.1-CMV-eGFP-miR-181 Sponge map. **B)** qRT-PCR analysis of miR-181a/b direct target transcripts (XIAP, NRF1, PRDX3, MCL1, ATG5, SIRT1, BCL2, BCL2, ERK2, PARK2, COX11) in SH-SY5Y upon 48h transfection with AAV2.1-CMV-eGFP-miR-181a/b Sponge (Sponge 181). Transcript levels of miR-181a/b targets are increased in Sponge 181 sample respect to control sample (CTRL= AAV2.1-CMV-eGFP transfected cells). N=3. This data indicates that the miR-181 Sponge sequesters the endogenous miR-181a/b, thus preventing the binding of their direct targets and inhibiting their activity.

### Brief Description of the Sequences in the Sequence listing

### Sequences comparison of miR-181 family members.

| | | |
|---|---|---|
| miR-181a-5p | AACAUUCAACGCUGUCGGUGAGU AACAUUCAUUGCUGUCGGUGGGU AACAUUCAAC-CUGUCGGUGAGU AACAUUCAUUGUUGUCGGUGGGU ********    ********  ** | |
| miR-181b-5p | | |
| miR-181c-5p | | |
| miR-181d-5p | | (*=identical sequence) |
| miR-181a-5p | AACAUUCAACGCUGUCGGUGAGU AACAUUCAAC-CUGUCGGUGAGU ********** ************ | |
| miR-181c-5p | | (*=identical sequence) |
| miR-181b-5p | AACAUUCAUUGCUGUCGGUGGGU AACAUUCAUUGUUGUCGGUGGGU *********** *********** | |
| miR-181d-5p | | (*=identical sequence) |

miR-181a (SEQ ID NO. 1)
   AACAUUCAACGCUGUCGGUGAGU
miR-181b (SEQ ID NO. 2)
   AACAUUCAUUGCUGUCGGUGGGU
miR-181c (SEQ ID NO. 3)
   AACAUUCAAC-CUGUCGGUGAGU
miR-181d (SEQ ID NO. 4)
   AACAUUCAUUGUUGUCGGUGGGU
miR-181 SEED Sequence (SEQ ID NO. 5)
   ACAUUCA LNA A (SEQ ID NO. 6)
   CGACAGCGTTGAATGT LNA B (SEQ ID NO. 7)
   CGACAGCAATGAATGT
miR-181 Sponge Sequence: (SEQ ID NO. 8)

3'TTGTAAGTGATAACAGCCACTCAtagcTTGTAAGTCGTAACAGCCACCCAtagcTTGTAAGTGATAACAGCCACT
CAtagcTTGTAAGTCGTAACAGCCACCCAtagcTTGTAAGTGATAACAGCCACTCAtagcTTGTAAGTCGTAACAGC
CACCCAtctagaTTGTAAGTGATAACAGCCACTCAtagcTTGTAAGTCGTAACAGCCACCCAtagcTTGTAAGTGATA
ACAGCCACTCAtagcTTGTAAGTCGTAACAGCCACCCAtagcTTGTAAGTGATAACAGCCACTCAtagcTTGTAAGT
CGTAACAGCCACCCA-5'

Legend of miR-181 Sponge:
**miR-181 Sponge "MBS SEQ A" in bold font;** miR-181 Sponge "MBS SEQ B" in underlined font; Spacer "1" : *tagc in* *italic;* Spacer "2" or Central Spacer: Tctaga (restriction enzyme)
miR-181 Sponge Sequence: SEQ ID NO. 9 
3'TTGTAAGTGTAAACAGCCACTCAtagcTTGTAAGTCTTAACAGCCACCCAtagcTTGTAAGTGTAAACAGCCACT
CAtagcTTGTAAGTCTTAACAGCCACCCAtagcTTGTAAGTGTAAACAGCCACTCAtagcTTGTAAGTCTTAACAGCC
ACCCAtctagaTTGTAAGTGTAAACAGCCACTCAtagcTTGTAAGTCTTAACAGCCACCCAAtagcTTGTAAGTGTAA
ACAGCCACTCAtagcTTGTAAGTCTTAACAGCCACCCAtagcTTGTAAGTGTAAACAGCCACTCAtagcTTGTAAGT
CTTAACAGCCACCCA-5'
miR-181 Sponge Sequence: SEQ ID NO. 10 
3'TTGTAAGTCTAAACAGCCACTCAtagcTTGTAAGTCTTAACAGCCACCCAtagcTTGTAAGTCTAAACAGCCACT
CAtagcTTGTAAGTCTTAACAGCCACCCAtagcTTGTAAGTCTAAACAGCCACTCAtagcTTGTAAGTCTTAACAGCC
ACCCAtctagaTTGTAAGTCTAAACAGCCACTCAtagcTTGTAAGTCTTAACAGCCACCCAtagcTTGTAAGTCTAAA
CAGCCACTCAtagcTTGTAAGTCTTAACAGCCACCCAtagcTTGTAAGTCTAAACAGCCACTCAtagcTTGTAAGTCT
TAACAGCCACCCA-5'
miR-181 Sponge Sequence: SEQ ID NO. 11 
3'TTGTAAGTCTGGACAGCCACTCAtagcTTGTAAGTCGAAACAGCCACCCAtagcTTGTAAGTCTGGACAGCCACT
CAtagcTTGTAAGTCGAAACAGCCACCCAtagcTTGTAAGTCTGGACAGCCACTCAtagcTTGTAAGTCGAAACAGC
CACCCAtctagaTTGTAAGTCTGGACAGCCACTCAtagcTTGTAAGTCGAAACAGCCACCCAtagcTTGTAAGTCTGG
ACAGCCACTCAtagcTTGTAAGTCGAAACAGCCACCCAtagTTGTAAGTCTGGACAGCCACTCAtagcTTGTAAGTC
GAAACAGCCACCCA-5'
miR-181 Sponge Sequence: SEQ ID NO. 12 
3'TTGTAAGTGTAAACAGCCACTCAtagcTTGTAAGTCTTAACAGCCACCCAtagcTTGTAAGTGTAAACAGCCACT
CAtagcTTGTAAGTCTTAACAGCCACCCAtagcTTGTAAGTGTAAACAGCCACTCAtagcTTGTAAGTCTTAACAGCC
ACCCAtctagaTTGTAAGTCTGGACAGCCACTCAtagcTTGTAAGTCGAAACAGCCACCCAtagcTTGTAAGTCTGG
ACAGCCACTCAtagcTTGTAAGTCGAAACAGCCACCCAtagTTGTAAGTCTGGACAGCCACTCAtagcTTGTAAGTC
GAAACAGCCACCCA-5'
miR-181 Sponge Sequence: SEQ ID NO. 13 
3'TTGTAAGTGTAAACAGCCACTCAtagcTTGTAAGTCTTAACAGCCACCCAtagcTTGTAAGTGTAAACAGCCACT
CAtagcTTGTAAGTCTTAACAGCCACCCAtagcTTGTAAGTGTAAACAGCCACTCAtagcTTGTAAGTCTTAACAGCC
ACCCAtctagaTTGTAAGTCTGGACAGCCACTCAtagcTTGTAAGTCGAAACAGCCACCCAtagcTTGTAAGTCTGG
ACAGCCACTCAtagcTTGTAAGTCGAAACAGCCACCCAtagTTGTAAGTCTGGACAGCCACTCAtagcTTGTAAGTC
GAAACAGCCACCCA-5'
AAV2.1 CMV-eGFP-miR-181 Sponge sequence (SEQ ID NO. 14)

> [AAV2.1_CMV_eGFP_miR-181sponge] - 5838 bp
agcgcccaatacgcaaaccgcctctccccgcgcgttggccgattcattaatgcagctggcacgacaggtttcccgactggaaagcggg
cagtgagcgcaacgcaattaatgtgagttagctcactcattaggcaccccaggctttacactttatgcttccggctcgtatgttgtgtgga
attgtgagcggataacaatttcacacaggaaacagctatgaccatgattacgccagatttaattaaggctgcgcgctcgctcgctcact
gaggccgcccgggcaaagcccgggcgtcgggcgacctttggtcgcccggcctcagtgagcgagcgagcgcgcagagagggagtggc
caactccatcactaggggttccttgtagttaatgattaacccgccatgctacttatctacgtagccatgctctaggaagatcggaattcgc
ccttaagctagctagttattaatagtaatcaattacggggtcattagttcatagcccatatatggagttccgcgttacataacttacggta
aatggcccgcctggctgaccgcccaacgacccccgcccattgacgtcaataatgacgtatgttcccatagtaacgccaatagggacttt
ccattgacgtcaatgggtggagtatttacggtaaactgcccacttggcagtacatcaagtgtatcatatgccaagtacgccccctattga
cgtcaatgacggtaaatggcccgcctggcattatgcccagtacatgaccttatgggactttcctacttggcagtacatctacgtattagtc
atcgctattaccatggtgatgcggttttggcagtacatcaatgggcgtggatagcggtttgactcacggggatttccaagtctccacccc
attgacgtcaatgggagtttgttttggcaccaaaatcaacgggactttccaaaatgtcgtaacaactccgccccattgacgcaaatggg
cggtaggcgtgtacggtgggaggtctatataagcagagctggtttagtgaaccgtcagatcctgcagaagttggtcgtgaggcactgg
gcaggtaagtatcaaggttacaagacaggtttaaggagaccaatagaaactgggcttgtcgagacagagaagactcttgcgtttctga
taggcacctattggtcttactgacatccactttgcctttctctccacaggtgtccaggcggccgccatggtgagcaagggcgaggagctg
ttcaccggggtggtgcccatcctggtcgagctggacggcgacgtaaacggccacaagttcagcgtgtccggcgagggcgagggcgat
gccacctacggcaagctgaccctgaagttcatctgcaccaccggcaagctgcccgtgccctggcccaccctcgtgaccaccctgaccta
cggcgtgcagtgcttcagccgctaccccgaccacatgaagcagcacgacttcttcaagtccgccatgcccgaaggctacgtccaggag
cgcaccatcttcttcaaggacgacggcaactacaagacccgcgccgaggtgaagttcgagggcgacaccctggtgaaccgcatcgag
ctgaagggcatcgacttcaaggaggacggcaacatcctggggcacaagctggagtacaactacaacagccacaacgtctatatcatg
gccgacaagcagaagaacggcatcaaggtgaacttcaagatccgccacaacatcgaggacggcagcgtgcagctcgccgaccacta
ccagcagaacacccccatcggcgacggccccgtgctgctgcccgacaaccactacctgagcacccagtccgccctgagcaaagaccc
caacgagaagcgcgatcacatggtcctgctggagttcgtgaccgccgccgggatcactctcggcatggacgagctgtacaagtaataa
gcttggatccaatcaacctctggattacaaaatttgtgaaagattgactggtattcttaactatgttgctccttttacgctatgtggatacg
ctgctttaatgcctttgtgctgcgctattgcttcccgtatggctttcattttctcctccttgtataaatcctggttgctgtctctttatgaggag
ttgtggcccgttgtcaggcaacgtggcgtggtgtgcactgtgtttgctgacgcaacccccactggttggggcattgccaccacctgtcag
ctcctttccgggactttcgctttccccctccctattgccacggcggaactcatcgccgcctgccttgcccgctgctggacaggggctcggct
gttgggcactgacaattccgtggtgttgtcggggaagctgacgtcctttccttggctgctcgcctgtgttgccacctggattctgcgcggg
acgtccttctgctacgtcccttcggccctcaatccagcggaccttccttcccgcggcctgctgccggctctgcggcctcttccgcgtcttcg
AGATCTTCTAGAACCCACCGACAATGCTGAATGTTTAGCACTCACCGACAATAGTGAATGTTTAGCACC

CACCGACAATGCTGAATGTTTAGCACTCACCGACAATAGTGAATGTTTAGCACCCACCGACAATGCTGA
ATGTTTAGCACTCACCGACAATAGTGAATGTTTCTAGAACCCACCGACAATGCTGAATGTTTAGCACTC
ACCGACAATAGTGAATGTTTAGCACCCACCGACAATGCTGAATGTTTAGCACTCACCGACAATAGTGAA
TGTTTAGCACCCACCGACAATGCTGAATGTTTAGCACTCACCGACAATAGTGAATGTTAGATCTgcctcga
ctgtgccttctagttgccagccatctgttgtttgcccctcccccgtgccttccttgaccctggaaggtgccactcccactgtcctttcctaat
aaaatgaggaaattgcatcgcattgtctgagtaggtgtcattctattctggggggtggggtggggcaggacagcaagggggaggattg
ggaagacaatagcaggcatgctggggactcgagttaagggcgaattcccgattaggatcttcctagagcatggctacgtagataagta
gcatggcgggttaatcattaactacaaggaacccctagtgatggagttggccactccctctctgcgcgctcgctcgctcactgaggccgg
gcgaccaaaggtcgcccgacgcccgggctttgcccgggcggcctcagtgagcgagcgagcgcgcagccttaattaacctaattcactg
gccgtcgttttacaacgtcgtgactgggaaaaccctggcgttacccaacttaatcgccttgcagcacatccccctttcgccagctggcgt
aatagcgaagaggcccgcaccgatcgcccttcccaacagttgcgcagcctgaatggcgaatgggacgcgccctgtagcggcgcatta
agcgcggcgggtgtggtggttacgcgcagcgtgaccgctacacttgccagcgccctagcgcccgctcctttcgctttcttcccttcctttct
cgccacgttcgccggctttccccgtcaagctctaaatcgggggctccctttagggttccgatttagtgctttacggcacctcgaccccaaa
aaacttgattagggtgatggttcacgtagtgggccatcgccccgatagacggtttttcgccctttgacgctggagttcacgttcctcaata
gtggactcttgttccaaactggaacaacactcaaccctatctcggtctattcttttgatttataagggatttttccgatttcggcctattggt
taaaaaatgagctgatttaacaaaaatttaacgcgaattttaacaaaatattaacgtttataatttcaggtggcatctttcggggaaatg
tgcgcggaacccctatttgtttatttttctaaatacattcaaatatgtatccgctcatgagacaataaccctgataaatgcttcaataatat
tgaaaaaggaagagtatgagtattcaacatttccgtgtcgcccttattcccttttttgcggcattttgccttcctgtttttgctcacccagaa
acgctggtgaaagtaaaagatgctgaagatcagttgggtgcacgagtgggttacatcgaactggatctcaatagtggtaagatccttg
agagttttcgccccgaagaacgttttccaatgatgagcacttttaaagttctgctatgtggcgcggtattatcccgtattgacgccgggca
agagcaactcggtcgccgcatacactattctcagaatgacttggttgagtactcaccagtcacagaaaagcatcttacggatggcatga
cagtaagagaattatgcagtgctgccataaccatgagtgataacactgcggccaacttacttctgacaacgatcggaggaccgaagga
gctaaccgcttttttgcacaacatgggggatcatgtaactcgccttgatcgttgggaaccggagctgaatgaagccataccaaacgacg
agcgtgacaccacgatgcctgtagtaatggtaacaacgttgcgcaaactattaactggcgaactacttactctagcttcccggcaacaa
ttaatagactggatggaggcggataaagttgcaggaccacttctgcgctcggcccttccggctggctggtttattgctgataaatctgga
gccggtgagcgtgggtctcgcggtatcattgcagcactggggccagatggtaagccctcccgtatcgtagttatctacacgacggggag
tcaggcaactatggatgaacgaaatagacagatcgctgagataggtgcctcactgattaagcattggtaactgtcagaccaagtttact
catatatactttagattgatttaaaacttcatttttaatttaaaaggatctaggtgaagatcctttttgataatctcatgaccaaaatccctt
aacgtgagttttcgttccactgagcgtcagaccccgtagaaaagatcaaaggatcttcttgagatcctttttttctgcgcgtaatctgctg
cttgcaaacaaaaaaaccaccgctaccagcggtggtttgtttgccggatcaagagctaccaactctttttccgaaggtaactggcttcag
cagagcgcagataccaaatactgtccttctagtgtagccgtagttaggccaccacttcaagaactctgtagcaccgcctacatacctcgc
tctgctaatcctgttaccagtggctgctgccagtggcgataagtcgtgtcttaccgggttggactcaagacgatagttaccggataaggc

gcagcggtcgggctgaacggggggttcgtgcacacagcccagcttggagcgaacgacctacaccgaactgagatacctacagcgtga
gctatgagaaagcgccacgcttcccgaagggagaaaggcggacaggtatccggtaagcggcagggtcggaacaggagagcgcacg
agggagcttccagggggaaacgcctggtatctttatagtcctgtcgggtttcgccacctctgacttgagcgtcgatttttgtgatgctcgtc
aggggggcggagcctatggaaaaacgccagcaacgcggcctttttacggttcctggccttttgctgcggttttgctcacatgttctttcct
gcgttatcccctgattctgtggataaccgtattaccgcctttgagtgagctgataccgctcgccgcagccgaacgaccgagcgcagcga
gtcagtgagcgaggaagcggaag

### Definitions

The terms "polynucleotide," "oligonucleotide," "nucleic acid" and "nucleic acid molecule" are used herein to include a polymeric form of nucleotides of any length, either ribonucleotides or deoxyribonucleotides. This term refers only to the primary structure of the molecule. Thus, the term includes triple-, double- and single-stranded DNA, as well as triple-, double- and single-stranded RNA. It also includes modifications, such as by methylation and/or by capping, and unmodified forms of the polynucleotide. More particularly, the terms "polynucleotide," "oligonucleotide," "nucleic acid" and "nucleic acid molecule" include polydeoxyribonucleotides (containing 2-deoxy-D-ribose), polyribonucleotides (containing D-ribose), any other type of polynucleotide which is an N- or C-glycoside of a purine or pyrimidine base, and other polymers containing non nucleotidic backbones, for example, polyamide (e.g., peptide nucleic acids (PNAs)) and polymorpholino (commercially available from the Anti-Virals, Inc., Corvallis, Oreg., as Neugene) polymers, and other synthetic sequence-specific nucleic acid polymers providing that the polymers contain nucleobases in a configuration which allows for base pairing and base stacking, such as is found in DNA and RNA. There is no intended distinction in length between the terms "polynucleotide," "oligonucleotide," "nucleic acid" and "nucleic acid molecule," and these terms will be used interchangeably. Thus, these terms include, for example, 3'-deoxy-2',5'-DNA, oligodeoxyribonucleotide N3' P5' phosphoramidates, 2'-O-alkyl-substituted RNA, double- and single-stranded DNA, as well as double- and single-stranded RNA, microRNA, DNA:RNA hybrids, and hybrids between PNAs and DNA or RNA, and also include known types of modifications, for example, labels which are known in the art, methylation, "caps," substitution of one or more of the naturally occurring nucleotides with an analog (e.g., 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oooadenosine, 8-oxoguanosine, O(6)-methylguanine, and 2-thiocytidine), internucleotide modifications such as, for example, those with uncharged linkages (e.g., methyl phosphonates, phosphotriesters, phosphoramidates, carbamates, etc.), with negatively charged linkages (e.g., phosphorothioates, phosphorodithioates, etc.), and with positively charged linkages (e.g., aminoalklyphosphoramidates, aminoalkylphosphotriesters), those containing pendant moieties, such as, for example, proteins (including nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide or oligonucleotide. The term also includes locked nucleic acids (e.g., comprising a ribonucleotide that has a methylene bridge between the 2'-oxygen atom and the 4'-carbon atom). See, for example, (Elayadi & Corey, 2001; Koshkin et al, 1998; Kurreck et al, 2002; Obika et al, 1998; Orum & Wengel, 2001).

The terms "label" and "detectable label" refer to a molecule capable of detection, including, but not limited to, radioactive isotopes, fluorescers, chemiluminescers, enzymes, enzyme substrates, enzyme co factors, enzyme inhibitors, chromophores, dyes, metal ions, metal sols, ligands (e.g., biotin or haptens) and the like. The term "fluorescer" refers to a substance or a portion thereof that is capable of exhibiting fluorescence in the detectable range. Particular examples of labels that may be used with the invention include, but are not limited to phycoerythrin, Alexa dyes, fluorescein, YPet, CyPet, Cascade blue, allophycocyanin, Cy3, Cy5, Cy7, rhodamine, dansyl, umbelliferone, Texas red, luminol, acradimum esters, biotin, green fluorescent protein (GFP), enhanced green fluorescent protein (EGFP), yellow fluorescent protein (YFP), enhanced yellow fluorescent protein (EYFP), blue fluorescent protein (BFP), red fluorescent protein (RFP), Dronpa, mCherry, mOrange, mPlum, Venus, firefly luciferase, Renilla luciferase, NADPH, beta-galactosidase, horseradish peroxidase, glucose oxidase, alkaline phosphatase, chloramphenical acetyl transferase, urease, MRI contrast agents (e.g., gadodiamide, gadobenic acid, gadopentetic acid, gadoteridol, gadofosveset, gadoversetamide, and gadoxetic acid), and computed tomography (CT) contrast agents (e.g., Diatrizoic acid, Metrizoic acid, lodamide, lotalamic acid, loxitalamic acid, loglicic acid, Acetrizoic acid, locarmic acid, Methiodal, Diodone, Metrizamide, lohexol, loxaglic acid, lopamidol, lopromide, lotrolan, loversol, lopentol, lodixanol, lomeprol, lobitridol, loxilan, lodoxamic acid, lotroxic acid, loglycamic acid, Adipiodone, lobenzamic acid, lopanoic acid, locetamic acid, Sodium iopodate, Tyropanoic acid, and Calcium iopodate).

"Recombinant" as used herein to describe a nucleic acid molecule means a polynucleotide of genomic, RNA, miRNA, cDNA, viral, semisynthetic, or synthetic origin which, by virtue of its origin or manipulation is not associated with all or a portion of the polynucleotide with which it is associated in nature.

The term "recombinant" as used with respect to a protein or polypeptide means a polypeptide produced by expression of a recombinant polynucleotide. In general, the gene of interest is cloned and then expressed in transformed organisms, as described further below.

### microRNA

As used herein, the terms "micro RNA," "miRNA," "mature micro RNA," and "mature miRNA" refer to a non-coding single-stranded RNA molecule that is about 19 to about 25 nucleotides in length (including about 19, about 20, about 21, about 22, about 23, about 24, and about 25 nucleotides) that effectively reduces the expression level of target polynucleotides and polypeptides through the RNA interference pathway (i.e., through association with the RISC and subsequent degradation of target mRNA or translational inhibition). The term "microRNA" refers to both endogenous miRNAs that have been found in any organism (e.g., plants, animals) and artificial miRNAs that include single-stranded RNA molecules with sequences of about 19-25 nucleotides in length other than those found in endogenous miRNAs that effectively reduce the expression of target polynucleotides through RNA interference.

MicroRNAs are grouped into families based on their targeting properties, which depend primarily on the identity of their extended seed region (miRNA nucleotides 2-8); members of the same seed family are usually evolutionarily related, and evolutionarily related miRNAs are usually members of the same seed family. However, the use of the term "family" does not strictly denote common ancestry, as described in (Bartel, 2018).

miRNAs of the present invention belong to the miRNA 181 family of miRNAs.

As defined herein, miRNA 181 are a group of miRNAs sharing the same Seed sequence, namely ACAUUCA (SEQ ID NO. 5). Preferably, said group of miRNA is defined herein as a family of miRNAs.

The miR-181 family members are produced by three independent primary transcripts localized to three separate chromosomes. Each transcript produces two precursor sequences for a total of six precursor sequences. Each precursor produces two miRNA strands: the guide strand or 5p, and the passenger strand or 3p, for a total of ten mature miRNA forms: miR-181a-5p, miR-181a-1-3p, miR-181a-2-3p, miR-181b-5p, miR-181b-1-3p, miR-181b-2-3p miR-181c-5p, miR-181c-3p, miR-181d-5p and miR-181d-3p. The 5p mature sequences are those displaying the most abundant expression levels in mammalian tissues (Karali et al, 2016) and contain the same 5' "seed" sequence suggesting a significant degree of functional redundancy (Henao-Mejia et al, 2013; Ji et al, 2009). As used herein, the six mature 5p miRNAs will be defined as: miR-181a-1, miR-181a-2, miR-181b-1, miR-181b-2, miR-181c, and miR-181d. The mature forms of miR-181a-1 and miR-181a-2, as well as miR-181b-1 and miR-181b-2 are identical in sequence; miR-181c mature form has one nucleotide difference compared to miR-181a, whilst miR-181d mature form has one nucleotide difference compared to miR-181b.

A "target site" is the nucleic acid sequence recognized by a microRNA. A single target site typically has about six to about ten nucleotides. Typically, the target site is located within the 3'UTR of a mRNA, but the target site may also be located in the 5'UTR or the coding region of a mRNA.

An "inhibitor target site" is a sequence within the mature sequence of a miRNA 181 complementary to said inhibitor nucleic acid. The miRNA 181 sequence may be a precursor sequence or a mature sequence. Preferably, the miRNA 181 sequence is a mature sequence.

### Inhibitory Nucleic Acids

Inhibitory nucleic acids comprise inhibitory RNA and inhibitory DNA, e.g. may be ribonucleotides or deoxyribonucleotides. By the term "inhibitory nucleic acid" is meant a nucleic acid molecule that contains a sequence that is complementary to a target nucleic acid (e.g., a target microRNA e.g., any of the microRNAs defined by SEQ ID NO. 1, 2, 3, or 4) and binds it, mediating a decrease in the level or activity of the target nucleic acid (e.g., activity in mRNA target binding) by steric hindrance or by degradation of the target. Non-limiting examples of inhibitory nucleic acids include interfering RNA, shRNA, siRNA, ribozymes, antagomirs, LNA, miR sponge, and antisense oligonucleotides. Methods of making inhibitory RNAs are described herein. Additional methods of making inhibitory nucleic acids are known in the art.

As used herein, "an interfering RNA" refers to any double stranded or single stranded RNA sequence, capable- either directly or indirectly (i.e., upon conversion)- of inhibiting or down regulating gene expression by mediating RNA interference. Interfering RNA includes but is not limited to small interfering RNA ("siRNA") and small hairpin RNA ("shRNA"). "RNA interference" refers to the selective degradation of a sequence-compatible messenger RNA transcript.

As used herein "an shRNA" (small hairpin RNA) refers to an RNA molecule comprising an antisense region, a loop portion and a sense region, wherein the sense region has complementary nucleotides that base pair with the antisense region to form a duplex stem.

Following post-transcriptional processing, the small hairpin RNA is converted into a small interfering RNA by a cleavage event mediated by the enzyme Dicer, which is a member of the RNase III family.

A "small interfering RNA" or "siRNA" as used herein refers to any small RNA molecule capable of inhibiting or down regulating gene expression by mediating RNA interference in a sequence specific manner. The small RNA can be, for example, about 18 to 21 nucleotides long.

As used herein, an "antagomir" refers to a small synthetic RNA having complementarity to a specific microRNA target, optionally with either mispairing at the cleavage site or one or more base modifications to inhibit cleavage.

As used herein, the phrase "post-transcriptional processing" refers to mRNA processing that occurs after transcription and is mediated, for example, by the enzymes Dicer and/or Drosha. Inhibitory agents useful in the methods of treatment described herein include inhibitory nucleic acid molecules that decrease the expression or activity of any of the microRNAs (e.g., mature microRNA or precursor microRNA) of SEQ ID No. 1, 2, 3 or 4 or a combination thereof.

Inhibitory nucleic acids useful in the present methods and compositions include antisense oligonucleotides, ribozymes, external guide sequence (EGS) oligonucleotides, siRNA compounds, single- or double-stranded RNA interference (RNAi) compounds, such as siRNA compounds, modified bases/locked nucleic acids (LNAs), antagomirs, peptide nucleic acids (PNAs), and other oligomeric compounds, or oligonucleotide mimetics which hybridize to at least a portion of the target nucleic acid and modulate its function. In some embodiments, the inhibitory nucleic acids include antisense RNA, antisense DNA, chimeric antisense oligonucleotides, antisense oligonucleotides comprising modified linkages, interference RNA (RNAi), short interfering R A (siRNA); a micro, interfering R A (miRNA); a small, temporal RNA (stRNA); or a short, hairpin RNA (shRNA); small RNA-induced gene activation (RNAa); small activating RNAs (saRNAs), or combinations thereof. See, e.g., WO 2010/040112.

In some embodiments, the inhibitory nucleic acids are 10 to 50, 13 to 50, or 13 to 30 nucleotides in length. One having ordinary skill in the art will appreciate that this embodies oligonucleotides having antisense portions of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides in length, or any range therewithin. In some embodiments, the oligonucleotides are 15 nucleotides in length. In some embodiments, the antisense or oligonucleotide compounds of the invention are 12 or 13 to 30 nucleotides in length. One having ordinary skill in the art will appreciate that this embodies inhibitory nucleic acids having antisense portions of 12, 13, 14, 15, 16, 17, 18, 19, 20, 21, 22, 23, 24, 25, 26, 27, 28, 29, or 30 nucleotides in length, or any range therewithin.

In some embodiments, the inhibitory nucleic acids are chimeric oligonucleotides that contain two or more chemically distinct regions, each made up of at least one nucleotide. These oligonucleotides typically contain at least one region of modified nucleotides that confers one or more beneficial properties (such as, for example, increased nuclease resistance, increased uptake into cells, increased binding affinity for the target) and a region that is a substrate for enzymes capable of cleaving RNA:DNA or RNA:RNA hybrids. Chimeric inhibitory nucleic acids of the invention may be formed as composite structures of two or more oligonucleotides, modified oligonucleotides, oligonucleosides, and/or oligonucleotide mimetics as described above. Such compounds have also been referred to in the art as hybrids or gapmers. Representative United States patents that teach the preparation of such hybrid structures comprise, but are not limited to, U.S. Patent Nos. 5,013,830; 5,149,797; 5, 220,007; 5,256,775; 5,366,878; 5,403,711; 5,491,133; 5,565,350; 5,623,065; 5,652,355; 5,652,356; and 5,700,922.

In some embodiments, the inhibitory nucleic acid comprises at least one nucleotide modified at the 2' position of the sugar, most preferably a 2'-0-alkyl, 2'-0-alkyl-0-alkyl or 2'- fluoro-modified nucleotide. In other preferred embodiments, RNA modifications include 2'- fluoro, 2'-amino, and 2' O-methyl modifications on the ribose of pyrimidines, basic residues, or an inverted base at the 3' end of the RNA. Such modifications are routinely incorporated into oligonucleotides and these oligonucleotides have been shown to have a higher Tm (i.e., higher target binding affinity) than 2'-deoxyoligonucleotides against a given target.

A number of nucleotide and nucleoside modifications have been shown to make the oligonucleotide into which they are incorporated more resistant to nuclease digestion than the native oligodeoxynucleotide the modified oligos survive intact for a longer time than unmodified oligonucleotides. Specific examples of modified oligonucleotides include those comprising modified backbones, for example, phosphorothioates, phosphotriesters, methyl phosphonates, short-chain alkyl or cycloalkyl intersugar linkages, or short-chain heteroatomic or heterocyclic intersugar linkages. Most preferred are oligonucleotides with phosphorothioate backbones and those with heteroatom backbones, particularly CH2-NH-0-CH2, CH,^{~}N(CH3)^{~}0^{~}CH2 (known as a methylene(methylimino) or MMI backbone], CH2 -O-N (CH3)-CH2, CH2 -N (CH3)-N (CH3)-CH2 and O-N (CH3)- CH2 -CH2 backbones, wherein the native phosphodiester backbone is represented as O- P- O- CH,); amide backbones (see (De Mesmaeker et al, 1995); morpholino backbone structures (see U.S. Patent No. 5,034,506); peptide nucleic acid (PNA) backbone (wherein the phosphodiester backbone of the oligonucleotide is replaced with a polyamide backbone, the nucleotides being bound directly or indirectly to the aza nitrogen atoms of the polyamide backbone, see (Nielsen et al, 1991). Phosphorus-containing linkages include, but are not limited to, phosphorothioates, chiral phosphorothioates, phosphorodithioates, phosphotriesters, aminoalkylphosphotriesters, methyl and other alkyl phosphonates comprising 3'alkylene phosphonates and chiral phosphonates, phosphinates, phosphoramidates comprising 3'-amino phosphoramidate and aminoalkylphosphoramidates, thionophosphoramidates, thionoalkylphosphonates, thionoalkylphosphotriesters, and boranophosphates having normal 3'- 5' linkages, 2'-5' linked analogs of these, and those having inverted polarity wherein the adjacent pairs of nucleoside units are linked 3'-5' to 5'-3' or 2'-5' to 5'-2'; see U.S. Patent Nos. 3,687,808; 4,469,863; 4,476,301; 5,023,243; 5, 177,196; 5,188,897; 5,264,423; 5,276,019; 5,278,302; 5,286,717; 5,321,131; 5,399,676; 5,405,939; 5,453,496; 5,455, 233; 5,466,677; 5,476,925; 5,519,126; 5,536,821; 5,541,306; 5,550,111; 5,563, 253; 5,571,799; 5,587,361; and 5,625,050. Morpholino-based oligomeric compounds are described in (Braasch & Corey, 2002; Heasman, 2002; Lacerra et al, 2000; Nasevicius & Ekker, 2000) and U.S. Patent No. 5,034,506. Cyclohexenyl nucleic acid oligonucleotide mimetics are described in (Wang et al, 2000a).

Modified oligonucleotide backbones that do not include a phosphorus atom therein have backbones that are formed by short-chain alkyl or cycloalkyl internucleoside linkages, mixed heteroatom and alkyl or cycloalkyl internucleoside linkages, or one or more short-chain heteroatomic or heterocyclic internucleoside linkages. These comprise those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts; see U.S. Patent Nos. 5,034,506; 5,166,315; 5,185,444; 5,214,134; 5,216,141; 5,235,033; 5,264, 562; 5, 264,564; 5,405,938; 5,434,257; 5,466,677; 5,470,967; 5,489,677; 5,541,307; 5,561,225; 5,596, 086; 5,602,240; 5,610,289; 5,602,240; 5,608,046; 5,610,289; 5,618,704; 5,623, 070; 5,663,312; 5,633,360; 5,677,437; and 5,677,439.

One or more substituted sugar moieties can also be included, e.g., one of the following at the 2' position: OH, SH, SCH3, F, OCN, OCH3 OCH3, OCH3 0(CH2)n CH3, 0(CH2)n NH2 or 0(CH2)n CH3, where n is from 1 to about 10; Ci to CIO lower alkyl, alkoxyalkoxy, substituted lower alkyl, alkaryl or aralkyl; Cl; Br; CN; CF3 ; OCF3; 0-, S-, or N-alkyl; 0-, S-, or N-alkenyl; SOCH3; S02 CH3; ON02; N02; N3; NH2; heterocycloalkyl; heterocycloalkaryl; aminoalkylamino; polyalkylamino; substituted silyl; an RNA cleaving group; a reporter group; an intercalator; a group for improving the pharmacokinetic properties of an oligonucleotide; or a group for improving the pharmacodynamic properties of an oligonucleotide and other substituents having similar properties. A preferred modification includes 2'-methoxyethoxy [2'- 0-CH2CH2OCH3, also known as 2'-0-(2-methoxyethyl)] (Martin, 1995). Other preferred modifications include 2'-methoxy (2'-0-CH3), 2'-propoxy (2'-OCH2 CH2CH3) and 2'-fluoro (2'-F). Similar modifications may also be made at other positions on the oligonucleotide, particularly the 3' position of the sugar on the 3' terminal nucleotide and the 5' position of 5' terminal nucleotide. Oligonucleotides may also have sugar mimetics, such as cyclobutyls in place of the pentofuranosyl group.

Inhibitory nucleic acids can also include, additionally or alternatively, nucleobase (often referred to in the art simply as "base") modifications or substitutions. As used herein, "unmodified" or "natural" nucleobases include adenine (A), guanine (G), thymine (T), cytosine (C) and uracil (U). Modified nucleobases include nucleobases found only infrequently or transiently in natural nucleic acids, e.g., hypoxanthine, 6-methyladenine, 5-Me pyrimidines, particularly 5-methylcytosine (also referred to as 5-methyl-2' deoxy cytosine and often referred to in the art as 5-Me-C), 5-hydroxymethylcytosine (HMC), glycosyl HMC, and gentobiosyl HMC, as well as synthetic nucleobases, e.g., 2-aminoadenine, 2- (methylamino)adenine, 2-(imidazolylalkyl)adenine, 2-(aminoalklyamino)adenine or other heterosubstituted alkyladenines, 2-thiouracil, 2-thiothymine, 5-bromouracil, 5- hydroxymethyluracil, 8-azaguanine, 7- deazaguanine, N6 (6-aminohexyl)adenine, and 2,6- diaminopurine. See Kornberg, A., DNA Replication, W. H. Freeman & Co., San Francisco, 1980, pp75-77; and (Gebeyehu et al, 1987). A "universal" base known in the art, e.g., inosine, can also be included. 5-Me-C substitutions have been shown to increase nucleic acid duplex stability by 0.6- 1.2<0>C (Sanghvi, Y. S., in Crooke, S. T. and Lebleu, B., Eds., Antisense Research and Applications, CRC Press, Boca Raton, 1993, pp. 276-278) and are presently preferred base substitutions.

It is not necessary for all positions in a given oligonucleotide to be uniformly modified, and in fact more than one of the aforementioned modifications may be incorporated in a single oligonucleotide or even at within a single nucleoside within an oligonucleotide.

In some embodiments, both a sugar and an internucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar- backbone of an oligonucleotide is replaced with an amide containing backbone, for example, an aminoethylglycine backbone. The nucleobases are retained and are bound directly or indirectly to aza nitrogen atoms of the amide portion of the backbone. Representative United States patents that teach the preparation of PNA compounds comprise, but are not limited to, US Patent Nos. 5,539,082; 5,714,331; and 5,719,262. Further teaching of PNA compounds can be found in (Nielsen et al, 1991).

Inhibitory nucleic acids can also include one or more nucleobase (often referred to in the art simply as "base") modifications or substitutions. As used herein, "unmodified" or "natural" nucleobases comprise the purine bases adenine (A) and guanine (G), and the pyrimidine bases thymine (T), cytosine (C), and uracil (U). Modified nucleobases comprise other synthetic and natural nucleobases, such as 5-methylcytosine (5-me-C), 5-hydroxymethyl cytosine, xanthine, hypoxanthine, 2-aminoadenine, 6-methyl, and other alkyl derivatives of adenine and guanine, 2- propyl and other alkyl derivatives of adenine and guanine, 2-thiouracil, 2-thiothymine, and 2- thiocytosine, 5-halouracil and cytosine, 5-propynyl uracil and cytosine, 6-azo uracil, cytosine and thymine, 5-uracil (pseudo-uracil), 4-thiouracil, 8-halo, 8-amino, 8-thiol, 8- thioalkyl, 8-hydroxyl and other 8-substituted adenines and guanines, 5-halo particularly 5- bromo, 5-trifluoromethyl and other 5 -substituted uracils and cytosines, 7-methylquanine and 7-methyladenine, 8-azaguanine and 8-azaadenine, 7-deazaguanine, and 7-deazaadenine, and 3-deazaguanine and 3-deazaadenine.

Further, nucleobases comprise those disclosed in United States Patent No. 3,687,808, those disclosed in 'The Concise Encyclopedia of Polymer Science And Engineering', pages 858- 859, Kroschwitz, J.I., Ed. John Wiley & Sons, 1990, those disclosed by Englisch et al, Angewandle Chemie, International Edition', 1991, 30, page 613, and those disclosed by Sanghvi, Y. S., Chapter 15, Antisense Research and Applications', pages 289- 302, Crooke, S.T. and Lebleu, B. ea., CRC Press, 1993. Certain of these nucleobases are particularly useful for increasing the binding affinity of the oligomeric compounds of the invention. These include 5- substituted pyrimidines, 6-azapyrimidines, and N-2, N-6 and 0-6 substituted purines, comprising 2-aminopropyladenine, 5-propynyluracil, and 5- propynylcytosine. 5-methylcytosine substitutions have been shown to increase nucleic acid duplex stability by 0.6-1.2<0>C (Sanghvi, Y.S., Crooke, S.T. and Lebleu, B., Eds, Antisense Research and Applications', CRC Press, Boca Raton, 1993, pp. 276-278) and are presently preferred base substitutions, even more particularly when combined with 2'-0-methoxyethyl sugar modifications. Modified nucleobases are described in U.S. Patent Nos. 3,687,808, as well as 4,845,205; 5,130,302; 5,134,066; 5,175, 273; 5, 367,066; 5,432,272; 5,457,187; 5,459,255; 5,484,908; 5,502,177; 5,525,711; 5,552,540; 5,587,469; 5,596,091; 5,614,617; 5,750,692, and 5,681,941.

In some embodiments, the inhibitory nucleic acids are chemically linked to one or more moieties or conjugates that enhance the activity, cellular distribution, or cellular uptake of the oligonucleotide. Such moieties comprise but are not limited to, lipid moieties such as a cholesterol moiety (Letsinger et al, 1989), cholic acid (Manoharan et al, 1994), a thioether, e.g., hexyl-S- tritylthiol (Manoharan et al, 1992; Manoharan et al, 1993), a thiocholesterol (Oberhauser & Wagner, 1992), an aliphatic chain, e.g., dodecandiol or undecyl residues (Kabanov et al, 1990; Svinarchuk et al, 1993), a phospholipid, e.g., di- hexadecyl-rac-glycerol or triethylammonium 1,2-di-O-hexadecyl- rac-glycero-3-H-phosphonate (Manoharan et al, 1995; Shea et al, 1990), a polyamine or a polyethylene glycol chain (Mancharan et al, Nucleosides & Nucleotides 14:969-973, 1995), or adamantane acetic acid (Manoharan et al, 1995), a palmityl moiety (Mishra et al, 1995), or an octadecylamine or hexylamino-carbonyl-t oxycholesterol moiety (Crooke et al, 1996). See also U.S. Patent Nos. 4,828,979; 4,948,882; 5,218,105; 5,525,465; 5,541,313; 5,545,730; 5,552, 538; 5,578,717, 5,580,731; 5,580,731; 5,591,584; 5,109,124; 5,118,802; 5,138,045; 5,414,077; 5,486, 603; 5,512,439; 5,578,718; 5,608,046; 4,587,044; 4,605,735; 4,667,025; 4,762, 779; 4,789,737; 4,824,941; 4,835,263; 4,876,335; 4,904,582; 4,958,013; 5,082, 830; 5,112,963; 5,214,136; 5,082,830; 5,112,963; 5,214,136; 5, 245,022; 5,254,469; 5,258,506; 5,262,536; 5,272,250; 5,292,873; 5,317,098; 5,371,241, 5,391, 723; 5,416,203, 5,451,463; 5,510,475; 5,512,667; 5,514,785; 5, 565,552; 5,567,810; 5,574,142; 5,585,481; 5,587,371 ; 5,595,726; 5,597,696; 5,599,923; 5,599, 928 and 5,688,941.

These moieties or conjugates can include conjugate groups covalently bound to functional groups such as primary or secondary hydroxyl groups. Conjugate groups of the invention include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugate groups include cholesterols, lipids, phospholipids, biotin, phenazine, folate, phenanthridine, anthraquinone, acridine, fluoresceins, rhodamines, coumarins, and dyes. Groups that enhance the pharmacodynamic properties, in the context of this invention, include groups that improve uptake, enhance resistance to degradation, and/or strengthen sequence-specific hybridization with the target nucleic acid. Groups that enhance the pharmacokinetic properties, in the context of this invention, include groups that improve uptake, distribution, metabolism, or excretion of the compounds of the present invention. Representative conjugate groups are disclosed in International Patent Application No. PCT/US92/09196, filed Oct. 23, 1992, and U.S. Patent No. 6,287,860. Conjugate moieties include, but are not limited to, lipid moieties such as a cholesterol moiety, cholic acid, a thioether, e.g., hexyl-5- tritylthiol, a thiocholesterol, an aliphatic chain, e.g., dodecandiol or undecyl residues, a phospholipid, e.g., di-hexadecyl-rac-glycerol or triethylammonium 1,2-di-O-hexadecyl-rac- glycero-3-H-phosphonate, a polyamine or a polyethylene glycol chain, or adamantane acetic acid, a palmityl moiety, or an octadecylamine or hexylamino-carbonyl-oxy cholesterol moiety. See, e.g., U.S. Patent Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

The inhibitory nucleic acids useful in the present methods are sufficiently complementary to the target miRNA, i.e., hybridize sufficiently well and with sufficient specificity, to give the desired effect. "Complementary" refers to the capacity for pairing, through hydrogen bonding, between two sequences comprising naturally or non-naturally occurring bases or analogs thereof. For example, if a base at one position of an inhibitory nucleic acid is capable of hydrogen bonding with a base at the corresponding position of a miRNA, then the bases are considered to be complementary to each other at that position. In some embodiments, 100% complementarity is not required. In some embodiments, 100% complementarity is required. Routine methods can be used to design an inhibitory nucleic acid that binds to the target sequence with sufficient specificity.

While the specific sequences of certain exemplary target segments are set forth herein, one of skill in the art will recognize that these serve to illustrate and describe particular embodiments within the scope of the present invention. Additional target segments are readily identifiable by one having ordinary skill in the art in view of this disclosure. Target segments of 5, 6, 7, 8, 9, 10 or more nucleotides in length comprising a stretch of at least five (5) consecutive nucleotides within the seed sequence, or immediately adjacent thereto, are considered to be suitable for targeting as well. In some embodiments, target segments can include sequences that comprise at least the 5 consecutive nucleotides from the 5 '-terminus of one of the seed sequence (the remaining nucleotides being a consecutive stretch of the same RNA beginning immediately upstream of the 5 '-terminus of the seed sequence and continuing until the inhibitory nucleic acid contains about 5 to about 30 nucleotides). In some embodiments, target segments are represented by RNA sequences that comprise at least the 5 consecutive nucleotides from the 3 '- terminus of one of the seed sequence (the remaining nucleotides being a consecutive stretch of the same miRNA beginning immediately downstream of the 3 '-terminus of the target segment and continuing until the inhibitory nucleic acid contains about 5 to about 30 nucleotides). One having skill in the art armed with the sequences provided herein will be able, without undue experimentation, to identify further preferred regions to target. In some embodiments, an inhibitory nucleic acid contain a sequence that is complementary to at least 5, 6, 7, 8, 9, 10, 11, 12, 13, 14, 15, 16, 17, 18, 19, 20, 21, 22, 23, 24, or 25 contiguous nucleotides present in the target (e.g., the target miRNA, e.g., mature or precursor miR-181, or the target mRNA).

Once one or more target regions, segments or sites have been identified, inhibitory nucleic acid compounds are chosen that are sufficiently complementary to the target, i.e., that hybridize sufficiently well and with sufficient specificity (i.e., do not substantially bind to other non-target RNAs), to give the desired effect. In the context of this invention, hybridization means hydrogen bonding, which may be Watson-Crick, Hoogsteen or reversed Hoogsteen hydrogen bonding, between complementary nucleoside or nucleotide bases. For example, adenine and thymine are complementary nucleobases which pair through the formation of hydrogen bonds. Complementary, as used herein, refers to the capacity for precise pairing between two nucleotides. For example, if a nucleotide at a certain position of an oligonucleotide is capable of hydrogen bonding with a nucleotide at the same position of a miRNA molecule or an mRNA molecule, then the inhibitory nucleic acid and the miRNA or mRNA are considered to be complementary to each other at that position. The inhibitory nucleic acids and the miRNA or mRNA are complementary to each other when a sufficient number of corresponding positions in each molecule are occupied by nucleotides which can hydrogen bond with each other. Thus, "specifically hybridizable" and "complementary" are terms which are used to indicate a sufficient degree of complementarity or precise pairing such that stable and specific binding occurs between the inhibitory nucleic acid and the miRNA target. For example, if a base at one position of an inhibitory nucleic acid is capable of hydrogen bonding with a base at the corresponding position of a miRNA or a mRNA, then the bases are considered to be complementary to each other at that position. 100% complementarity is not required.

It is understood in the art that a complementary nucleic acid sequence need not be 100% complementary to that of its target nucleic acid to be specifically hybridizable. A complementary nucleic acid sequence for purposes of the present methods is specifically hybridisable when binding of the sequence to the target miRNA or mRNA molecule interferes with the normal function of the target miRNA or mRNA to cause a loss of expression or activity, and there is a sufficient degree of complementarity to avoid non-specific binding of the sequence to non-target RNA sequences under conditions in which specific binding is desired, e.g., under physiological conditions in the case of in vivo assays or therapeutic treatment, and in the case of in vitro assays, under conditions in which the assays are performed under suitable conditions of stringency. For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and more preferably at least about 50% formamide. Stringent temperature conditions will ordinarily include temperatures of at least about 30° C, more preferably of at least about 37° C, and most preferably of at least about 42° C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred embodiment, hybridization will occur at 30° C in 750 mM NaCl, 75 mM trisodium citrate, and 1% SDS. In a more preferred embodiment, hybridization will occur at 37° C in 500 mM NaCl, 50 mM trisodium citrate, 1% SDS, 35% formamide, and 100 µg/ml denatured salmon sperm DNA (ssDNA). In a most preferred embodiment, hybridization will occur at 42° C in 250 mM NaCl, 25 mM trisodium citrate, 1% SDS, 50% formamide, and 200 µg/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25° C, more preferably of at least about 42° C, and even more preferably of at least about 68° C. In a preferred embodiment, wash steps will occur at 25° C in 30 mM NaCl, 3 mM trisodium citrate, and 0.1% SDS. In a more preferred embodiment, wash steps will occur at 42° C in 15 mM NaCl, 1.5 mM trisodium citrate, and 0.1% SDS. In a more preferred embodiment, wash steps will occur at 68° C in 15 mM NaCl, 1.5 mM trisodium citrate, and 0.1% SDS. Additional variations on these conditions will be readily apparent to those skilled in the art. Hybridization techniques are well known to those skilled in the art and are described, for example, in {Benton, 1977; Grunstein, 1975 and in Ausubel et al. (Current Protocols in Molecular Biology, Wiley Interscience, New York, 2001); Berger and Kimmel (Guide to Molecular Cloning Techniques, 1987, Academic Press, New York); and Sambrook et al, Molecular Cloning: A Laboratory Manual, Cold Spring Harbor Laboratory Press, New York.

In general, the inhibitory nucleic acids useful in the methods described herein have at least 80% sequence complementarity to a target region within the target nucleic acid, e.g., 90%, 95%, or 100% sequence complementarity to the target region within an miRNA. For example, an antisense compound in which 18 of 20 nucleobases of the antisense oligonucleotide are complementary, and would therefore specifically hybridize, to a target region would represent 90 percent complementarity. Percent complementarity of an inhibitory nucleic acid with a region of a target nucleic acid can be determined routinely using basic local alignment search tools (BLAST programs) (Altschul, 1990; Zhang, 1997). Antisense and other compounds of the invention that hybridize to an miRNA or a mRNA are identified through routine experimentation. In general the inhibitory nucleic acids must retain specificity for their target, i.e., must not directly bind to, or directly significantly affect expression levels of, transcripts other than the intended target wherein said intended target may be one or more than one miRNA belonging to the same family or to different families.

For further disclosure regarding inhibitory nucleic acids, please see US2010/0317718 (antisense oligos); US2010/0249052 (double-stranded ribonucleic acid (dsRNA)); US2009/0181914 and US2010/0234451 (LNAs); US2007/0191294 (siRNA analogues); US2008/0249039 (modified siRNA); and WO2010/129746 and WO2010/040112 (inhibitory nucleic acids).

### Antisense

Antisense oligonucleotides are typically designed to block expression of a DNA or RNA target by binding to the target and halting expression at the level of transcription, translation, or splicing. Antisense oligonucleotides of the present invention are complementary nucleic acid sequences designed to hybridize under stringent conditions to the target microRNA or the target inflammatory marker mRNA. Thus, oligonucleotides are chosen that are sufficiently complementary to the target, i.e., that hybridize sufficiently well and with sufficient specificity, to give the desired effect.

### Modified Bases/Locked Nucleic Acids (LNAs)

In some embodiments, the inhibitory nucleic acids used in the methods described herein comprise one or more modified bonds or bases. Modified bases include phosphorothioate, methylphosphonate, peptide nucleic acids, or locked nucleic acid (LNA) molecules.

LNAs are modified ribonucleotides that contain an extra bridge between the 2' and 4' carbons of the ribose sugar moiety resulting in a "locked" conformation that confers enhanced thermal stability. LNAs therefore comprise a class of bicyclic RNA analogs in which the furanose ring in the sugar-phosphate backbone is chemically locked in an RNA mimicking N-type (C3'-endo) conformation by the introduction of a 2'-O, 4'-C methylene bridge. Said modifications increase nuclease resistance and binding affinity of the antisense oligonucleotide to their cognate miRNAs.

Preferably, the modified nucleotides are locked nucleic acid molecules, including [alpha]-L-LNAs. LNAs comprise ribonucleic acid analogues wherein the ribose ring is "locked" by a methylene bridge between the 2'-oxgygen and the 4'-carbon - i.e., oligonucleotides containing at least one LNA monomer, that is, one 2'-0,4'-C-methylene-D-ribofuranosyl nucleotide. LNA bases form standard Watson-Crick base pairs but the locked configuration increases the rate and stability of the base pairing reaction (Jepsen et al, 2004). LNAs also have increased affinity to base pair with RNA as compared to DNA. These properties render LNAs especially useful as probes for fluorescence in situ hybridization (FISH) and comparative genomic hybridization, as knockdown tools for miRNAs, and as antisense oligonucleotides to target mRNAs or other RNAs, e.g., miRNAs and mRNAs as described herein.

The LNA molecules can include molecules comprising 10-30, e.g., 12-24, e.g., 12, 13, 14, 15, 16, 17, 18, 19, 20, 21, 22, 23, 24, 25, 26, 27, 28, 29, or 30 nucleotides in each strand, wherein one of the strands is substantially identical, e.g., at least 75%, 80%, 85%, 90%, 91%, 92%, 93%, 94%, 95%, 96%, 97%, 98%, or 99%, or 100% identical, e.g., having 3, 2, 1, or 0 mismatched nucleotide(s), to a target region in the miRNA or the mRNA. The LNA molecules can be chemically synthesized using methods known in the art.

The LNA molecules can be designed using any method known in the art; a number of algorithms are known, and are commercially available (e.g., on the internet, for example at exiqon.com). See, e.g., (Levin et al, 2006; McTigue et al, 2004; You et al, 2006). For example, "gene walk" methods, similar to those used to design antisense oligos, can be used to optimize the inhibitory activity of the LNA; for example, a series of oligonucleotides of 10-30 nucleotides spanning the length of a target miRNA or mRNA can be prepared, followed by testing for activity.

Optionally, gaps, e.g., of 5-10 nucleotides or more, can be left between the LNAs to reduce the number of oligonucleotides synthesized and tested. GC content is preferably between about 30-60%. General guidelines for designing LNAs are known in the art; for example, LNA sequences will bind very tightly to other LNA sequences, so it is preferable to avoid significant complementarity within an LNA. Contiguous runs of three or more Gs or Cs, or more than four LNA residues, should be avoided where possible (for example, it may not be possible with very short (e.g., about 9-10 nt) oligonucleotides). In some embodiments, the LNAs are xylo-LNAs.

In some embodiments, the LNA molecules can be designed to target a specific region of the miPvNA. For example, a specific functional region can be targeted, e.g., a region comprising a seed sequence. Alternatively or in addition, highly conserved regions can be targeted, e.g., regions identified by aligning sequences from disparate species such as primate (e.g., human) and rodent (e.g., mouse) and looking for regions with high degrees of identity. Percent identity can be determined routinely using basic local alignment search tools (BLAST programs) (Altschul et al, 1990; Zhang & Madden, 1997) (Altschul et al, J. Mol. Biol. 215:403-410, 1990; Zhang and Madden, Genome Res. 7:649-656, 1997), e.g., using the default parameters.

For additional information regarding LNAs see U.S. Patent Nos. 6,268,490; 6,734,291; 6,770,748; 6,794,499; 7,034,133; 7,053,207; 7,060,809; 7,084,125; and 7,572,582; and U.S. Pre-Grant Pub. Nos. 2010/0267018; 2010/0261175; and 2010/0035968; Koshkin et al, Tetrahedron 54:3607-3630, 1998; Obika et al, Tetrahedron Lett. 39:5401-5404, 1998; Jepsen et al, Oligonucleotides 14:130-146 , 2004; Kauppinen et al, Drug Disc. Today 2(3):287-290, 2005; and Ponting et al, Cell 136(4): 629-641, 2009, "Locked nucleic acids" (LNAs), J Am Chem Soc. 2002 May 29;124(21):5974-82."Locked nucleic acid (LNA) recognition of RNA: NMR solution structures of LNA:RNA hybrids".Petersen M1, Bondensgaard K, Wengel J, Jacobsen JP ; Handb Exp Pharmacol. 2006;(173):405-22."Locked nucleic acid: high-affinity targeting of complementary RNA for RNomics"Kauppinen S1, Vester B, Wengel J.; RNA Biol. 2009 Jul-Aug;6(3):321-3. Epub 2009 Jul 18.

"Locked nucleic acid as a novel class of therapeutic agents."Veedu RN1, Wengel J.Chem Biodivers. 2010 Mar;7(3):536-42. doi: 10.1002/cbdv.200900343. "Locked nucleic acids: promising nucleic acid analogs for therapeutic applications."Veedu RN1, Wengel J.Curr Opin Mol Ther. 2001 Jun;3(3):239-43.; "Locked nucleic acids: a promising molecular family for gene-function analysis and antisense drug development".Orum H1, Wengel J.and references cited therein.

See also USSN 61/412,862.

Alternatively, the antisense oligonucleotides may comprise peptide nucleic acids (PNAs), which contain a peptide-based backbone rather than a sugar-phosphate backbone, e.g. the deoxyribose phosphate backbone is replaced by a polyamide chain of N-(2.aminoethyl)-glycine units (Fabani, M.M.; Abreu-Goodger, C.;Williams, D.; Lyons, P.A.; Torres, A.G.; Smith, K.G.; Enright, A.J.; Gait, M.J.;Vigorito, E. Efficient inhibition of miR-155 function in vivo by peptide nucleic acids. Nucleic Acids Res. 2010,38, 4466-4475). The antisense oligonucleotides may contain one or more chemical modifications, including, but are not limited to, sugar modifications, such as 2'-O-alkyl (e.g. 2'-O-methyl, 2'-O-methoxyethyl), 2'-fluoro, and 4' thio modifications, and backbone modifications, such as one or more phosphorothioate, morpholino, or phosphonocarboxylate linkages (see, for example, U.S. Pat. Nos. 6,693,187 and 7,067,641). In some embodiments, suitable antisense oligonucleotides are 2'-O-methoxyethyl "gapmers" which contain 2'-O-methoxyethyl-modified ribonucleotides on both 5' and 3' ends with at least ten deoxyribonucleotides in the center. These "gapmers" are capable of triggering RNase H-dependent degradation mechanisms of RNA targets. Other modifications of antisense oligonucleotides to enhance stability and improve efficacy, such as those described in U.S. Pat. No. 6,838,283, are known in the art and are suitable for use in the methods of the invention. Further antisense oligonucleotides of the invention include Small RNA zippers

Antisense oligonucleotides, useful for inhibiting the activity of micro RNAs, are about 19 to about 25 nucleotides in length. Antisense oligonucleotides may comprise a sequence that is at least partially complementary to a mature and/or precursor miRNA sequence, e.g., at least about 75%, 80%, 85%, 90%, 91%, 92%, 93%, 94%, 95%, 96%, 97%, 98%, or 99% complementary to a mature and/or precursor miRNA sequence. In some embodiments, the antisense oligonucleotide may be substantially complementary to a mature and/or precursor miRNA sequence, that is at least about 95%, 96%, 97%, 98%), or 99% complementary to a target polynucleotide sequence. In one embodiment, the antisense oligonucleotide comprises a sequence that is 100% complementary to a mature and/or precursor miRNA sequence.

Antisense nucleotides of the present invention may target a sequence of miR-181, including but not limited to, miR-181a (SEQ ID NO. 1), miR-181b (SEQ ID NO. 2), miR-181c (SEQ ID NO. 3), or miR-181d (SEQ ID NO. 4) or a combination thereof.

Preferred antisense nucleotides of the invention are locked nucleic acids that comprise a sequence that is at least partially complementary to a mature miR-181a sequence of SEQ ID NO 1, or 3, e.g. at least 75%, 80%, 85%, 90%, 91%, 92%, 93%, 94%, 95%, 96%, 97%, 98%, or 99% complementary to said miRNA sequence and targets miRNA 181a and/or mir-181 c.

Further preferred antisense nucleotides of the invention are locked nucleic acids that comprise a sequence that is at least partially complementary to a mature miR-181a sequence of SEQ ID NO 2 or 4 , e.g. at least 75%, 80%, 85%, 90%, 91%, 92%, 93%, 94%, 95%, 96%, 97%, 98%, or 99% complementary to said miRNA sequence and targets miRNA 181b and/or mir-181 d.

Particularly preferred locked nucleic acids comprise SEQ ID No. 1, 2, 3 or 4 and are modified at one or more nucleotides

A preferred embodiment of the invention comprises a combination of LNAs that target mir-181a/c and miR-181b/d , a particularly preferred embodiment comprises a combination of LNA A (SEQ ID NO. 6) and LNA B (SEQ ID NO. 7) as described herein.

### Antagomirs

In some embodiments, the antisense is an antagomir (Krutzfeldt, J.; Rajewsky, N.; Braich, R.; Rajeev, K.G.; Tuschl, T.; Manoharan, M.; Stoffel, M. Silencing of microRNAs in vivo with 'antagomirs'. Nature 2005, 438, 685-689.). Antagomirs are chemically- modified antisense oligonucleotides that target a microRNA (e.g., target hsa-miR-181). For example, an antagomir for use in the methods described herein can include a nucleotide sequence sufficiently complementary to hybridize to a miRNA target sequence of about 12 to 25 nucleotides, preferably about 15 to 23 nucleotides. Suitably, antagomirs may be single-stranded, chemically-modified ribonucleotides that are at least partially complementary to the miRNA sequence. Antagomirs may comprise one or more modified nucleotides, such as 2'-O-methyl-sugar modifications.

In general, antagomirs include a cholesterol moiety, e.g., at the 3'-end. In some embodiments, antagomirs have various modifications for RNase protection and pharmacologic properties such as enhanced tissue and cellular uptake. For example, in addition to the modifications discussed above for antisense oligos, an antagomir can have one or more of complete or partial 2'-0-methylation of sugar and/or a phosphorothioate backbone. Phosphorothioate modifications provide protection against RNase activity and their lipophilicity contributes to enhanced tissue uptake. In some embodiments, the antagomir can include six phosphorothioate backbone modifications; two phosphorothioates are located at the 5 '-end and four at the 3'-end. See, e.g., Krutzfeldt et al, Nature 438:685-689, 2005; Czech, N. Engl. J. Med. 354: 1194-1195, 2006; Robertson et al, Silence 1 : 10, 2010; Marquez and McCaffrey, Human Gene Ther. 19(I):27-38, 2008; van Rooij et al, Circ. Res. 103(9):919-928, 2008; and Liu et al, Int. J. Mol. Sci. 9:978-999, 2008.

Antagomirs useful in the present methods can also be modified with respect to their length or otherwise the number of nucleotides making up the antagomir. In general, the antagomirs are about 20 - 21 nucleotides in length for optimal function, as this size matches the size of most mature microRNAs. The antagomirs must retain specificity for their target, i.e., must not directly bind to, or directly significantly affect expression levels of, transcripts other than the intended target.

Antagomirs may be at least about 75%, 80%, 85%, 90%, 91%, 92%, 93%, 94%, 95%, 96%, 97%, 98%, or 99% complementary to a mature and/or precursor miRNA sequence. In some embodiments, the antagomir may be substantially complementary to a mature miRNA sequence, that is at least about 95%, 96%, 97%, 98%, or 99% complementary to a target polynucleotide sequence. In other embodiments, the antagomirs are 100% complementary to the mature miRNA sequence.

In some embodiments, the inhibitory nucleic acid is locked and includes a cholesterol moiety (e.g., a locked antagomir).

### siRNA

In some embodiments, the nucleic acid sequence that is complementary to a target miRNA or a target mRNA can be an interfering RNA, including but not limited to a small interfering RNA ("siRNA") or a small hairpin RNA ("shRNA"). Methods for constructing interfering RNAs are well known in the art. For example, the interfering RNA can be assembled from two separate oligonucleotides, where one strand is the sense strand and the other is the antisense strand, wherein the antisense and sense strands are self-complementary (i.e., each strand comprises nucleotide sequence that is complementary to nucleotide sequence in the other strand; such as where the antisense strand and sense strand form a duplex or double stranded structure); the antisense strand comprises nucleotide sequence that is complementary to a nucleotide sequence in a target nucleic acid molecule or a portion thereof (i.e., an undesired gene) and the sense strand comprises nucleotide sequence corresponding to the target nucleic acid sequence or a portion thereof. Alternatively, interfering RNA is assembled from a single oligonucleotide, where the self-complementary sense and antisense regions are linked by means of nucleic acid based or non-nucleic acid-based linker(s). The interfering RNA can be a polynucleotide with a duplex, asymmetric duplex, hairpin or asymmetric hairpin secondary structure, having self-complementary sense and antisense regions, wherein the antisense region comprises a nucleotide sequence that is complementary to nucleotide sequence in a separate target nucleic acid molecule or a portion thereof and the sense region having nucleotide sequence corresponding to the target nucleic acid sequence or a portion thereof. The interfering can be a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either in vivo or in vitro to generate an active siRNA molecule capable of mediating RNA interference.

In some embodiments, the interfering RNA coding region encodes a self-complementary RNA molecule having a sense region, an antisense region and a loop region. Such an RNA molecule when expressed desirably forms a "hairpin" structure and is referred to herein as an "shRNA." The loop region is generally between about 2 and about 10 nucleotides in length. In some embodiments, the loop region is from about 6 to about 9 nucleotides in length. In some embodiments, the sense region and the antisense region are between about 15 and about 20 nucleotides in length. Following post-transcriptional processing, the small hairpin RNA is converted into a siRNA by a cleavage event mediated by the enzyme Dicer, which is a member of the RNase III family. The siRNA is then capable of inhibiting the expression of a gene with which it shares homology. For details, see Brummelkamp et al, Science 296:550-553, 2002; Lee et al, Nature BiotechnoL, 20, 500-505, 2002; Miyagishi and Taira, Nature Biotechnol. 20:497-500, 2002; Paddison et al, Genes & Dev. 16:948-958, 2002; Paul, Nature Biotechnol. 20, 505-508, 2002; Sui, Proc. Natl. Acad. Sci. U.S.A., 99(6):5515-5520, 2002; Yu et al, Proc. Natl. Acad. Sci. U.S.A. 99:6047-6052, 2002.

### miRNA Sponges

MiRNA sponges or decoys (Ebert et al., Nat Methods. 2007 Sep;4(9):721-6. Epub 2007 Aug 12.. Generation of miRNA sponge constructs. Kluiver, J., Slezak-Prochazka, I., Smigielska-Czepiel, K., Halsema, N., Kroesen, B.J., and Anke van den Berg. Methods - 23 July 2012. E-pub ahead of print.; F.C. Tay, et al., Using artificial microRNA sponges to achieve microRNA loss-of-function in cancer cells, Adv. Drug Deliv. Rev. (2014))are in vivo expressed transcripts that contain multiple high affinity miRNA antisense binding sites (MBS)) that mimic those found in mRNAs and complementary to a miRNA to be targeted; these transcripts can efficiently sequester specific miRNAs and, thereby, prevent their binding to endogenous target genes. These binding sites are usually tandem repeats of identical sites designed to target either single specific miRNAs or miRNA family members sharing the same seed region; considering that the interaction between microRNA and target is dependent on base-pairing in the seed region (positions 2-8 of the microRNA), a decoy target should interact with all members of a microRNA seed family. In so doing, it should better inhibit functional classes of microRNAs than do antisense oligonucleotides, which are thought to block single microRNA sequences.

Design of miRNA sponges or decoys have been disclosed for instance in "Ebert MS, Neilson JR, Sharp PA (2007) MicroRNA sponges: competitive inhibitors of small RNAs in mammalian cells. Nat Methods 4: 721-72" and "Kluiver J, Slezak-Prochazka I, Smigielska-Czepiel K, Halsema N, Kroesen BJ, van den Berg A (2012) Generation of miRNA sponge constructs. Methods 58: 113-117"

Sponge sequences typically consist of multiple miRNA biding sites (MBS) separated by 4-6 nucleotides- long spacer sequences. MBSs are either perfectly anti-sense or contain a bulge at the central position. However, perfectly base-paired miRNA-sponge interaction is vulnerable to Ago2-mediated cleavage, therefore MBSs containing 4 nucleotide central bulge are reported to be more effective. MiRNA sponges offer an advantage of inhibiting all seed family members and also when multiple MBS are introduced, they can be used for inhibition of whole miRNA clusters. Given the advantages over other means of miRNA inhibition, miRNA sponges have the potential for use in disease treatment (Ebert MS et al., Nat Methods 2007, Ebert MS and Sharp PA, RNA 2010, Liu Z et al., Int J Mol Sci 2008, KluiverJ et al., Methods 2012, Kluiver et al., PLoS One 2012). miRNA sponge construct design and off-targets testing may be performed using a web-based tool MiRNAsong: microRNA SpONge Generator and tester.

Suitably, a Sponge is composed of a high affinity miRNA antisense binding site (MBS) which includes a sequence complementary to the target miRNA, and a "bulge" region, i.e. a mismatched region in nucleotides 9 to 12 of the MBS sequence. Suitably, the complementary region is at least 75 %, 80 %, 85 %, 90%, 95%, 99% or 100% identical to the target miRNA. Most preferably, the complementary region is at least 80% identical to the target miRNA. Suitably, sequence complementary to the target miRNA sequence includes the SEED sequence, e.g. the sequence within a given miRNA which is essential to the target transcript recognition, and the "bulge" region is outside said Seed sequence. Each MBS sequence (MBS SEQ) is the same length as the mature target miRNA. Each MBS SEQ is repeated several times, between 6 and 24 times, preferably between 6 and 12 times, most preferably 6 times. Suitably, MBS SEQs separated from each other by a "spacer" sequence consisting of 4to 6 nucleotides, preferably 4 nucleotides.

Suitably, the miRNA sponge of the invention may comprise two or more MBS sequences targeting two or more miRNAs, preferably two or more miRNAs within the same family, alternatively two or more miRNAs from different families.

Suitably, a miRNA Sponge comprising an MBS sequence complementary to a given Seed sequences inhibits all miRNAs carrying said Seed sequence. Suitably, a family of miRNAs comprises miRNAs with highly related sequences, suitably, miRNAs from the same family share the same Seed sequence.

Inhibitors of the invention are composed of two MBS sequences separated by spacer sequences; suitably, the two MBS sequences may target the same or different miRNAs.

For instance, the miRNAs inhibitor may have the following formula:

(X1sX2sX3)n₁S(X1sX2sX3)n₂

Wherein
X1, X2 and X3 are independently chosen from
   - an MBS sequence complementary to miR-181a and/or miR-181c, preferably modified from nucleotide 9 to nucleotide 12 to create a bulge or
   - an MBS sequence complementary to miR-181b and/or miR-181d, preferably modified from nucleotide 9 to nucleotide 12 to create a bulge;
X3 may be present or absent;
s is a Spacer sequence of 4 to 6 nucleotides, preferably 4 nucleotides;
S2 is a central Spacer sequence of 4 to 6 nucleotides, preferably 6 nucleotides, preferably corresponding to a restriction enzyme site, preferably a site for the Xbal restriction enzyme
n₁ and n₂ is a number independently selected from 3, 6 and 24, preferably between 3, 6 and 12, most preferably n is 3, n₁ and n₂ may be identical or different.

Preferred miRNAs inhibitors of the invention are composed of two MBS sequences separated by spacer sequences with the following formula:

(AsB)n₁S2(AsB)n₂

wherein:
A is an MBS sequence complementary to miR-181a and/or miR-181c, preferably modified from nucleotide 9 to nucleotide 12 to create a bulge;
B is an MBS sequence complementary to miR-181b and/or miR-181d, preferably modified from nucleotide 9 to nucleotide 12 to create a bulge;
s is a Spacer sequence of 4 to 6 nucleotides, preferably 4 nucleotides;
S2 is a central Spacer sequence of 4 to 6 nucleotides, preferably 6 nucleotides, preferably corresponding to a restriction enzyme site, preferably a site for the Xbal restriction enzyme
n₁ and n₂ is a number independently selected from 3, 6 and 24, preferably between 3, 6 and 12, most preferably n is 3, n₁ and n₂ may be identical or different .

Preferably X1, X2, X3, A is 3' TTGTAAGTVHDHACAGCCACTCA 5'
wherein according to IUPAC nomenclature:

| | |
|---|---|
| V= | A or C or G |
| H= | A or C or T |
| D= | A or G or T |

More preferably X1, X2, X3, B is 3' TTGTAAGTBBDHACAGCCACCCA 5'
wherein according to IUPAC nomenclature

| | |
|---|---|
| B= | C or G or T |
| D= | A or G or T |
| H= | A or C or T |

Still preferably n₁ = 3 and n₂ = 3

Preferably S consists of 4 nucleotides, preferably S= tagc.

Still preferably S2 consists of 6 nucleotides, preferably S2 = Tctaga

Suitably, in some embodiments, inhibitors of miR-181 are chemically modified antisense oligonucleotides, suitably LNAs, RNA inhibitors e.g. antagomirs, or miRNA sponges comprising a sequence that is substantially complementary to a mature sequence of miR-181a and/or miR-181b or a combination thereof.

As used herein, "substantially complementary" refers to a sequence that is at least about 95%, 96%, 97%, 98%, 99%, or 100% complementary to a target polynucleotide sequence (e.g., mature or precursor miRNA sequence).

Antisense oligonucleotides may comprise a sequence that is substantially complementary to a precursor miRNA sequence (pre-miRNA). The antisense oligonucleotide comprises a sequence that is substantially complementary to a sequence located outside the stem-loop region of the pre-miRNA sequence. Antisense oligonucleotides of the present invention are suitably complementary to the mature miRNA sequence.

The target RNA cleavage reaction guided by siRNAs is highly sequence specific. In general, siRNA containing a nucleotide sequences identical to a portion of the target nucleic acid (i.e., a target region comprising the seed sequence of a target miRNA or mRNA) are preferred for inhibition. However, 100% sequence identity between the siRNA and the target gene is not required to practice the present invention. Thus, the invention has the advantage of being able to tolerate sequence variations that might be expected due to genetic mutation, strain polymorphism, or evolutionary divergence. For example, siRNA sequences with insertions, deletions, and single point mutations relative to the target sequence have also been found to be effective for inhibition. Alternatively, siRNA sequences with nucleotide analog substitutions or insertions can be effective for inhibition. In general, the siRNAs must retain specificity for their target, i.e., must not directly bind to, or directly significantly affect expression levels of, transcripts other than the intended target.

"Therapeutically effective dose or amount" of a miRNA, miRNA mimic, or miRNA inhibitor is intended an amount that, when administered as described herein, brings about a positive therapeutic response, such as increase of mRNA levels of specific miR-181 targets, amelioration of mitochondrial function and/or increased number of mitochondria, reduction of oxidative stress and cell death with an amelioration of cell survival.

### Ribozymes

Trans-cleaving enzymatic nucleic acid molecules can also be used; they have shown promise as therapeutic agents for human disease (Usman & McSwiggen, Ann. Rep. Med. Chem. 30:285-294, 1995; Christoffersen and Marr, J. Med. Chem. 38:2023-2037, 1995). Enzymatic nucleic acid molecules can be designed to cleave specific miRNA or mRNA targets within the background of cellular RNA. Such a cleavage event renders the miRNA or mRNA nonfunctional.

In general, enzymatic nucleic acids with RNA cleaving activity act by first binding to a target RNA. Such binding occurs through the target binding portion of an enzymatic nucleic acid which is held in close proximity to an enzymatic portion of the molecule that acts to cleave the target RNA. Thus, the enzymatic nucleic acid first recognizes and then binds a target RNA through complementary base pairing, and once bound to the correct site, acts enzymatically to cut the target RNA. Strategic cleavage of such a target RNA will destroy its activity. After an enzymatic nucleic acid has bound and cleaved its RNA target, it is released from that RNA to search for another target and can repeatedly bind and cleave new targets.

Several approaches such as in vitro selection (evolution) strategies (Orgel, Proc. R. Soc. London, B 205:435, 1979) have been used to evolve new nucleic acid catalysts capable of catalyzing a variety of reactions, such as cleavage and ligation of phosphodiester linkages and amide linkages, (Joyce, Gene, 82, 83-87, 1989; Beaudry et al, Science 257, 635-641, 1992; Joyce, Scientific American 267, 90-97, 1992; Breaker et al, TIBTECH 12:268, 1994; Barrel et al, Science 261 : 1411-1418, 1993; Szostak, TIBS 17, 89-93, 1993; Kumar et al, FASEB J., 9: 1183, 1995; Breaker, Curr. Op. Biotech., 1 :442, 1996). The development of ribozymes that are optimal for catalytic activity would contribute significantly to any strategy that employs RNA- cleaving ribozymes for the purpose of regulating gene expression. The hammerhead ribozyme, for example, functions with a catalytic rate (kcat) of about 1 min⁻¹ in the presence of saturating (10 mM) concentrations of Mg²⁺ cofactor. An artificial "RNA ligase" ribozyme has been shown to catalyze the corresponding self-modification reaction with a rate of about 100 min⁻¹. In addition, it is known that certain modified hammerhead ribozymes that have substrate binding arms made of DNA catalyze RNA cleavage with multiple turn-over rates that approach 100 min⁻¹.

### Making and Using Inhibitory Nucleic Acids

The nucleic acid sequences used to practice the methods described herein, whether RNA, cDNA, genomic DNA, vectors, viruses or hybrids thereof, can be isolated from a variety of sources, genetically engineered, amplified, and/or expressed/generated recombinantly.

Recombinant nucleic acid sequences can be individually isolated or cloned and tested for a desired activity. Any recombinant expression system can be used, including e.g., in vitro, bacterial, fungal, mammalian, yeast, insect, or plant cell expression systems. Nucleic acid sequences of the invention (e.g., any of the inhibitory nucleic acids or sense nucleic acids described herein) can be inserted into delivery vectors and expressed from transcription units within the vectors. The recombinant vectors can be DNA plasmids or viral vectors. Generation of the vector construct can be accomplished using any suitable genetic engineering techniques well known in the art, including, without limitation, the standard techniques of PCR, oligonucleotide synthesis, restriction endonuclease digestion, ligation, transformation, plasmid purification, and DNA sequencing, for example as described in Sambrook et al. Molecular Cloning: A Laboratory Manual. (1989)), Coffin et al. (Retroviruses. (1997)) and "RNA Viruses: A Practical Approach" (Alan J. Cann, Ed., Oxford University Press, (2000)).

As will be apparent to one of ordinary skill in the art, a variety of suitable vectors are available for transferring nucleic acids of the invention into cells. The selection of an appropriate vector to deliver nucleic acids and optimization of the conditions for insertion of the selected expression vector into the cell, are within the scope of one of ordinary skill in the art without the need for undue experimentation. Viral vectors comprise a nucleotide sequence having sequences for the production of recombinant virus in a packaging cell. Viral vectors expressing nucleic acids of the invention can be constructed based on viral backbones including, but not limited to, a retrovirus, lentivirus, herpes virus, adenovirus, adeno-associated virus, pox virus, or alphavirus. The recombinant vectors (e.g., viral vectors) capable of expressing the nucleic acids of the invention can be delivered as described herein, and persist in target cells (e.g., stable transformants). For example, such recombinant vectors (e.g., a recombinant vector that results in the expression of an antisense oligomer that is complementary to hsa-miR-155) can be administered into (e.g., injection or infusion into) the cerebrospinal fluid of the subject (e.g., intracranial injection, intraparenchymal injection, intraventricular injection, and intrathecal injection, see, e.g., Bergen et al, Pharmaceutical Res. 25:983-998, 2007). A number of exemplary recombinant viral vectors that can be used to express any of the nucleic acids described herein are also described in Bergen et al. {supra). Additional examples of recombinant viral vectors are known in the art.

The nucleic acids provided herein (e.g., the inhibitory nucleic acids) can be further be complexed with one or more cationic polymers (e.g., poly-L-lysine and poly(ethylenimine), cationic lipids (e.g., l,2-dioleoyl-3-trimethylammonium propone (DOTAP), N-methyl-4-(dioleyl)methylpyridinium, and 3P-[N-(N',N'-dimethylaminoethane)-carbamoyl] cholesterol), and/or nanoparticles (e.g., cationic polybutyl cyanoacrylate nanoparticles, silica nanoparticles, or polyethylene glycol-based nanoparticles) prior to administration to the subject (e.g., injection or infusion into the cerebrospinal fluid of the subject). Additional examples of cationic polymers, cationic lipids, and nanoparticles for the therapeutic delivery of nucleic acids are known in the art. The therapeutic delivery of nucleic acids has also been shown to be achieved following intrathecal injection of polyethyleneimine/DNA complexes (Wang et al., Mol. Ther. 12:314-320, 2005). The methods for delivery of nucleic acids described herein are non-limiting. Additional methods for the therapeutic delivery of nucleic acids to a subject are known in the art.

In some embodiments, the inhibitory nucleic acids (e.g., one or more inhibitory nucleic acids targeting hsa-miR-155) can be administered systemically (e.g., intravenously, intaarterially, intramuscularly, subcutaneously, or intraperitoneally) or intrathecally (e.g., epidural administration). In some embodiments, the inhibitory nucleic acid is administered in a composition (e.g., complexed with) one or more cationic lipids. Non-limiting examples of cationic lipids that can be used to administer one or more inhibitory nucleic acids (e.g., any of the inhibitory nucleic acids described herein) include: Lipofectamine, the cationic lipid molecules described in WO 97/045069, and U.S. Patent Application Publication Nos. 2012/0021044, 2012/0015865, 2011/0305769, 2011/0262527, 2011/0229581, 2010/0305198, 2010/0203112, and 2010/0104629.Nucleic acid sequences used to practice this invention can be synthesized in vitro by well-known chemical synthesis techniques, as described in, e.g., Adams, J. Am. Chem. Soc. 105:661, 1983; Belousov, Nucleic Acids Res. 25:3440-3444, 1997; Frenkel, Free Radic. Biol. Med. 19:373-380, 1995; Blommers, Biochemistry 33:7886-7896, 1994; Narang, Meth. Enzymol. 68:90, 1994; Brown, Meth. Enzymol. 68: 109, 1979; Beaucage, Tetra. Lett. 22: 1859, 1981; and U.S. Patent No. 4,458,066.

Nucleic acid sequences of the invention can be stabilized against nucleolytic degradation such as by the incorporation of a modification, e.g., a nucleotide modification. For example, nucleic acid sequences of the invention include a phosphorothioate at least the first, second, or third internucleotide linkage at the 5' or 3' end of the nucleotide sequence. As another example, the nucleic acid sequence can include a 2'-modified nucleotide, e.g., a 2'-deoxy, 2'-deoxy-2'- fluoro, 2<*>-0-methyl, 2<*>-0-methoxyethyl (2<*>-0-MOE), 2<*>-0-aminopropyl (2<*>-0-AP), 2<*>-0-dimethylaminoethyl (2<*>-0-DMAOE), 2<*>-0-dimethylaminopropyl (2<*>-0-DMAP), 2<*>-0-dimethylaminoethyloxy ethyl (2'-0-DMAEOE), or 2'-0~N-methylacetamido (2'-0~NMA). As another example, the nucleic acid sequence can include at least one 2'-0-methyl-modified nucleotide, and in some embodiments, all of the nucleotides include a 2'-0-methyl modification. In some embodiments, the nucleic acids are "locked," i.e., comprise nucleic acid analogues in which the ribose ring is "locked" by a methylene bridge connecting the 2'-0 atom and the 4'-C atom (see, e.g., Kaupinnen et al, Drug Disc. Today 2(3):287-290, 2005; Koshkin et al, J. Am. Chem. Soc, 120(50): 13252-13253, 1998). For additional modifications see US 2010/0004320, US 2009/0298916, and US 2009/0143326.

Techniques for the manipulation of nucleic acids used to practice this invention, such as, e.g., subcloning, labeling probes (e.g., random-primer labeling using Klenow polymerase, nick translation, amplification), sequencing, hybridization, and the like are well described in the scientific and patent literature, see, e.g., Sambrook et al., Molecular Cloning; A Laboratory Manual 3d ed. (2001); Current Protocols in Molecular Biology, Ausubel et al, Eds. (John Wiley & Sons, Inc., New York 2010); Kriegler, Gene Transfer and Expression: A Laboratory Manual (1990); Laboratory Techniques In Biochemistry And Molecular Biology: Hybridization With Nucleic Acid Probes, Part I. Theory and Nucleic Acid Preparation, Tijssen, Ed. Elsevier, N.Y. (1993).

### Methods of Treatment

Also provided are methods of treating a mitochondrial disorder that include administering to a subject at least one (e.g., at least two, three, four, five, or six) agent (e.g., a nucleic acid) that decreases the level or activity of one or more (e.g., at least two, three, four, five, or six) of the microRNAs of SEQ ID No. 1, 2, 3 or 4 (e.g., an inhibitory nucleic acid, e.g., an antagomir).

In some embodiments, the subject is administered at least one inhibitory nucleic acid comprising a sequence that is complementary to a contiguous sequence present in miR-181 (e.g., a contiguous sequence present in mature or precursor hsa-miR-181). In non-limiting embodiments, the inhibitory nucleic acid can be an antisense oligonucleotide, a ribozyme, an siRNA, or an antagomir. In some embodiments, the at least one inhibitory nucleic acid is injected into the cerebrospinal fluid of a subject. In some embodiments, the injection is intracranial injection or intrathecal injection. In some embodiments, the at least one inhibitory nucleic acid is complexed with one or more cationic polymers and/or cationic lipids (e.g., any of the cationic polymers described herein or known in the art). Antagomirs to decrease the expression and/or activity of a specific target miRNA (e.g., miR-181) can be designed using methods known in the art (see, e.g., Krutzfeld et al, Nature 438:685-689, 2005). Additional exemplary methods for designing and making antagomirs and other types of inhibitory nucleic acids are described herein.

In some embodiments, the inhibitory nucleic acid that decreases miR-181 levels is the antogmir-181 LNA sequence. Methods for designing antagomirs to target microRNA molecules are described in Obad et al, Nature Genetics 43:371-378, 2011. Additional inhibitory nucleic acids for decreasing the levels or expression of miR-181 are described in Worm et al, Nucleic Acids Res. 37:5784-5792, 2009, and Murugaiyan et al, J. Immunol. 187:2213-2221, 2011.

A subject can be administered at least one (e.g., at least 2, 3, 4, or 5) dose of the agent (e.g., one or more inhibitory nucleic acids). The agent (e.g., one or more inhibitory nucleic acids) can be administered to the subject at least once a day (e.g., twice a day, three times a day, and four times a day), at least once a week (e.g., twice a week, three times a week, four times a week), and/or at least once a month. A subject can be treated (e.g., periodically administered the agent) for a prolonged period of time (e.g., at least one month, two months, six months, one year, two years, three years, four years, or five years). As described in detail herein, the dosage of the agent to be administered to the subject can be determined by a physician by consideration of a number of physiological factors including, but not limited to, the sex of the subject, the weight of the subject, the age of the subject, and the presence of other medical conditions. The agent can be administered to the subject orally, intravenously, intraarterially, subcutaneously, intramuscularly, intracranially, via injection into the cerebrospinal fluid, or via injection in the eyes (intravitreal or subretinal injection). Likewise, the agent may be formulated as a solid (e.g., for oral administration) or a physiologically acceptable liquid carrier (e.g., saline) (e.g., for intravenous, intraarterial, subcutaneous, intramuscular, cerebrospinal (intrathecal), intracranial, intravitreal or subretinal administration). In some embodiments, the agent (e.g., one or more inhibitory nucleic acids) can be administered by injection or can be administered by infusion over a period of time.

The agents to be administered to a subject for treatment of a mitochondrial disorder are described in the present document, and can be used in any combination (e.g., at least one, two, three, four, or five of any combination of the agents or classes of agents described below).

### Diseases of the invention

Diseases to be treated and/or prevented within the present invention include diseases such as PMDs including Leigh syndrome, Leber Hereditary optic Neuropathy (LHON), Mitochondrial Myopathy, Encephalopathy, Lactic Acidosis and Stroke Syndrome (MELAS), Myoclonic Epilepsy associated with Ragged-Red fibers (MERRF), Progressive External Ophthalmoplegia (PEO), Pearson's syndrome, Maternally inherited Myopathy and cardiomyopathy (MIMyCA), Kjer's optic neuropathy, Neuropathy ataxia and retinitis pigmentosa (NARP), Autosomal dominant optic atrophy, Kearns-Sayre syndrome, Myoclonus Epilepsy with Ragged-Red Fibers (MERRF) syndrome; SMDs including Friedreich's ataxia, Hereditary Spastic paraplegia, Charcot-Marie-Tooth disease; Neurodegenerative disorders affecting the central nervous system and/or the eye associated with mitochondrial dysfunction: including sporadic age-related neurodegenerative diseases, e.g. Parkinson's Disease (PD), Alzheimer's Disease (AD), Age-related Macular degeneration (AMD), Diabetic Retinopathy, Glaucoma; familiar neurodegenerative diseases including familiar PD, AD, Huntington's disease, Retinitis Pigmentosa (RP), Stargardt's disease

LHON is a non-syndromic form of mitochondrial optic neuropathy, characterized by RGSs degeneration that leads to loss of central vision. It is one of the most frequent mitochondrial diseases (prevalence 1:30000) typically occurring between the ages of 15 and 35. Similar to other MDs, LHON is characterized by high genetic heterogeneity since it is caused by point mutations in multiple genes encoded by the mitochondrial genome (mtDNA). In about 95% of LHON patients mutations are located in the *ND1, ND4,* or *ND6* genes, which encode complex I subunits {Carelli, 2004 ;Meyerson, 2015).

Microphthalmia with Linear Skin defects (MLS) syndrome, is an X-linked neurodevelopmental disorder characterized by microphthalmia, brain abnormalities and skin defects in heterozygous females and in utero lethality in hemizygous males (Indrieri, 2016). The disease is due to mutations in key players of the MRC, such as the holocytochrome c-type synthase (HCCS), involved in complex III functionality (Bernard et al, 2003; Indrieri et al, 2013; Wimplinger et al, 2006), and COX7B, the subunit 7B of Cytochrome c Oxidase (MRC complex-IV) (Indrieri et al, 2012)

Mutations in the rhodopsin gene are a cause of dominant and recessive retinitis pigmentosa (RP), accounting for about 10% of all cases. Dominant rhodopsin mutants are therefore gain-of-function or dominant-negative mutants. Several classes were defined: class I, are like wild type (wt) opsin in their ability to localize to the cytoplasmic membrane and to bind 11-cis-retinaldehyde to form spectrally active rhodopsin; class II, are probably defective in protein folding and/or stability, accumulate abnormally in the rough endoplasmic reticulum, are not transported efficiently to the cytoplasmic membrane as is wt opsin, and fail to bind 11-cisretinaldehyde to form a spectrally active photopigment; class III, constitutively activate transducin.

Among the class I mutants are those affecting proline-347 near the carboxyl terminus. This residue is conserved among all known visual pigments. Six different missense mutations affecting this residue have been identified among patients with RP, indicating that proline-347 mediates some vital function of rhodopsin.

### Delivery methods

In certain embodiments, the inhibitor of miR-181 is expressed in vivo from a vector. A "vector" is a composition of matter which can be used to deliver a nucleic acid of interest to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. Examples of viral vectors include, but are not limited to, adenoviral vectors, adeno-associated virus vectors, retroviral vectors, lentiviral vectors, and the like. An expression construct can be replicated in a living cell, or it can be made synthetically. For purposes of this application, the terms "expression construct," "expression vector," and "vector," are used interchangeably to demonstrate the application of the invention in a general, illustrative sense, and are not intended to limit the invention.

In one embodiment, an expression vector for an inhibitor of miR-181 comprises a promoter "operably linked" to a polynucleotide encoding the miR-181 inhibitor. The phrase "operably linked" or "under transcriptional control" as used herein means that the promoter is in the correct location and orientation in relation to a polynucleotide to control the initiation of transcription by RNA polymerase and expression of the polynucleotide.

Particularly preferred viral vectors are AAV vectors, suitably AAV9, AAV5 and AAV2 vectors (e.g. AAV2/2, AAV2/8, AAV2/7m8, AAV2/9, AAV2/PhP.B).

Preferred promoters of the present invention are ubiquitous promoters, e.g. promoters driving the ubiquitous expression of the miRNA of the invention, for instance CMV or CBA promoters; alternatively, promoters of the invention may be cell or tissue specific promoters, e.g. a-syn promoter for neuron specific expression or for instance RHO promoter for photoreceptor specific expression.

Non-viral delivery systems are complexes composed of plasmids and synthetic, usually positively charged, materials, such as liposomes, polysaccharides, polypeptides, and artificial polymers as those described in D. Ibraheem, A. Elaissari, H. Fessi, Gene therapy and DNA delivery systems, Int. J. Pharm. 459 (2014) 70-83. One such example is the delivery system based on the usage of dicetyl phosphate-tetraethylenepentamine-based polycation liposomes (TEPA-PCL). Through this system, miRNAs as well as other nucleotides, including miRNA sponges, are delivered into the cell via macropinocytosis and released into the cytoplasm with high efficiency. Non-viral delivery systems offer the possibility of low immunogenicity and low toxicity, thereby being attractive in providing improved safety.

### DETAILED DESCRIPTION OF THE INVENTION

### MATERIAL AND METHODS

### Animal Studies

Ethical statement: All studies on fish and mice were conducted in accordance with the institutional guidelines for animal research and approved by the Italian Ministry of Health; Department of Public Health, Animal Health, Nutrition and Food Safety in accordance to the law on animal experimentation (article 7; D.L. 116/92; protocol number: 389/2015-PR). All animal treatments were reviewed and approved in advance by the Institutional Ethics Committee at the Telethon Institute of Genetics and Medicine (Pozzuoli, Italy).

### Cell lines

HeLa and SH-SY5Y cells were obtained from ATCC ((CRM-CCL-2, CRL-2266) and maintained in culture respectively with Dulbecco's Modified Eagle Medium (GIBCO) or a 1:1 mixture of Dulbecco's Modified Eagle Medium and Nutrient Mixture F-12, supplemented with 10% FBS (SIGMA) and 1% penicillin/streptomycin, as suggested by the vendor.

### Luciferase assays

Human 3'UTR sequences containing miR-181a/b predicted binding sites were amplified by PCR and inserted in the pGL3-tk-Luciferase vector. Constructs containing mutagenized miR-181a/b binding sites were obtained using the QuikChange Site-Directed Mutagenesis Kit (Stratagene). Primer sequences are reported in Table I. Plasmids were transfected in HeLa cells (PolyFect reagent, QIAGEN). After 7h, cells were transfected with 100nM of miRIDIAN Negative Mimic or Mimic-miR-181 (DHARMACON) using Interferin (Polyplus). After 24h luciferase activities were quantified using Dual-Luciferase Reporter Assay (Promega).

**Table I: Primer sequences for human 3'UTR amplification and mutagenesis**

| **Transcript** | **Primer sequences** |
|---|---|
| hsa-*NRF1-3'UTR* | Fw-GCTCTAGAGACTTCTTTCTGCGGAAATG SEQ ID NO. 15 |
| | Rv-GCTCTAGACTGTTTTCTATGGCCAGGTG SEQ ID NO. 16 |
| hsa-*NRF1-3'UTR* mutagenesis | Fw-CCACAGGCAGATGCGCGTCTTGAAAGCTCCCGGGCC SEQ ID NO. 17 |
| | Rv-GGCCCGGGAGCTTTCAAGACGCGCATCTGCCTGTGG SEQ ID NO. 18 |
| hsa-*PPARGC1A-3'UTR* | Fw-GCTCTAGAGCACTACAGATATCATATTGAGG SEQ ID NO. 19 |
| | Rv-GCTCTAGATAGATTTGAAACATTCGTTTCCC SEQ ID NO. 20 |
| hsa-*PPARGC1A-3'UTR* mutagenesis | Fw-CTGAGCTAATAAAGGGAAACGCCGGTTTCAAATCTCTAGGTCGGG SEQ ID NO. 21 |
| | Rv-CCCGACCTAGAGATTTGAAACCGGCGTTTCCCTTTATTAGCTCAG SEQ ID NO. 22 |
| hsa-*COX1-3'UTR* | Fw-GCTCTAGAGCCTAGCTAGAATATATGAC SEQ ID NO. 23 |
| | Rv-GCTCTAGACACAGGCTTCCTACATTTAG SEQ ID NO. 24 |
| hsa-*COX11-3'UTR* mutagenesis | Fw-CAAGTCCATGCGCGTTAAAATGTACAGGTGGGATTG SEQ ID NO. 25 |
| | Rv-CAATCCCACCTGTACATTTTAACGCGCATGGACTTG SEQ ID NO. 26 |
| hsa-*COQ10-3'UTR* | Fw-GCTCTAGAGCCACCTGCTTCTGACTTTAG SEQ ID NO. 27 |
| | Rv-GCTCTAGACAACTTCAGTCCTTACATTG SEQ ID NO. 28 |
| hsa-*COQ10-3'UTR* mutagenesis | Fw-GAAGATAAGTTGGTTGGGCGTCTCCAGCACTATGCATCCC SEQ ID NO. 29 |
| | Rv-GGGATGCATAGTGCTGGAGACGCCCAACCAACTTATCTTC SEQ ID NO. 30 |
| hsa-*PRDX3-3'UTR* | Fw-GCTCTAGACTGAGAGAAGAACCACAGTTG SEQ ID NO. 31 |
| | Rv-GCTCTAGACCCTGGATTTGATAAATATCC SEQ ID NO. 32 |
| hsa-*PRDX3-3'UTR* mutagenesis site I | Fw-CGGTCCTGAAATTTTCATCTTGCCGGTCTTTGTATTAAACTGAATTTTC SEQ ID NO. 33 |
| | Rv-GAAAATTCAGTTTAATACAAAGACCGGCAAGATGAAAATTTCAGGACCG SEQ ID NO. 34 |
| hsa-*PRDX3-3'UTR* mutagenesis sitell | Fw-GCCGTGTAACTCCTGCAATGCCGGTTTATGTGATTGAAGC SEQ ID NO. 35 |
| | Rv-GCTTCAATCACATAAACCGGCATTGCAGGAGTTACACGGC SEQ ID NO. 36 |

### RNA extractions

Tissue or cells samples for total RNA extraction were processed in QIAzol Lysis Reagent (QIAGEN). Total RNA was extracted using the RNeasy extraction kit (QIAGEN), according to manufacturer's instructions.

### Quantitative Real-Time PCR

For quantitative Real-Time PCR (qRT-PCR) experiments, cDNAs were generated using QuantiTect Reverse Transcription Kit (QIAGEN), according to manufacturer's instructions.

Primers for qRT-PCR reactions were designed to span two different exons to avoid genomic DNA amplification using *in silico* tools (www.basic.northwestern.edu/biotools/oligocalc.html) to predict their melting temperature (*T*ₘ) and to avoid the possibility of self-annealing or primer dimerization. The specificity of the designed primers was tested *in silico* using the BLAT or BLAST tool in Genome Browser (https://genome.ucsc.edu/) or Ensembl (http://www.ensembl.org/index.html). Primers were tested according to (Bustin et al, 2009). Primer sequences are reported in Table II. Quantification data, obtained in qRT-PCR reactions on cDNAs obtained from the different treatments, are expressed in terms of cycle thresholds (Ct). The *HPRT* and *GAPDH* genes were used as endogenous reference controls for the experiments. The Ct values were averaged for each in-plate technical triplicate. The averaged Ct was normalized as difference in Ct values (ΔCt) between the analyzed mRNAs and each reference gene in each sample in analysis. The ΔCt values of each sample were then normalized with respect to the ΔCt values of the control (ΔΔCt). The variation was reported as fold change (2^{-ΔΔCt}). Each plate was performed in duplicate, and all the results are shown as means ± SEM of at least 3 independent biological assays.

For the analysis in FED and STARVED conditions: mice for the starved condition were euthanized after 12 hours of absence of nutrients (e.g. the food supply was withdrawn for 12 hrs), whilst mice for fed condition were euthanized after 12 hours of absence of nutrients and 1 hour of re-feeding (e.g. the food supply was returned to each cage).

**Table II: Primer sequences for qRT-PCR analysis.**

| **Transcript** | **Forward** | **Reverse** |
|---|---|---|
| hsa-*HPRT* | AACACCCTTTCCAAATCCTCA SEQ ID NO. 37 | TGGCGTCGTGATTAGTGATG SEQ ID NO. 38 |
| hsa-*GAPDH* | ATGTTCGTCATGGGTGTGAA SEQ ID NO. 39 | AGGGGTGCTAAGCAGTTGGT SEQ ID NO. 40 |
| hsa-*PPARGC1A* | GCTTGGCCCTCTCAGACTCT SEQ ID NO. 41 | GCCACTACAGACACCGCACG SEQ ID NO. 42 |
| hsa-*NRF1* | GAGTGATGTCCGCACAGAAG SEQ ID NO. 43 | GTTTGCGTTTGCTGATCTTC SEQ ID NO. 44 |
| hsa-*COX11* | GCTGCCGTACCCCTTTATCG SEQ ID NO. 45 | CACTGGAGACTTGCATGCAC SEQ ID NO. 46 |
| hsa-*COQ10* | GGCGTTTTAGCCCAGGTCTTC SEQ ID NO. 47 | GCTACCATCTGCTTCACAAC SEQ ID NO. 48 |
| hsa-*PRDX3* | CCCGAGACTACGGTGTGCTG SEQ ID NO. 49 | CACTGGGAGATCGTTGACGC SEQ ID NO. 50 |
| | | |
| mmu-*Hprt* | AGCTTGCTGGTGAAAAGGAC SEQ ID NO. 51 | GTCAAGGGCATATCCACAAC SEQ ID NO. 52 |
| mmu-*Gapdh* | GGTGCTGAGTATGTCGTGGA SEQ ID NO. 53 | CTAAGCAGTTGGTGGTGCAG SEQ ID NO. 54 |
| mmu-*Bcl2* | ACAACATCGCCCTGTGGATG SEQ ID NO. 55 | GTTTGTCGACCTCACTTGTG SEQ ID NO. 56 |
| mmu-*Mcl1* | GCTTCATCGAACCATTAGCAG SEQ ID NO. 57 | CCAGCAGCACATTTCTGATG SEQ ID NO. 58 |
| mmu-*Xiap* | GGTCCTGATTGCAGATCTTG SEQ ID NO. 59 | GTCAACTGCTTCTGCACAC SEQ ID NO. 60 |
| mmu-*Atg5* | GCCTATATGTACTGCTTCATC SEQ ID NO. 61 | CAACGTCAAATAGCTGACTC SEQ ID NO. 62 |
| *mmu-Erk2* | CACCAACCTCTCGTACATCG SEQ ID NO. 63 | GTGCCCGGATGATGTCATTG SEQ ID NO. 64 |
| *mmu-Park2* | CCATCAAGAAGACCACCAAG SEQ ID NO. 65 | CAAGTGACATCTCTCTCTAC SEQ ID NO. 66 |
| mmu-*Nrf1* | CTTACTGGAGTCCAAGATGC SEQ ID NO. 67 | GGAGCCAACAGAATCCTTTC SEQ ID NO. 68 |
| mmu-*Cox11* | GAATCCTACTGACAAACCAG SEQ ID NO. 69 | GAGGTCGACATTCACCATTC SEQ ID NO. 70 |
| *mmu-Coq10* | GATGATCATGGCAGCTCGGA SEQ ID NO. 71 | CTCGCACAGATCTCTTTAGG SEQ ID NO. 72 |
| mmu-*Prdx3* | GGAGTATTTCTGCCTCAACAG SEQ ID NO. 73 | CTCTCCATTGACAACAGCAG SEQ ID NO. 74 |
| mmu-*Ppargc1a* | GGAATGCACCGTAAAATCTGC SEQ ID NO. 75 | TTCTCAAGAGCAGCGAAAGC SEQ ID NO. 76 |
| mmu-*Sqstm1* | TTCTTTTCCCTCCGTGCTC SEQ ID NO. 77 | GGATCCGAGTGTGAATTTCC SEQ ID NO. 78 |
| mmu-*Map1lc3b* | TTATAGAGCGATACAAGGGGGAG SEQ ID NO. 79 | CGCCGTCTGATTATCTTGATGAG SEQ ID NO. 80 |
| mmu-*Rhodopsin* | GCCACACTTGGAGGTGAAATC SEQ ID NO. 81 | AAGCGGAAGTTGCTCATCG SEQ ID NO. 82 |
| mmu-*S-Opsin* | TGGACTTACGGCTTGTCACC SEQ ID NO. 83 | AAGCCCGGAACTGCTTATTC SEQ ID NO. 84 |
| mmu-*M-Opsin* | CAGCAACAGCACCAAAGGTC SEQ ID NO. 85 | ACAGATGCAACGACCACAAG SEQ ID NO. 86 |
| mmu- *Cone-Arrestin* | GGCTCTTAGCACAATTCTTCG SEQ ID NO. 87 | GAATGCCCCCATAGGACAC SEQ ID NO. 88 |
| mmu- *Pde6* 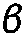 | CCCTAACTCCACAAACACCTG SEQ ID NO. 89 | ATGACATTGCCCGTTAGGAC SEQ ID NO. 90 |
| | | |
| ol-*hprt* | CTGAACAGGAACAGCGACC SEQ ID NO. 91 | TGAGGAGCTCCAATAACGTC SEQ ID NO. 92 |
| ol-*gapdh* | CGGCAAGCTGATAGTCGATG SEQ ID NO. 93 | AGAAACACTCCGGTGGACTC SEQ ID NO. 94 |
| ol-*bcl2* | GGATGACGGAGTATTTAAACG SEQ ID NO. 95 | GGCCGAAGACAGTCTTGATG SEQ ID NO. 96 |
| ol-*mcl1* | CGCGAAAAGAGTGGTGAAC SEQ ID NO. 97 | CCTTCAAGGACTGACACAC SEQ ID NO. 98 |
| ol-atg5 | CAGATGACAAAGACGTGCTG SEQ ID NO. 99 | CTCCACATCTTCTGTCCTCA SEQ ID NO. 100 |
| *ol-erk2* | GCAGCGACAGCAGATAGTTC SEQ ID NO. 101 | GCCGAGATGTTGTCCAACAG SEQ ID NO. 102 |
| ol-*cox11* | CACCGACAAACCCATCATC SEQ ID NO. 103 | GAATTCCGGGTCGATGTAG SEQ ID NO. 104 |
| ol-*coq10* | GTTCTGCAAGGCCAAACTG SEQ ID NO. 105 | CGTCTCCAAGTGGTTGAAG SEQ ID NO. 106 |
| ol-*prdx3* | GTCCTGTTCTTCTACCCTC SEQ ID NO. 107 | AGTGAGAATCCACAGACACC SEQ ID NO. 108 |
| ol-*ppargc1a* | GGACGGATTGCCTTCATTTG SEQ ID NO. 109 | GTTTGCAGGTGCCAGAAGG SEQ ID NO. 110 |

### SH-SY5Y transfections and FCCP treatment

SH-SY5Y cells were transfected with 100nM of Negative control miRNA inhibitor and miR-181a and miR-181b inhibitors (DHARMACON IH-300552-07-0005 and IH-300554-08-0005, respectively) using Dharmafect (DHARMACON).

The miR inhibitors are synthetic single-stranded RNA molecules designed to inhibit endogenous microRNA activity. Upon 72h of transfection, SH-SY5Y cells were treated with 25µM FCCP (Sigma, C2920). After 6h of FCCP treatment, cell death was evaluated by Trypan Blue staining.

### ARPE cells LNA treatment

ARPE-19 cells (CRL-2302) were transfected with 80nM of LNA- Negative control miRNA inhibitor and miR-181a (CGACAGCGTTGAATGT SEQ ID NO. 6) LNA and miR-181b (CGACAGCAATGAATGT SEQ ID NO.7) LNA inhibitors (Exiqon) using Interferin (Polyplus). Upon 48h of transfection, ARPE-19 cells were treated with 100uM of A2E (ACME BIOSCIENCE INC. Customer synthesis A2E, AB4344). After 6h of A2E treatment, cell autofluorence was evaluated.

### Mature miRNA quantitative assay

For mature quantitative detection of miR-181a and miR-181b, the inventors used hydrolysis probes (TaqMan, Applied Biosystem). The cDNAs for mature miRNAs analysis were generated using TaqMan MicroRNA Reverse Transcription Kit with miRNA-specific primers, according to manufacturer instructions. The quantification data, obtained in TaqMan-PCR reactions on TaqMan-cDNAs from the different treatments, are expressed in terms of cycle thresholds (Ct). A TaqMan probe for the RNA *sno234* was used as endogenous control for the experiments. The Ct values were analyzed as described in the "Quantitative Real-Time PCR" section. Each plate was performed in duplicate, and all the results are shown as means ± SEM of three independent biological replicates.

### RNA in-situ hybridization (ISH)

Mouse eyes or brains were fixed overnight in 4% PFA, cryoprotected with 30% sucrose and embedded in OCT. Twenty-micrometer cryosections were treated with 5 µg/mL proteinase K for 15 min. After washes with 2 mg/mL glycine and postfixation with 4% PFA/0.2% glutaraldehyde sections were prehybridized with 50% formamide, 5X sodium saline citrate buffer (SSC) and citric acid to pH 6,1% sodium dodecyl sulfate (SDS), 500 µg/mL yeast tRNA, and 50 µg/mL heparin. The miRCURY detection miR-181a and miR-181b Locked Nucleic Acid probes (Exiqon) were used at a final concentration of 30nM. Probe hybridization was performed overnight at 42°C. Hybridized sections were washed with 50% formamide, 2×SSC at the hybridization temperature. Sections were blocked for 1 hour in MABT (100mM maleic acid, 150mM NaCl, and 0.1% Tween20) containing 1% blocking reagent (Roche) and 10% sheep serum and incubated with alkaline phosphatase (AP)-labeled anti-digoxigenin antibody (1:2000; Roche) in MABT/1% blocking reagent overnight at 4°C. Sections were stained with NBT-BCIP (ROCHE) and photographed under Leica DM5000 microscope.

ISH on medakafish were performed as described (Carrella et al, 2015).

### Quantitative mitochondrial DNA content analysis

SYBR Green real-time PCR was performed using primers for the *COI* gene (mtDNA) and the RNaseP (nuclear gene reference), as described(Viscomi et al, 2009). mtDNA relative copy number was calculated from threshold cycle value (ΔCt), and mtDNA copy number/cell was calculated as 2 × 2^{-Δct} to account for the two copies of RNaseP in each nucleus.

### Protein isolation and Western blotting (WB)

Tissue and cells samples for total protein extraction were homogenized in RIPA buffer with protease inhibitor cocktail tablet (Roche). Protein extract concentrations were determined using the Bio-Rad protein assay (Bio-Rad). A total of 30µg protein from each sample was loaded in SDS-polyacrylamide gels. Mitochondrial and cytosolic extracts were obtained from whole eyes without lens as described (Comitato et al, 2014). 30µg of proteins from mitochondrial and cytosolic fractions from each sample were loaded in SDS-polyacrylamide gels. For WB, gels were electroblotted onto nitrocellulose filters and sequentially immunostained with the following primary antibodies: anti-p62 (Abnova, clone 2C11, 1:1000); anti-LC3B (Novus, 1:500); anti-Mfn2 (Abcam, ab56889, 1:1000); anti-Citrate Synthase (Abcam, ab9660, 1:1000); anti-p115 (Santa Cruz, sc-48363, 1:3000); anti-Tim23 (Santa Cruz, sc-13298, 1:250); anti-Ndufb11 (Proteintech, 1:1000); anti-Ndufb8 (Abcam, Total OXPHOS Rodent WB Antibody Cocktail ab110413, 1:250); anti-CoxIV (Cell Signaling 4844, 1:1000); anti-Parkin (Cell Signaling 4211, 1:500); anti-Gapdh (Santa Cruz sc-32233, 1:3000).

Proteins of interest were detected with horseradish peroxidase-conjugated goat anti-mouse or anti-rabbit IgG antibody (1:3000, GE Healthcare) visualized with the Luminata Crescendo substrate (Millipore) or the Super Signal West Femto substrate (Thermo Scientific), according to manufacturer's protocol. WB images were acquired using the Chemidoc-It imaging system (UVP) and band intensity was calculated using the ImageJ software. The signals for each protein staining were quantified and then normalized for the GAPDH or p115 or for Ndufb8 (mitochondrial fractions) in the same sample (internal normalization). These normalized values were then compared to the values in the control sample. Only bands on the same blot were compared. The average of the normalized values from three different biological replicates is reported as the relative fold change.

For the analysis in FED and STARVED conditions: mice for the starved condition were euthanized after 12 hours of absence of nutrients, whilst mice for fed condition were euthanized after 12 hours of absence of nutrients and 1 hour of re-feeding.

### Medakafish and Morpholino

Medakafish (Cab strain) were kept and staged as described (Iwamatsu, 2004). Design, specificity and inhibitory efficiency of MOs were previously described (Carrella et al, 2015; Indrieri et al, 2013; Indrieri et al, 2012). Morpholino oligos are advanced tools for blocking sites on RNA to obstruct cellular processes. A Morpholino oligo specifically binds to its selected target site to block access of cell components to that target site. This property can be exploited to block translation, block splicing, block miRNAs or their targets, and block ribozyme activity. A Morpholino oligo is radically different from natural nucleic acids, with methylenemorpholine rings replacing the ribose or deoxyribose sugar moieties and non-ionic phosphorodiamidate linkages replacing the anionic phosphates of DNA and RNA. Each morpholine ring suitably positions one of the standard DNA bases (A,C,G,T) for pairing, so that a 25-base Morpholino oligo strongly and specifically binds to its complementary 25-base target site in a strand of RNA via Watson-Crick pairing. Because the uncharged backbone of the Morpholino oligo is not recognized by enzymes, it is completely stable to nucleases. hccs-MO, cox7b-MO, miR-181a-MO and miR-181b-MOs (MO-miR-181a 5'-AACTCACCGACAGCGTTGAATGTTC-3' SEQ ID NO. 111; MO-miR-181b 5'-AACCCACCGACAGCAATGAATGTTG-3' SEQ ID NO. 112) were injected into fertilized one-cell medaka embryos at a concentration of 0.3mM, 0.1mM, 0.075mM and 0.075mM, respectively.

### Tunel assay

Whole mount TUNEL assay was performed on st30 medakafish embryos using In Situ Cell Death Detection Kit (Roche) as described (Indrieri et al, 2013). After staining, embryos were embedded in a mix of BSA/Gelatine and vibratome-sectioned. Sections were analyzed with a Leica DM-6000 microscope and TUNEL-positive cells were manually counted in the entire retina.

### Caspase assays

Twenty embryos for sample were dechorionated at st32 and frozen in liquid nitrogen for at least 24h. Caspase assays were performed on protein lysates using caspase-3/7- and caspase-9-GLO luciferase reagent (Promega) as previously reported (Indrieri et al, 2013). The emitted luminescence signals were normalized for the protein concentration of each sample.

### Drug treatments in medakafish

St24 embryos were dechorionated and incubated for 24h in 50nM Bafilomycin A (SIGMA, B1793), 25µM PD98059 (Cell Signaling, #9900) or 6µM HA14-1 (SIGMA, 48787). Drugs were diluted in 3% DMSO/embryo medium. The PD medakafish model was generated by growing embryos from st32 to st38 in 500µM 6-OHDA (SIGMA, H4381) in 3%DMSO/0.2% ascorbic acid/embryo medium. Medium was refreshed twice a day. Control embryos were grown in 3% DMSO/embryo medium.

### Dopaminergic neuron detection in medakafish

Control and miR-181a/b-MOs DMSO- or 6-OHDA-treated fish were fixed in 4% PFA. Fish were treated with 10µg/ml of proteinase K for 1h and 30min, washed with 2mg/ml glycine and fixed in 4% PFA for 20min. Embryos were then incubated with 0,1% sodium citrate/0,1% Triton X-100 for 10min in ice. After an overnight incubation with an anti-TH antibody (Abcam ab112, 1:500), fish were incubated with a peroxidase-conjugated anti-rabbit antibody (1:200; Vector Laboratories) followed by diaminobenzidine staining (DAB; Sigma). Embryos were embedded in a mix of BSA/Gelatine and vibratome-sectioned. Sections were observed with a Leica DM-6000 microscope and anti-TH positive cells were manually counted in the entire medaka *locus coeruleus.*

### Rotenone injections

Rotenone (SIGMA, R8875-) was injected intravitreally at the final concentration of 5mM, as reported (Heitz et al, 2012). For histological analysis of RGCs, one eye of two-months old mice, *miR-181a*/*b-*^{*+*/}*⁺ and miR-181a*/*b-1*^{*-*/}*⁻,* was injected with Rotenone and the contralateral eye with DMSO (internal control). For Optokinetic tests mice were bilaterally injected with Rotenone 5mM or DMSO.

### NADH dehydrogenase activity staining

NADH dehydrogenase histochemical reaction was performed as described {Marella, 2010 } with minor modifications. Briefly, retinal sections were washed three times in PBS and then incubated in a solution containing 2 mg/ml nitro blue tetrazolium, 0.5 mM NADH, 50 mM Tris-Cl, pH 7.6 at 37°C for 30 min. The slides were washed twice with PBS and mounted with PBS/Glycerol 50%.

### Optokinetic tracking

Visual acuity of rotenone-injected mice was assessed by using the Optomotor system (OptoMotry; Cerebral Mechanics) as described (Chadderton et al, 2013).

### Immunofluorescence and immunohistochemistry analysis

For immunofluorescence analysis, mouse eyes were fixed in 4% PFA, cryoprotected with 30% sucrose, embedded in OCT and cryosectioned. Sections were permeabilized by boiling in Sodium Citrate Buffer or by incubation with 1% NP40 for 15 min for cone-Arrestin immunostaining. The following primary antibodies were incubated overnight: anti-Rhodopsin (Abcam, ab3267, 1:5000); anti-Cone-Arrestin (Millipore, 1:1000); anti-Pax6 (Covance, 1:250); anti-GS6, (Millipore, 1:100); anti-Syntaxin (SIGMA 1:100); anti-NeuN (Millipore, clone A60 1:400); anti-CoxIV (Cell Signaling 4844, 1:400). Sections were then incubated with the Alexa Fluor secondary antibodies (1:1000; Invitrogen). The sections were counterstained with DAPI (Vector Laboratories). RGCs stained using anti-NeuN antibody were counted on 8 different slides for each eye in the areas surrounding the optic nerve. Sections were photographed under LSM710 Zeiss confocal microscopy.

For dopaminergic neurons detection in mouse, mice were anesthetized and then perfused with 4% PFA after 30-35 days from surgery. Brains were post-fixed in 4% PFA and vibratome-sectioned. The full-depth of the midbrain was sectioned, and the sections were consecutively collected. Floating sections were incubated for 20min in PBS1x/FBS10%/Triton0.1% and then for 1h in BSA0.1%/FBS10%/Triton0.1% in PBS1x. After an overnight incubation with an anti-TH antibody (Abcam ab112, 1:1000), sections were incubated with a peroxidase-conjugated anti-rabbit antibody (1:200; Vector Laboratories, Burlingame, CA, USA) followed by diaminobenzidine staining (DAB; Sigma). All sections containing SN area were selected using the mouse brain atlas{Paxinos, 2004 } (Bregma -2.54 to -3.64 mm). Every fourth section covering the entire extent of SN was used for counting. Brain sections were observed with a Leica DM-6000 microscope and TH-positive cells were manually counted in both the 6-OHDA-injected and the control side of each animal. The criterion for counting TH-positive cells was the presence of their nuclei in the focal plane. The SN pars compacta was carefully defined using the mouse brain atlas {Paxinos, 2004 }, and particular care was taken not to include other TH-positive regions of the SN as well as the ventral tegmental area.

### Electroretinogram (ERG)

Mice were dark reared for 3h and anesthetized. Flash electroretinograms (ERGs) were evoked by 10-ms light flashes generated through a Ganzfeld stimulator (CSO, Costruzione Strumenti Oftalmici, Florence, Italy) and registered as previously described (Botta et al, 2016; Surace et al, 2005). ERGs and b-wave thresholds were assessed using the following protocol. Eyes were stimulated with light flashes increasing from -5.2 to +1.3 log cd*s/m² (which correspond to 1×10^{-5 2} to 20.0 cd*s/m²) in scotopic conditions. For ERG analysis in scotopic conditions the responses evoked by 11 stimuli (from -5.2 to +1.3 log cd*s/m²) with an interval of 0.6 log unit were considered. To minimize the noise, three ERG responses were averaged at each 0.6 log unit stimulus from -5.2 to 0.0 log cd*s/m² while one ERG response was considered for higher stimuli (from 0.0 to +1.3 log cd*s/m²). a- and b-waves amplitudes recorded in scotopic conditions were plotted as a function of increasing light intensity (from -4 to +1.3 log cd*s/m²). The photopic ERG was recorded after the scotopic session by stimulating the eye with ten 10 ms flashes of 20.0 cd*s/m² over a constant background illumination of 50 cd/m².

### 6-OHDA Injection and behavioral assays

Two months old male mice, *miR-181a*/*b-*^{*+*/*+*} and *miR-181a*/*b-1*^{*-*/}*⁻,* were anesthetized and placed in a sterotaxic frame (Kopf Instruments). 6-OHDA (2 mg/kg; Sigma) was dissolved in a solution of NaCl 0.9%/0.01% ascorbic acid and unilaterally injected in the right side, in the *substantia nigra* at the coordinates: AP=-3.1; L=±0.8; DV=-4.8, -5.2 according to {Paxinos, 2004 }. To protect the noradrenergic system {De Leonibus, 2007 }, mice received an injection of desipramine (35 mg/kg i.p.; Sigma), 15min before the anesthesia. The total 6-OHDA injection volume was 0.8µL {0.5µL at DV=-5.2 and 0.3 µL at DV=-4.8). The other side of the brain was injected with saline solution. After surgery, animals received 1ml saline solution, and the first 3 days they received 1ml of sucrose solution twice a day to prevent excessive body weight loss associated to the lesion-induced aphagia. After 15 days animals were tested for contralateral rotation induced by L-DOPA, as read-out parameter of the lesion extent {De Leonibus, 2009 }. Behavioral testing was performed during light period. Before testing animals were acclimatized to the behavioral room for at least 30min. Mice were placed into a plexiglass cylinder (diameter, 19cm) and injected with benserazide (20mg/kg, i.p., Sigma) and video tracked (AnyMaze, Stoelting) for 20min. After this habituation period, mice were injected with L-DOPA (25mg/kg, i.p., Sigma) and video tracked for additional 40min. On spot rotations are defined as a complete full 360° turn of the body.

P347S mice are transgenic mice carrying a human proline-347 to serine (P347S) mutation, described in PMID:8943080

### Statistical Analysis

Animals were allocated to experimental and control groups randomly. No data were excluded from the analysis. Behavioral and visual tests were carried out blinded. The number of experimental replicates is indicated in each figure legend. In all experiments the significance of differences between groups was evaluated by one-way or two-way ANOVA with post-hoc Tukey analysis, one-tailed or two-tailed Student's t-test, Analysis of Deviance for Generalized Linear Model or Analysis of Deviance for Negative Binomial Generalized Linear Model as reported in figure legends. p<0.05 was considered significant. Quantitative data are presented as the mean ± SEM, p< 0.05 was considered significant.

### EXAMPLES

### Example 1

### miR-181a/b control mitochondrial turnover

The present inventors identified *PPARGC1A* and *NRF1,* master regulators of mitochondrial biogenesis (Finck & Kelly, 2006; Wu et al, 1999), *COX11* and *COQ10B,* involved in mitochondrial respiratory chain (MRC) assembly (Carr et al, 2002; Desbats et al, 2015), and *PRDX3,* an important mitochondrial ROS scavenger (Wonsey et al, 2002), as putative miR-181a/b target genes through a bioinformatic search (Gennarino et al, 2012). Interestingly, qRT-PCR analysis indicated increased levels of all of the above-mentioned transcripts in SH-SY5Y human neuroblastoma cells following miR-181a/b silencing with inhibitors purchased from Dharmacon as described in detail below (Fig. 1A).

To validate the newly-predicted miR-181a/b targets, the 3'-UTR of each human gene *(PPARGC1A, NRF1, COX11, COQ10B* and *PRDX3*)*,* including the predicted miR-181 target site was cloned in the pGL3-TK-Luciferase plasmid, downstream of the coding region of the Luciferase reporter gene. The ability of transfected Mimic-miR-181 to affect Luciferase activity was then tested. The presence of the 3'-UTR sequence of the analyzed genes inhibited Luciferase activity in response to mimic-miR-181 (synthetic duplex oligonucleotides identical in sequence to miRNA 181 a and b which mimic its function, Fig. 1B, purchased by Dharmacon as described in detail in the methods section). In addition, point mutations in the miR-181a/b binding site in the 3'-UTR of each gene abolished Luciferase repression demonstrating that these miRNAs directly and specifically target the *NRF1, COX11, COQ10B* and *PRDX3* genes (Fig. 1B). Direct targeting of *PPARGC1A* was not validated (Fig. 1B), indicating that the upregulation observed by qRT-PCR after miR-181a/b silencing (Fig. 1A) could be the result of an indirect effect.

Based on the above results, the inventors reasoned that the downregulation of miR-181a/b could stimulate mitochondrial biogenesis and tested this hypothesis *in vivo.* In mammals, miR-181a and miR-181b are organized in two clusters, namely *miR-181a*/*b-1* and *miR-181a*/*b-2,* localized to different genomic loci (chromosomes 1 and 2, respectively). The mature forms of miR-181a-1 and miR-181a-2, as well as those of miR-181b-1 and miR-181b-2, display identical sequences. Furthermore, both miR-181a and miR-181b contain the same "seed" sequence (Ji et al, 2009) ACAUUCA, i.e., the region that is believed to play the most important role in target recognition (Bartel, 2009). The mouse model harboring a targeted deletion of the *miR-181a*/*b-*1 cluster described in (Henao-Mejia et al, 2013) was selected, since said cluster accounts for most of the expression of mature miR-181a/b in both brain and retina, as demonstrated by RNA *in situ* hybridization, Taqman assays and the increase of several validated miR-181a/b targets, such as *Bcl2, Mcl1, Xiap, Atg5, Erk2* and *Park2*(Cheng et al, 2016; He et al, 2013; Hutchison et al, 2013; Ouyang et al, 2012; Rodriguez-Ortiz et al, 2014; Tekirdag et al, 2013) (Fig. 7 A-D; 9). Interestingly, increased expression levels of *Nrf1, Ppargcla, Cox11, Coq10b* and *Prdx3* in the eye of *miR-181a*/*b-1*^{*-*/*-*} mice were observed by RT-PCR, as shown in Figure 1C. Increased levels of *Nrf1* and *Ppargc1a* indicate enhanced mitochondrial biogenesis (Finck & Kelly, 2006; Wu et al, 1999). In line with this observation, an increase of mitochondrial DNA (mtDNA) was detected as measured by qRT-PCR and shown in figure 1D, as well as an increase of the protein levels of MRC complex subunits (Ndufb11 and Cox4) and of general mitochondrial markers (Mfn2, Tim23 and Citrate Synthase [Cs]), as assessed by Western blot (WB) in the eye of *miR-181a*/*b-1*^{*-*/*-*} mice and shown in figure 1 E. Overall, these data demonstrate that miR-181a/b inactivation stimulates mitochondrial biogenesis in the CNS, and suggest a broad action of these two miRNAs on global mitochondrial turnover. The inventors further investigated whether miR-181a/b inactivation enhances mitophagy in the mouse eye. As shown in Figure 8A, the transcript levels of the genes, as measured by RT-PCR, were upregulated in *miR-181a*/*b-1*^{*-*/*-*} eyes. Moreover, enhanced recruitment of Park2/Parkin and of the autophagic adaptor *Sqstm1*/p62 in mitochondrial fractions was detected by Western Blot analysis and shown in Figure 1F, indicating an increase of mitophagy in *miR-181a*/*b-1*^{*-*/*-*} eyes. General autophagy was further analyzed by evaluating the levels of *Sqstm1*/p62 and of the autophagic marker *Map*/*1c3b*/Lc3-II in total protein extracts. By WB, decreased levels of these two proteins were observed, more evident in starved conditions (Fig. 8B), without changes in the corresponding transcript levels (Fig. 8C), indicating an increase in the autophagic flux rate in *miR-181a*/*b-1*^{*-*/*-*} eyes.

Taken together, these results uncover an important role of miR-181a/b in the regulation of mitochondrial turnover through the coordination of mitochondrial biogenesis and clearance in *vivo.*

### Example 2

### miR-181a/b inhibition protects neurons from cell death and ameliorates the phenotype of in vivo models of MLS syndrome

The present inventors have sursprisingly found that miR-181a/b inactivation protects from mitochondrial damage. First, they tested whether miR-181a/b downregulation in vitro protects SH-SY5Y cells from FCCP, a potent uncoupler of oxidative phosphorylation (OXPHOS): while 50% of control cells died within 6 hours (h) of the treatment with FCCP, miR-181a/b-silenced cells did not display any change in the extent of cell death (Fig. 9), demonstrating that miR-181a/b-silencing indeed protects cells from mitochondrial damage.

The inventors further evaluated the neuroprotective effect of miR-181a/b inactivation in *in vivo* models of mitochondria-mediated neurodegeneration in two fish models for a rare form of MD, Microphthalmia with Linear Skin lesions (MLS) syndrome. MLS is a neurodevelopmental disorder characterized by microphthalmia, brain abnormalities and skin defects in heterozygous females and *in utero* lethality in hemizygous males {Indrieri, 2016 }. The disease is due to mutations in key players of the MRC, such as the holocytochrome c-type synthase (*HCCS*)*,* involved in complex III functionality (Bernard et al, 2003; Indrieri et al, 2013; Wimplinger et al, 2006), and *COX7B,* the subunit 7B of Cytochrome c Oxidase (MRC complex-IV) (Indrieri et al, 2012). Two medaka fish (*Oryzias latipes*) models of MLS were previously generated by knocking down, using a Morpholino(MO)-based approach, *hccs* or *cox7B* expression (Indrieri et al, 2013; Indrieri et al, 2012). Both models (*hccs-MO* and *cox7b-MO*) show a severe microphthalmic and microcephalic phenotype due to increased cell death in the CNS (Indrieri et al, 2013; Indrieri et al, 2012). In medaka, the mature forms of miR-181a and b are perfectly conserved compared to their mammalian counterparts, both in terms of sequence identity (100%) and of pattern of expression, particularly in the retina (Carrella et al, 2015). MO-mediated silencing of miR-181a/b in medaka leads to increased levels of the majority of the targets involved in mitochondrial functions and in autophagy (Fig. 10). Interestingly, downregulation of miR-181a/b in either MLS medaka models led to a notable reduction of cell death in the brain and eye, as showed by TUNEL and caspase activation assays in figure 2. Accordingly, miR-181a/b downregulation resulted in a full rescue of the disease phenotype in about 50% of both hccs (Fig. 3 A-C, M) and cox7B morphants (Fig. 3 G-I, N), while MO-mediated silencing of miR-181a/b did not cause any obvious morphological alteration (Fig. 11 A, B). These data demonstrate that the downregulation of miR-181a/b ameliorates the phenotype in both MRC complex III and IV defective models indicating that the protective effect of miR-181a/b silencing in MLS models is gene-independent.

Since miR-181a/b regulates mitophagy and autophagy in the eye, the inventors tested whether said processes are implicated in the amelioration of the phenotype mediated by miR-181a/b downregulation. For this purpose, *hccs*-MO/miR-181a/b-MO- and *cox7b*-MO/miR-181a/b-MO-injected embryos were treated with Bafilomycin A1 (Baf-A1), a general autophagy inhibitor, at 50 nM. This concentration is able to block autophagy without inducing any obvious morphological abnormality in control embryos (Fig. 11 C, F). Interestingly, Baf-A1 treatment abolished the protective effect of miR-181a/b downregulation in a significant number of both *hccs*-MO/miR-181a/b-MO (Fig. 3 D, M) and *cox7b*-MO/miR-181a/b-MO embryos (Fig. 3 J, N), indicating that increased autophagy/mitophagy contributes substantially to the phenotype amelioration in the MLS medaka models.

Since miR-181a/b downregulation also increased the mRNA levels of *erk2,* a key component of the MAPK/ERK cascade, and of *bcl2* and *mcl1,* members of the Bcl2 antiapoptotic family as shown in figure 10, the inventors also tested whether the latter two pathways were involved in the amelioration of the MLS phenotype. Therefore, the inventors treated *hccs*-MO/miR-181a/b-MO- and *cox7b*-MO/miR-181a/b-MO-injected embryos with PD98059, a selective inhibitor of the MAPK/ERK pathway(Alessi et al, 1995), or with HA14-1, an inhibitor of Bcl-2 proteins(Wang et al, 2000b). PD98059 and HA14-1 were used at concentrations that did not induce any morphological alterations in control embryos [(Carrella et al, 2015) and Fig. 11 D, E]. PD98059 treatment did not have any effect on the extent of phenotype rescue in neither MLS models (Fig. 3 E, K, M, N), suggesting that the MAPK pathway is not primarily involved in the protective effect exerted by miR-181a/b downregulation in the analyzed MLS models. On the other hand, HA14-1 significantly reduced the extent of phenotype rescue in *hccs*-MO/miR-181a/b-MO-injected embryos, but not in *cox7b*-MO/miR-181a/b-MO-injected embryos (Fig. 3 F, L-N). Taken together these data suggest that increased autophagy/mitophagy are involved in the amelioration of the MLS phenotype in both models, indicating a primary role for this pathway in the miR-181a/b-downregulation-mediated neuronal protection. Moreover, increased levels of *bcl2* play a role in the amelioration of hccs-defective embryos only, indicating that the contribution of the Bcl2-mediated apoptotic pathway may vary depending on the specific mitochondrial defect.

### Example 3

### miR-181a/b inhibition ameliorates the phenotype of a mouse model of LHON syndrome

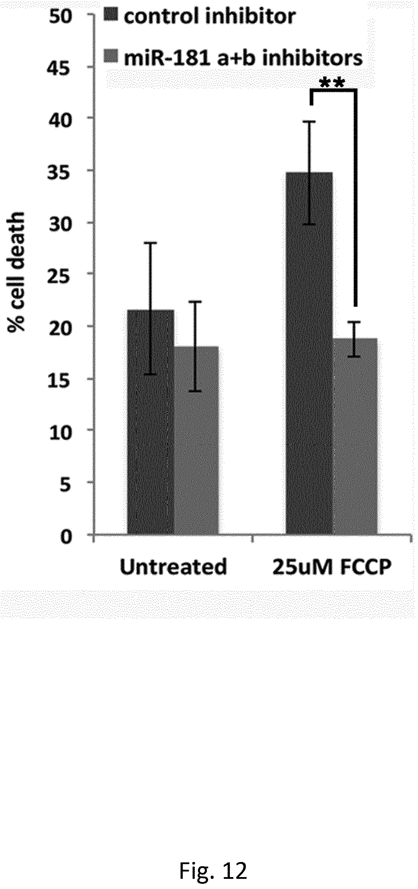

The efficacy of inhibition of miR-181a/b was further tested in a drug-induced mouse model of Leber hereditary optic neuropathy (LHON). LHON is a non-syndromic form of mitochondrial optic neuropathy characterized by Retinal Ganglion Cells (RGC) degeneration that leads to loss of central vision. LHON represents one of the most frequent forms of MD with a prevalence of 1:30000 individual with an onset occurring between the ages of 15 and 35 (Carelli et al, 2004). Similar to other MDs, LHON is characterized by high genetic heterogeneity since it is caused by point mutations in multiple genes encoded by the mitochondrial genome (Carelli et al, 2004; Meyerson et al, 2015). In about 95% of LHON patients, mutations are located in the *ND1, ND4,* or *ND6* genes, which encode complex I subunits (Carelli et al, 2004; Meyerson et al, 2015). Intravitreal injection of rotenone, an MRC complex I inhibitor, leads to a selective damage of RGCs, and injected mice are considered a reliable drug-induced LHON model (Carelli et al, 2013). In order to assess whether the genetic inactivation of miR-181a/b exerts a protective effect against rotenone-induced LHON, *miR-181a*/*b-1*^{*+*/*+*} and *miR-181a*/*b-1*^{*-*/*-*} mice were intravitreally injected with rotenone unilaterally. As internal controls, contralateral eyes were injected with DMSO. As previously reported (Heitz et al, 2012), *miR-181a*/*b-1*^{*+*/*+*} control mice injected with rotenone displayed a notable reduction in the number of RGCs (Fig. 4 A, B). In contrast, there was no difference in the number of RGCs between rotenone- and DMSO-injected retina in *miR-181a*/*b-1*^{*-*/*-*} mice, both one and two weeks after the injection (Fig. 4 A, B). Interestingly, rotenone-injected *miR-181a*/*b-1*^{*-*/*-*} mice also showed preserved MRC complex I activity in RGCs, as demonstrated by the staining of NADH dehydrogenase activity, with respect to rotenone-injected *miR-181a*/*b-1*^{*+*/*+*} mice (Fig. 4C). Accordingly, CoxIV immunofluorescence analysis highlighted that *miR-181a*/*b-1* depletion protects mitochondrial network integrity from rotenone-induced damage (Fig. 4D). Finally, the inventors also tested retinal function by Optokinetic Response (OKR). For this analysis *miR-181a*/*b-1*^{*+*/*+*} and *miR-181a*/*b-1*^{*-*/*-*} mice were bilaterally injected with rotenone or DMSO. Interestingly, *miR-181a*/*b-1*^{*+*/*+*} mice showed a severe decrease of visual performance starting from 1 week after rotenone administration, whereas *miR-181a*/*b-1*^{*-*/*-*} mice were significantly protected against rotenone-induced toxicity (Fig. 4E). Ablation of *miR-181a*/*b-1* rescued visual dysfunction even two weeks after rotenone injection (Fig. 4 A, E). Importantly, *miR-181a*/*b-1* ablation does not cause *per se* abnormality in retinal morphology and RGCs number [as shown by immunofluorescence analysis with representative retinal cell markers (Fig. 12 A-J, M)] nor alteration of retinal function [as assessed by Electroretinogram (ERG) analysis (Fig. 12 K-L)]. Altogether, these data indicate that miR-181a/b inhibition exerts a protective effect in a mammalian model of LHON and therefore represents a therapeutic strategy for this condition.

### Example 4

### miR-181a/b inactivation rescues the LHON phenotype in the Ndufs4^{-/-} mouse model.

Rotenone-injected mice are considered a reliable model of LHON (Carelli et al, 2013). However, the latter represents an acute model in which it is difficult to dissect the mechanism by which miR-181a/b downregulation exert its protective function. Therefore, the inventors exploited the *Ndufs4*^{*-*/*-*} mouse model (Kruse et al, 2008) to further characterize and validate the efficacy of miR-181a/b inactivation in mammalian models of MDs. Homozygous mutations in *NDUFS4* are responsible for one of the most severe forms of Leigh Syndrome (LS), an inherited neurodegenerative condition, characterized by poor prognosis as patients typically die before 3 years of age. NDUFS4 is a nuclear DNA-encoded MRC Complex I subunit located in the NADH-dehydrogenase domain of the complex, responsible for its catalytic activity (Breuer et al, 2013; Stroud et al, 2016; Zhu et al, 2016). Mice with homozygous *Ndufs4* inactivation develop a severe encephalopathy resembling LS that results in death between 50 and 60 postnatal (P) days (Breuer et al, 2013; Kruse et al, 2008). In addition, these mice also show a LHON phenotype characterized by RGCs dysfunction starting from P16-P20 and leading to a detectable RGCs death after P42 (Song et al, 2017; Yu et al, 2015), which makes the *Ndufs4* knockout line a good model to study the effect of miR-181a/b inactivation in LHON. By crossing *miR-181a*/*b-1*^{*-*/*-*} with *Ndufs4*^{*-*/*-*} mice, we obtained *Ndufs4*^{*-*/*-*}/*miR-181a*/*b-1*^{*-*/*-*} animals to study the effect of miR-181a/b inactivation on the retinal phenotype. As previously reported (Yu et al, 2015), *Ndufs4*^{*-*/*-*} mice showed a significant reduction in the number of RGCs at P55, as confirmed by NeuN-positive cells count (Fig. 5A,B,E). This reduction was rescued in *Ndufs4*^{*-*/*-*}/*miR-181a*/*b-1*^{*-*/*-*} retinas (Fig. 5C,E), in which the number of NeuN-positive cells was indistinguishable from both WT and *miR-181a*/*b-1*^{*-*/*-*} mice (Fig. 5D,E). Moreover, the OKR test showed a significant amelioration of visual acuity in *Ndufs4*^{*-*/*-*}/*miR-181a*/*b-1*^{*-*/*-*} compared to *Ndufs4*^{*-*/*-*} mice (Fig. 5F) at P25, indicating a remarkable effect of miR-181a/b downregulation on both RGCs dysfunction and death. Finally, the ERG response, previously reported to be altered in *Ndufs4*^{*-*/*-*} mice with the absence of b-wave response (Kruse et al, 2008; Yu et al, 2015) was also analysed.. A significant amelioration of the b-wave (both in scotopic and photopic ranges) in *Ndufs4*^{*-*/*-*}/*miR-181a*/*b-l^{-l-}* with respect to *Ndufs4*^{*-*/*-*} mice was observed (Fig. 5G-H), indicating a significant improvement of retinal response to light stimuli at P30. Altogether these results indicate that in the *Ndufs4*^{*-*/*-*} model miR-181a/b downregulation preserves RGCs from death and ameliorates the visual function before the miRNAs exert their activity on RGCs death.

To gain insight into the molecular mechanisms underlying the amelioration of the miR-181a/b inhibition-induced *Ndufs4*^{*-*/*-*} retinal phenotype, the expression levels of miR-181a/b targets in *Ndufs4*^{*-*/*-*}/*miR-181a*/*b-1*^{*-*/*-*} mice was analyzed and a significant increase of the *Nrf1* and *Park2* transcripts as compared to *Ndufs4*^{*-*/*-*} was observed (Fig. 6A). As previously mentioned, *Park2* and *Nrf1* are important players in the control of, respectively, mitophagy (Narendra et al, 2008) and mitochondrial biogenesis (Finck & Kelly, 2006; Wu et al, 1999), i.e., pathways that we showed to be upregulated in *miR-181a*/*b-1*^{*-*/*-*} vs. WT mice (Fig. 1). The inventors therefore hypothesized that the activation of these pathways significantly contributes to the phenotypic improvement observed in *Ndufs4*^{*-*/*-*}/*miR-181a*/*b-1*^{*-*/*-*} mice. Notably, the levels of Parkin and p62 were increased in mitochondrial fractions from *Ndufs4*^{*-*/*-*}/*miR-181a*/*b-1*^{*-*/*-*} with respect to *Ndufs4*^{*-*/*-*} mice (Fig. 6C, D) indicating enhanced mitophagy activation in response to miR-181a/b depletion. Electron Microscopy (EM) studies showed lack of accumulation of autophagosomes/autolysosomes containing undigested material excluding a possible mitophagy block at the level of autophagosomes recruitment or autophagosome/lysosome fusion (Fig. 7B). Moreover, consistent with *Nrf1* upregulation, an increased number of mitochondria at both P30 and P55 in RGCs of *Ndufs4*^{*-*/*-*}/*miR-181a*/*b-1*^{*-*/*-*} with respect to *Ndufs4*^{*-*/-}mice was seen by EM (Fig. 7A-C). The augmented mitochondrial biogenesis was also confirmed by increase of mtDNA in *Ndufs4*^{*-*/*-*}/*miR-181a*/*b-1*^{*-*/}*⁻ vs. Ndufs4*^{*-*/*-*} mice, as measured by q-PCR (Fig. 7D). Interestingly, the increase in number of mitochondria in RGCs of *Ndufs4*^{*-*/*-*}/*miR-181a*/*b-1*^{*-*/-} mice was accompanied by a notable amelioration of their morphological structure, such as increased number of cristae and more electrondense matrix, with respect to *Ndufs4*^{*-*/*-*} mice (Fig. 7A, B). Notably *NRF1* downregulation significantly abolished the mir-181a/b-mediated rescue in SH-SY5Y cells treated with FCCP, indicating that indeed NRF1 is implicated in the neuroprotective effect exerted by miR-181a/b downregulation (Fig. 6B). Finally, the biochemical activity of MRC complexes I, II and IV was also analysed. The latter analyses showed an increase in complex I and II activities, and a similar trend for complex IV activity, normalized to the activity of the Krebs cycle enzyme citrate synthase in the *Ndufs4*^{*-*/*-*}/*miR-181a*/*b-1*^{*-*/*-*} mice compared with the *Ndufs4*^{*-*/*-*} mice at P30 (Fig. 7E), indicating that miR-181a/b downregulation is partially able to counteract the complex I biochemical defect of the MRC in the *Ndufs4*^{*-*/*-*} mouse. Altogether these data indicate that miR-181a/b downregulation exerts its protective effect mainly through the simultaneous stimulation of mitophagy and mitochondrial biogenesis, which leads to enhancement of mitochondrial turnover in the retina that ultimately results in amelioration of the mitochondrial morphological and biochemical phenotypes.

### Example 5

### miR-181a/b inhibition protects dopaminergic neurons in 6-OHDA-induced models of Parkinson's Disease

miR-181a/b are significantly expressed in the substantia nigra (SN) and in the striatum [(Boudreau et al, 2014) and Fig. 10B)] and were previously shown to be upregulated in the brain of PD patients The effect of miR-181a/b inactivation in drug-induced models of PD generated in fish and mice using the neurotoxin 6-hydroxydopamine (6-OHDA) was evaluated. 6-OHDA induces a selective death of nigro-striatal dopaminergic (DA) neurons in fish (Parng et al, 2007) and mice (Jagmag et al, 2015) and is largely used to investigate motor and biochemical dysfunctions in PD. miR-181a and miR-181b expression in brain is conserved in medaka, with a strong signal in the *locus coeruleus* where teleost DA neurons are clustered (Fig. 8A). In control medaka larvae, 6-OHDA treatment led to the loss of about 30% of DA neurons in the *locus coeruleus,* as shown by tyrosine hydroxylase (TH) immunostaining and count (Fig. 8 B, C, E). Conversely, animals injected with miR-181a/b MOs described in [39] (MO-miR-181a 5'-AACTCACCGACAGCGTTGAATGTTC-3' SEQ ID NO. 111; MO-miR-181b 5'-AACCCACCGACAGCAATGAATGTTG-3' SEQ ID NO. 112) were protected against 6-OHDA toxicity and displayed no significant differences in the number of TH-positive cells as compared to untreated controls (Fig. 8 B, D, E).

In addition, a murine PD model generated by stereotaxic injection of 6-OHDA was analyzed. In line with reported data (Jagmag et al, 2015), unilateral injection of 6-OHDA in the SN of *miR-181a*/*b-1*^{*+*/*+*} mice resulted in a massive destruction of DA neurons (Fig. 9A) and their terminals in the striatum (Fig. 9C), compared to the control saline-injected side, as shown by immunostaining and count of TH-positive cells (Fig. 9 A, C, E). Interestingly, *miR-181a*/*b-1*^{*-*/*-*} mice showed an increased survival of DA neurons in the 6-OHDA-injected side (Fig. 9 B, D, E). Contralateral rotation induced by L-DOPA is the most widely used read-out of nigral and striatal DA neuronal degeneration in rodents unilaterally injected with 6-OHDA {De Leonibus, 2009}. 6-OHDA-treated *miR-181a*/*b-1*^{*-*/*-*} mice injected with L-DOPA showed lower rotational behavior compared to *miR-181a*/*b-1*^{*+*/*+*} mice, thus confirming a reduced extent of denervation in *miR-181a*/*b-1*^{*-*/*-*} animals (Fig. 9F). Notably, similar to what observed in the retina, miR-181a/b-1 ablation did not cause *per se* any obvious morphological abnormality in the SN, as shown by NISSL staining (Fig. 9E).

Altogether the present data indicate that miR-181a/b downregulation ameliorates the phenotype of preclinical models of PD by protecting DA neurons from 6-OHDA-induced death in medakafish and mice.

### Example 6

### miR-181 a/b inhibition in age-related macular degeneration

Mitochondrial dysfunction has been implicated in the pathophysiology of several age-related diseases, including age-related macular degeneration (AMD), a complex multifactorial degenerative disease. Vision impairment in AMD results from photoreceptor damage but the primary pathogenic event involves retinal pigment epithelium (RPE) degeneration. In AMD, accumulation in the RPE of lipofuscin, a product of photoreceptor outer segments (POS) turnover, has been hypothesized to be the primary cause of the disease leading to RPE dysfunction and apoptosis through production of Reactive Oxygen Species (ROS). Increasing evidence indicates that impaired autophagy and mitochondrial dysfunction in both RPE and photoreceptors, exacerbate oxidative stress and contribute to the pathogenesis of AMD. The inventors tested the efficacy of miR-181a/b inhibition in ameliorating the AMD phenotype. In particular, an in vitro model of AMD consisting in the ARPE-19 cell line (RPE) loaded with lipofuscin (A2E) was used. Accumulation of A2E in ARPE-19 could be measured by quantification of autofluorescence. ARPE-19 cells were transfected with Locked nucleic acid (LNA) oligonucleotide antisense, that inhibit miR-181 activity, and compared them to control samples. Two days after transfection, control and LNA-miR-181 transfected cells, were loaded with 100µM of A2E for 6 hours (Fig. 16A). miR-181 inhibition reduces A2E autofluorescence of about 50%, suggesting decreased accumulation of lipofuscin compared to control cells, quantified in the graph (A2E intensity mean, Fig 16B). Moreover, the miR-181 downregulation led to a decrease of A2E spot intensity and A2E spot area compared to control cells (Fig 16B), suggesting that miR-181 inhibition leads to amelioration of retinal pathology related to lipofuscin accumulation.

AMD is mostly caused by environmental and lifestyle factors, but also by genetic factors, such as polymorphism in *ABCA4* that enhance AMD risk. Recessive mutations in the *ABCA4* gene are responsible for Stargardt disease, a macular dystrophy characterized by lipofuscin accumulation, a condition that is commonly found in AMD. Hence the *Abca4*^{*-*/*-*} mouse model is considered a suitable in vivo model for the evaluation of AMD therapeutic strategies. The inventor tested the effect of miR-181a/b downregulation crossing *Abca4*^{*-*/*-*} mouse with *miR-181a*/*b-1*^{*-*/*-*} mouse. Interestingly, in the RPE of *Abca4*^{*-*/*-*}/*miR-181a*/*b-1*^{*-*/*-*} mouse they observed increased levels of miR-181a/b target mRNAs as well as of *Sod1* and *Cat1* that encode for antioxidant protein (Fig.17A). *Abca4*^{*-*/*-*} mice present marked accumulation of A2E in the RPE and photoreceptor outer segments (POS) that appears to have a key role in retinal disease pathophysiology. The levels of lipofuscin autofluorescence (excitation: 560 ± 40 nm; emission: 645 ± 75) was evaluated on retinal cryosections (in either the RPE alone and in POS). Interestingly preliminary data on 5-months-old mice, show a decrease of lipofuscin autofluorescence (AF) in *Abca4*^{*-*/*-*}/*miR-181a*/*b-1*^{*-*/*-*} RPE and POS compared to *Abca4*^{*-*/*-*} mice (Fig.17B,C).

### Example 7

### miR-181 inhibition in retinitis pigmentosa

Mutations in the rhodopsin gene are a cause of dominant and recessive retinitis pigmentosa (RP), accounting for about 10% of all cases. Dominant rhodopsin mutants are therefore gain-of-function or dominant-negative mutants. Several classes were defined:
- class I, are like wild type (wt) rhodopsin in their ability to localize to the cytoplasmic membrane and to bind 11-cis-retinaldehyde to form spectrally active rhodopsin; the mutations that affect the ciliary targeting signal, VXPX, are mislocalised, including at the synapse, where they could inhibit synaptic vesicle fusion or be abnormally activate transducin.
- class II, are probably defective in protein folding and/or stability, accumulate abnormally in the rough endoplasmic reticulum, are not transported efficiently to the cytoplasmic membrane and fail to bind 11-cisretinaldehyde to form a spectrally active photopigment;
- class III, constitutively activate transducin.

Among the class I mutants are those affecting proline-347 near the carboxyl terminus. This residue is conserved among all known visual pigments. Six different missense mutations affecting this residue have been identified among patients with RP, indicating that proline-347 mediates some vital function of rhodopsin.

Transgenic mice carrying a human proline-347 to serine (P347S) mutation (ref PMID:8943080) develop photoreceptor degeneration. At one month of age (P30) the photoreceptor layer is already dramatically reduced, and at three months (P90) only one row of photoreceptor cell bodies is present in the P347S retina. Concordantly the Electroretinogram (ERG) response results to be dramatically reduced at P30 and almost null at P90.

The inventors crossed the P347S transgenic animals with miR-181a/b-1 heterozygous animals. Retina from P347S transgenic mice was compared to retina from P347S/miR-181a/b +/- mice from the same litter. Mice were characterized at P30 and P90 with ERG test and by histological and molecular analysis.

The ERG response (b-wave) of P347S/miR-181a/b +/- retina compared to P347S retina at P30 is significantly higher as shown in Figure 18 (N=16). The photopic response, due to cones functionality, shows less increase. In accordance, immunofluorescence (IF) staining for two main markers of rods (Rhodoposin, Rhod) and cones (C-arrestin, C-arr), respectively, showed increased levels of rhodopsin stainig and no differences in C-arrestin staining (Fig. 19A), despite the outer nuclear layer (ONL) thickness showed no difference (Fig. 19B). The increased levels of Rhodopsin, as well as other photoreceptor markers, were confirmed also by qRT-PCR analysis reported in Fig. 19C. Moreover at P30 the inventors also observed an amelioration of Outer Segment (OS) length, quantified in the graph shown in Fig 20.

At P90 the ERG response of P347S/miR-181a/b +/- animals results to be still significantly higher compared to P347S animals (Fig 21). The IF staining for Rhod and C-arr showed increased levels of both markers at P90 (Fig 22) and the histological sections reveals that the P347S/miR-181a/b+/- ONL is constituted of more than one row of photoreceptor cell bodies, the difference compared to the P347S ONL is reported in the graph in Fig. 22B. The increased levels of Rhodopsin and C-arrestin, as well as other photoreceptor markers, were analysed also by qRT-PCR analysis reported in Fig. 22C.

Transcriptome data from the P347S mouse model (Fig. 23A) which revealed that in the P347S mouse model there is a strong downregulation of mitochondria-related genes and pathways in addition to data showing how the miR-181a/b can regulate mitochondrial-related pathways, prompted the inventors to analyze the mitochondrial phenotype in P347S mice harboring heterozygous deletion of the miR-181a/b-1 genomic cluster.

In order to understand how the miR-181a/b downregulation ameliorates the retinal phenotype of P347S mice, the inventors verified whether the miR-181a/b validated targets were upregulated in the retinas of P347S/miR-181a/b^{+/-} mice compared to the P347S model. The molecular analysis was performed by qRT-PCR (Fig. 23B) on the following miR-181a/b validated targets: *Ppargc1a* and *Nrf1,* which are involved in mitochondrial biogenesis; *Cox11* and *Coq10b,* which are components of the mitochondrial respiratory chain; *Prdx3,* which encodes a protein with antioxidant function, *Erk2, Atg5* and *Park2,* which are involved in the regulation of autophagy/mitophagy pathway; *Xiap,* which plays a crucial role in the regulation of mitochondria-mediated cell death; *Bcl2* and *Mcl1,* which are anti-apoptotic and pro-survival genes. The inventors found an upregulation of all of the targets: *Cox11, Erk2, Mcl1, Bcl2, Nrf1, Coq10b, Xiap, Ppargc1a, Park2 and Prdx3,* except for *Atg5.* All these data together suggest that the upregulation of the above miR-181a/b target genes can in principle contribute to the PR protection observed in P347S/miR-181a/b^{+/-} mice.

To gain further insight into the protective effect exerted by the depletion of the miR-181a/b-cluster-1 in P347S mice, the inventors performed a cell death assay. As previously shown, miR-181a/b target a number of anti-apoptotic and pro-survival genes. First, they performed immunofluorescence on frozen retinas for anti- active Caspase3 in P347S and P347S/miR-181a/b^{+/-}mice (Fig. 24A). Caspase-3 is activated in apoptotic cells both by extrinsic (death ligand) and intrinsic (mitochondrial) pathways. This protein exists as inactive proenzyme that undergoes proteolytic processing to produce an active enzyme playing a major role in the apoptotic pathway. As revealed by the immunofluorescence assay and its quantification (Fig. 24B), in the P347S/miR-181a/b^{+/-} mouse model there is a significative reduction of anti- active Caspase3 positive cells, indicating that the miR-181a/b downregulation indeed protects cells from apoptosis. Moreover the inventors confirmed the data using an additional marker of cell death, the TUNEL-POD assay. Terminal deoxynucleotidyl transferase dUTP nick end labeling (TUNEL) is a method for detecting DNA fragmentation by labeling 3'- hydroxyl termini. Hallmark of cell death is DNA degradation. The DNA cleavage may yield double-stranded and single-stranded DNA breaks (nicks). Both types of breaks can be detected by labeling the free 3'-OH termini with modified nucleotides (e.g., biotin-dUTP, fluorescein-dUTP) in an enzymatic reaction. The enzyme terminal deoxyribonucleotidyl transferase (TdT) catalyzes the template-independent polymerization of deoxyribonucleotides to the 3'-end of single- and double-stranded DNA. Using this technique, it is possible to detect all types of cell death independently from the mechanism. As shown in Fig 25 A, in which dying cells are labeled in dark grey and indicated by arrows, and quantified in fig. 25B, there is a strong reduction of dead cells in the retinas of P347S/miR-181a/b^{+/-} vs. P347S mice.

All these data together show that the miR-181a/b-cluster-1 depletion protects cells from death in P347S mice.

Accordingly with the transcriptome data (Fig. 23A), Transmission Electron Microscopy (TEM) revealed that in the P347S mouse model there is a mitochondria phenotype characterized by smaller and fragmented mitochondria (Fig 26A).

By TEM analysis, the retina of P347S/miR-181^{+/-} mice displayed a better structure compared to P347S/miR-181^{+/+}. The OS of P347S/miR-181^{+/+} mice is completely destroyed whereas in P347S/miR-181^{+/-} animals both OS and IS are better organized and packaged (Fig 26A). Moreover, the difference in mitochondrial morphology between the two models is striking. In P347S retinas, mitochondria are fragmented and very small. Conversely, in P347S/miR-181^{+/-} animals, mitochondria are more elongated and with a better morphology (Fig 26A, quantified in graph in Fig. 26B) consistently with all the previous data indicating the involvement of miR-181 in mitochondrial functionality.

The mitochondria-related phenotype amelioration was further investigated by WB. The inventors analyzed the protein levels of Mfn2 (Mitofusin) a GTPase located in the outer mitochondria membrane (Fig. 27A). Mfn2 is a protein involved in mitochondrial fusion. Within the cell, mitochondria exist in an ever-changing dynamic state, constantly elongating and dividing (fusion and fission respectively). Fission plays a role in segregating dysfunctional mitochondria while, on the other hand, fusion has been shown to aid in equilibrium of matrix metabolites and intact mtDNA. Certain damaged mitochondria can fuse with other healthy mitochondria in an attempt to rescue the damaged ones. The balance of these two events is thought to be essential for mitochondrial homeostasis, cell stability and cell survival. In particular mitochondria fusion is mediated by three different GTPases including Mfn2. The WB analysis revealed a trend towards an increase of Mfn2 protein levels in P347S/miR-181a/b^{+/-}compared to P347S mice. It is tempting to speculate that Mfn2 upregulation could be implicated in the amelioration of mitochondrial morphology visible by TEM in mouse retinas of P347S/miR181-a/b^{+/+} mice. The inventors also performed an OXPHOS BLOT to analyze the MRC (Fig. 27B). This procedure relies on a western blot in which antibodies against specific antigens of the five mitochondrial chain complexes are simultaneously used. They observed a downregulation of these proteins in the P347S mouse model with respect to WT mouse eyes. In the P347S/miR-181a/b^{+/-} model, was detected a significant rescue of protein levels. In addition they found an upregulation of protein levels for the general mitochondrial marker Citrate Synthase (Cs) an enzyme involved in the first step of the citric acid cycle (Krebs cycle) (Fig. 27C). This protein is localized in the mitochondrial matrix and can provide an indication of an increase of the mitochondrial mass in P347S/miR-181a/b^{+/-} eyes respect to P347S eyes. Data are quantified in the graph in Figure 27D.

### Example 8

### In vitro experimental validation of miR-181 sponge efficiency

AAV vectors (serotype 2) expressing a "sponge" construct for miR-181 inhibition (see miR-181 Sponge Sequence above) under the control of the cytomegalovirus (CMV) constitutive promoter was generated (AAV.CMV.miR-181.sponge). The AAV2 serotype was already reported to efficiently transduce RGCs upon intravitreal administration in several species, including mice and non-human primate (Koilkonda et al, 2014a; Koilkonda et al, 2014b; Kwong et al, 2015; Tshilenge et al, 2016). This AAV serotype is commonly used by researchers involved in gene therapy for retinal diseases (Botta et al, 2016; Maguire et al, 2009; Maguire et al, 2008; Mussolino et al, 2011). miRNA sponges are RNA molecules with tandemly repeated miRNA antisense sequences (miRNA binding sites (MBS) that can sequester miRNAs from their endogenous targets and are valuable tools to achieve long-term miRNA loss-of-function in vivo(Ebert & Sharp, 2010). miRNA sponges have been successfully applied in animal models (Ebert & Sharp, 2010; Tay et al, 2015). Sponge design was carried out according to (Ebert et al, 2007; Gentner et al, 2009; Kluiver et al, 2012). Briefly the inventors designed 6 MBS antisense to miRNA181a and 6 MBS antisense to miRNA181b with a central mismatch ("bulge") at position 9-12 of the miR-181a or b sequences. The bulge was created by changing the nucleotides at position 9-12 to reduce the chances of base paring (including G-U wobbling). The two MBS will be separated by a 4 nt sequence ("spacer"). Sponge oligonucleotide duplexes (double stand) were cloned in a vector containing an expression cassette with a miRNA binding sponge sequence inserted into the 3' UTR of a GFP reporter gene (Karali et al, 2011).

qRT-PCR analysis of miR-181a/b direct target transcripts (XIAP, NRF1, PRDX3, MCL1, ATG5, SIRT1, BCL2, BCL2, ERK2, PARK2, COX11) in SH-SY5Y cells upon 48h transfection with AAV2.1-CMV-eGFP-miR-181Sponge (Sponge 181). Transcript levels of miR-181a/b targets are increased in Sponge 181 sample compared to control sample (CTRL= AAV2.1-CMV-eGFP transfected cells, N=3) (Fig. 28). This data indicates that the miR-181 Sponge is sequestering the endogenous miR-181, thus preventing the binding of their direct targets and inhibiting their activity

One of the pathways modulated by miR-181a/b is mitochondrial-dependent cell death in which the Bcl-2 protein family has a key regulatory role. This protein family controls mitochondrial outer membrane permeabilization (MOMP), the formation of an inner membrane channel termed mitochondrial permeability transition pore (PTP) and the release of cytochrome c and of other apoptotic triggers (Rasola & Bernardi, 2007; Tait & Green, 2010). Although the contribution of apoptosis to the pathogenesis of primary MDs is not univocally established, increasing the levels of anti-apoptotic Bcl-2 proteins may represent an effective strategy to prolong cell survival and slow down disease progression. Notably, inhibition of MOMP and PTP, either by overexpression of the Bcl-2 family protein Bcl-xL or by cyclosporine A (CsA) treatment, counteracted increased cell death and ameliorated the phenotype of an hccs-deficient MLS medaka model (Indrieri et al, 2013). The importance of mitochondrial-dependent cell death in diseases associated with mitochondrial dysfunction has also been demonstrated by the positive effect of CsA treatment in patients with Ullrich congenital muscular dystrophy and Bethlem myopathy (Merlini et al, 2008), and in animal models of PD (Tamburrino et al, 2015). Here the inventors show that miR-181a/b inhibition leads to increased levels of Bcl-2 family members, such as Bcl-2 and Mcl-1, and rescues the phenotype in both MLS models tested. Moreover, HA14-1, a potent inhibitor of Bcl-2 proteins (Wang et al, 2000b), significantly abolishes the miR-181a/b-mediated amelioration of the MLS phenotype in the hccs-defective, but not in the cox7b-defective model. These data indicate that a single pathway, e.g., mitochondrial-dependent cell death, and its modulation, may have different effects on highly related forms of MDs, depending on the underlying genetic defect. Moreover, this observation strengthens our hypothesis that acting simultaneously on more than one pathway represents a more effective strategy to treat the genetically heterogeneous MDs.

Increased autophagy and mitophagy have been shown to exert protective effects in different neurodegenerative diseases including PD (Decressac et al, 2013; Martinez-Vicente, 2017). Of note, two gene products mutated in early-onset familial forms of PD, i.e., Parkin and PINK1 are involved in the clearance of damaged mitochondria via the autophagy pathway (Geisler et al, 2010; Vives-Bauza et al, 2010). Moreover, treatment with the mTOR inhibitor rapamycin, which activates autophagy, significantly delayed both neurodegeneration progression and the fatal outcome of the Ndufs4^{-/-} mouse, a model for Leigh syndrome, one of the most severe forms of MD (Johnson et al, 2013). Here, the inventors show that the downregulation of miR-181a/b leads to increased autophagy and mitophagy in the CNS. In agreement with these data, the inhibition of general authophagy in both hccs/miR-181a/b-MOs and cox7B/miR-181a/b-MOs embryos reduce the neuroprotective effect of miR-181a/b downregulation on the MLS phenotype, indicating that increased autophagy/mitophagy significantly contributes to the phenotypic rescue in both models.

Although the removal of dysfunctional mitochondria would limit the risk of apoptosis, an uncontrolled activation of mitophagy without an appropriate compensatory mitochondrial biogenesis may contribute to further mitochondrial dysfunction resulting in loss of mitochondrial mass, energetic collapse and cell death (Villanueva Paz et al, 2016; Zhu et al, 2013). Furthermore, enhanced mitochondrial biogenesis could be *per se* protective in MDs. Variability in mitochondrial DNA content and mitochondrial biogenesis are indeed associated with incomplete penetrance in non-manifesting carriers of LHON (Giordano et al, 2014), and increased biogenesis was found to improve the phenotype of different *in vitro* and *in vivo* MD models (Komen & Thorburn, 2014). However, some discrepant and contradictory results obtained using compounds that trigger mitochondrial biogenesis (including bezafibrate, resveratrol and AICAR) (Komen & Thorburn, 2014) clearly indicate that additional work is needed to effectively exploit modulation of mitochondrial biogenesis to increase mitochondrial functionality in patients. The tight balance between mitophagy and mitochondrial biogenesis is emerging as a critical aspect in the determination of cell viability in diseases characterized by mitochondrial dysfunction (Zhu et al, 2013). Here the inventors show that miR-181a/b can simultaneously control both processes. Concomitantly to the increased mitophagy observed following miR-181a/b inactivation, the inventors observed increased levels of several mitochondrial proteins and of mtDNA, indicating enhanced mitochondrial biogenesis. Accordingly, miR-181a/b-depleted animals showed enhanced MRC complex I activity and preserved mitochondrial integrity in the rotenone-induced LHON model. Altogether, the inventors demonstrate that miR-181a/b represent novel controllers of mitochondrial turnover and this activity may play a key role in neuroprotection.

MDs are a genetically heterogeneous group of disorders. Although each individual disorder is rare, collectively the total prevalence of adult MDs is estimated to be of approximately 1 in 4300 representing one of the most prevalent groups of inherited neurological diseases (Gorman et al, 2015). In this study, the inventors demonstrate that downregulation of miR-181a/b exerts a neuroprotective effect in multiple models of MDs. Our results are of particular interest for genetically heterogeneous conditions, such as LHON for which a gene-independent therapeutic strategy is highly desirable. Moreover, the proof of principle data the inventors generated in *in vivo* models of PD highlight miR-181a/b as new possible therapeutic targets for this disease.

It is to be noted that *miR-181a*/*b-1*^{*-*/*-*} mice show normal life-span (Henao-Mejia et al, 2013) and no obvious abnormalities in the brain and eyes, which provides initial support to the safety of the downregulation of these two miRNAs in the CNS. The modulation of miR-181a/b in the CNS represent a promising strategy for the treatment of a number of rare and common neurodegenerative disorders associated with primary or secondary mitochondrial dysfunction that altogether represent an important burden for public health.

### REFERENCES

Alessi DR, Cuenda A, Cohen P, Dudley DT, Saltiel AR (1995) J Biol Chem 270: 27489-27494
Altschul SF, Gish W, Miller W, Myers EW, Lipman DJ (1990) J Mol Biol 215: 403-410
Barnes DE, Yaffe K (2011) Lancet Neurol 10: 819-828
Bartel DP (2009) MicroRNAs: target recognition and regulatory functions. Cell 136: 215-233
Bartel DP (2018) Metazoan MicroRNAs. Cell 173: 20-51
Bernard DG, Gabilly ST, Dujardin G, Merchant S, Hamel PP (2003) J Biol Chem 278: 49732-49742
Botta S, et al (2016) Rhodopsin targeted transcriptional silencing by DNA-binding. Elife 5
Boudreau RL,et al., (2014) Neuron 81: 294-305
Braasch DA, Corey DR (2002) Biochemistry 41: 4503-4510
Breuer ME, et al., (2013) IUBMB Life 65: 202-208
Broderick JA, Zamore PD (2011) MicroRNA therapeutics. Gene Ther 18: 1104-1110
Bustin SA, et al (2009) Clin Chem 55: 611-622
Carelli V, La Morgia C, Sadun AA (2013) Curr Opin Neurol 26: 52-58
Carelli V, Ross-Cisneros FN, Sadun AA (2004) Prog Retin Eye Res 23: 53-89
Carr HS, George GN, Winge DR (2002) J Biol Chem 277: 31237-31242
Carrella S, et al., (2015) Dev Neurobiol 75: 1252-1267
Cerutti R, et al (2014) Cell Metab 19: 1042-1049
Chadderton N, et al (2013) Eur J Hum Genet 21: 62-68
Cheng M, et al., (2016) Oncotarget 7: 42274-42287
Christopher AF, Kaur RP, Kaur G, Kaur A, Gupta V, Bansal P (2016) Perspect Clin Res 7: 68-74
Civiletto G, et al., (2018) EMBO Mol Med 10
Civiletto G, et al., (2015) Cell Metab 21: 845-854
Comitato A, et al., (2014) Invest Ophthalmol Vis Sci 55: 3555-3562
Crooke ST, et al (1996) J Pharmacol Exp Ther 277: 923-937
de Lau LM, Breteler MM (2006) Epidemiology of Parkinson's disease. Lancet Neurol 5: 525-535
De Mesmaeker A, Haener R, Martin P, Moser HEJAoCR (1995) Antisense oligonucleotides. 28: 366-374
Decressac M, et al., (2013) Proc Natl Acad Sci U S A 110: E1817-1826
Desbats MA, Lunardi G, Doimo M, Trevisson E, Salviati L (2015) J Inherit Metab Dis 38: 145-156
Ebert MS, Neilson JR, Sharp PA (2007) Nat Methods 4: 721-726
Ebert MS, Sharp PA (2010) MicroRNA sponges: progress and possibilities. RNA 16: 2043-2050
Elayadi AN, Corey DR (2001) Curr Opin Investig Drugs 2: 558-561
Finck BN, Kelly DP (2006) J Clin Invest 116: 615-622
Gebeyehu G, Rao PY, SooChan P, Simms DA, Klevan L (1987) Nucleic Acids Res 15: 4513-4534
Geisler S, et al., (2010) Nat Cell Biol 12: 119-131
Gennarino VA, et al (2012) Genome Res 22: 1163-1172
Gentner B, et al., (2009) Nat Methods 6: 63-66
Giordano C, et al (2014) Brain 137: 335-353
Gorman GS, et al (2015). Ann Neurol 77: 753-759
He Q, et al., (2013) Biochim Biophys Acta 1830: 5258-5266
Heasman J (2002) Dev Biol 243: 209-214
Hebert SS, De Strooper B (2009) Trends Neurosci 32: 199-206
Heitz FD, Erb M, Anklin C, Robay D, Pernet V, Gueven N (2012) PLoS One 7: e45182
Henao-Mejia J, et al., (2013) Immunity 38: 984-997
Hutchison ER, et al (2013) Glia 61: 1018-1028
Indrieri A, et al (2013) EMBO Mol Med 5: 280-293
Indrieri A, et al (2012) Am J Hum Genet 91: 942-949
Iwamatsu T (2004) Mech Dev 121: 605-618
Jagmag SA, Tripathi N, Shukla SD, Maiti S, Khurana S (2015) Front Neurosci 9: 503
Janssen HL, et al (2013) N Engl J Med 368: 1685-1694
Jepsen JS, Sorensen MD, Wengel J (2004) Oligonucleotides 14: 130-146
Ji J, et al (2009) Hepatology 50: 472-480
Johnson SC, et al (2013) Science 342: 1524-1528
Kabanov AV, et al., (1990) FEBS Lett 259: 327-330
Karali M, et al (2011) PLoS One 6: e22166
Karali M, et al (2016) Nucleic Acids Res 44: 1525-1540
Kluiver J, et al., (2012) Methods 58: 113-117
Koilkonda R, et al (2014a) Invest Ophthalmol Vis Sci 55: 7739-7753
Koilkonda RD, et al (2014b) JAMA Ophthalmol 132: 409-420
Komen JC, Thorburn DR (2014) Br J Pharmacol 171: 1818-1836
Koshkin AA, et al., (1998) Tetrahedron 54: 3607-3630
Kruse SE, et al., (2008) Cell Metab 7: 312-320
Kurreck J, Wyszko E, Gillen C, Erdmann VA (2002) Nucleic Acids Res 30: 1911-1918
Kwong JM, et al., (2015) Gene Ther 22: 138-145
Lacerra G, et al., (2000) Proc Natl Acad Sci U S A 97: 9591-9596
Letsinger RL, et al., (1989) Proc Natl Acad Sci U S A 86: 6553-6556
Levin JD, Fiala D, Samala MF, Kahn JD, Peterson RJ (2006) Nucleic Acids Res 34: e142
Lightowlers RN, Taylor RW, Turnbull DM (2015) Science 349: 1494-1499
Ling H, Fabbri M, Calin GA (2013) Nat Rev Drug Discov 12: 847-865
Lyseng-Williamson KA (2016). Drugs 76: 805-813
Maguire AM, et al (2009) Lancet 374: 1597-1605
Maguire AM, et al (2008) N Engl J Med 358: 2240-2248
Manoharan M, Johnson L, McGee D, Guinosso C, Ramasamy K, Springer R, Bennett C, Ecker D, Vickers T, Cowsert LJAotNYAoS (1992) Chemical modifications to improve uptake and bioavailability of antisense oligonucleotides. 660: 306-309
Manoharan M, Johnson LK, Bennett CF, Vickers TA, Ecker DJ, Cowsert LM, Freier SM, Cook PDJB, Letters MC (1994) Cholic acid-oligonucleotide conjugates for antisense applications. 4: 1053-1060
Manoharan M, Johnson LK, Tivel KL, Springer RH, Cook PDJB, Letters MC (1993) Introduction of a lipophilic thioether tether in the minor groove of nucleic acids for antisense applications. 3: 2765-2770
Manoharan M, Tivel KL, Cook PDJTL (1995) Lipidic nucleic acids. 21: 3651-3654
Martin PJHCA (1995) Ein neuer Zugang zu 2'-O-Alkylribonucleosiden und Eigenschaften deren Oligonucleotide. 78: 486-504
Martinez-Vicente M (2017) Front Mol Neurosci 10: 64
McTigue PM, Peterson RJ, Kahn JD (2004) Biochemistry 43: 5388-5405
Merlini L, et al., (2008) Proc Natl Acad Sci U S A 105: 5225-5229
Meyerson C, Van Stavern G, McClelland C (2015) Clin Ophthalmol 9: 1165-1176
Mishra RK, et al., (1995) Biochim Biophys Acta 1264: 229-237
Mussolino C, et al (2011) Gene Ther 18: 637-645
Narendra D, Tanaka A, Suen DF, Youle RJ (2008) J Cell Biol 183: 795-803
Nasevicius A, Ekker SC (2000) Nat Genet 26: 216-220
Nielsen PE, Egholm M, Berg RH, Buchardt O (1991) Science 254: 1497-1500
Oberhauser B, Wagner E (1992) Nucleic Acids Res 20: 533-538
Obika S, et al. (1998) Tetrahedron Letter 39: 5401-5404
Orum H, Wengel J (2001) Curr Opin Mol Ther 3: 239-243
Ouyang YB, Lu Y, Yue S, Giffard RG (2012) Mitochondrion 12: 213-219
Parng C, Roy NM, Ton C, Lin Y, McGrath P (2007) J Pharmacol Toxicol Methods 55: 103-112
Rasola A, Bernardi P (2007) Apoptosis 12: 815-833
Rodriguez-Blazquez C, et al., (2015) Expert Rev Pharmacoecon Outcomes Res 15: 889-911
Rodriguez-Ortiz CJ, et al., (2014) J Alzheimers Dis 42: 1229-1238
Sanchez MI, Crowston JG, Mackey DA, Trounce IA (2016) Pharmacol Ther
Shea RG, Marsters JC, Bischofberger N (1990) Nucleic Acids Res 18: 3777-3783
Song L, Yu A, Murray K, Cortopassi G (2017) Brain Res 1657: 232-244
Stroud DA, et al (2016) Nature 538: 123-126
Surace EM, et al (2005) Mol Ther 12: 652-658
Svinarchuk FP,et al., (1993) Biochimie 75: 49-54
Tait SW, Green DR (2010) Nat Rev Mol Cell Biol 11: 621-632
Tamburrino A, et al (2015) C Acta Neuropathol Commun 3: 84
Tay FC, Lim JK, Zhu H, Hin LC, Wang S (2015) Adv Drug Deliv Rev 81: 117-127
Tekirdag KA, Korkmaz G, Ozturk DG, Agami R, Gozuacik D (2013) Autophagy 9: 374-385
Tshilenge KT, al (2016) Hum Gene Ther Methods 27: 122-134
Villanueva Paz M, et al., (2016) Expert Opin Ther Targets 20: 487-500
Viscomi C, et al. (2011) Cell Metab 14: 80-90
Viscomi C, Bottani E, Zeviani M (2015) Biochim Biophys Acta 1847: 544-557
Viscomi C, et al., (2009) Hum Mol Genet 18: 12-26
Vives-Bauza C, et al (2010) Proc Natl Acad Sci U S A 107: 378-383
Wang J, et al. (2000a) Journal of the American Chemical Society 122: 8595-8602
Wang JL, et al. (2000b) Proc Natl Acad Sci U S A 97: 7124-7129
Wimplinger I, et al (2006) Am J Hum Genet 79: 878-889
Wonsey DR, Zeller KI, Dang CV (2002) Proc Natl Acad Sci U S A 99: 6649-6654
Wu Z, et al (1999) Cell 98: 115-124
You Y, Moreira BG, Behlke MA, Owczarzy R (2006) Nucleic Acids Res 34: e60
Yu AK, et al. (2015) Hum Mol Genet 24: 2848-2860
Zhang J, Madden TL (1997) Genome Res 7: 649-656
Zhu J, Vinothkumar KR, Hirst J (2016) Nature 536: 354-358
Zhu J, Wang KZ, Chu CT (2013) Autophagy 9: 1663-1676

## Claims

1. An inhibitor of miR-181 for use in the treatment and/or prevention of a mitochondrial disorder selected from the group consisting of: Leigh syndrome, Leber Hereditary optic Neuropathy (LHON), Mitochondrial Myopathy, Encephalopathy, Lactic Acidosis and Stroke Syndrome (MELAS), Myoclonic Epilepsy associated with Ragged-Red fibers (MERRF), Progressive External Ophthalmoplegia (PEO), Pearson's syndrome, Maternally inherited Myopathy and cardiomyopathy (MIMyCA), Kjer's optic neuropathy, Neuropathy ataxia and retinitis pigmentosa (NARP), Autosomal dominant optic atrophy, Kearns-Sayre syndrome, Friedreich's ataxia, Hereditary Spastic paraplegia, Charcot-Marie-Tooth disease, sporadic age-related neurodegenerative disease, Parkinson's Disease (PD), Alzheimer's Disease (AD), Age-related Macular degeneration (AMD), Diabetic Retinopathy, Glaucoma, familiar neurodegenerative disease, familiar PD, familiar AD, Huntington's disease, Retinitis Pigmentosa (RP), and Stargardt's disease, said inhibitor being an inhibitory nucleic acid agent comprising at least one sequence complementary to miR-181 wherein said inhibitor comprises a sequence complementary to ACAUUCA (SEQ ID NO. 5).

2. The inhibitor of miR-181 for use according to claim 1, wherein said miR-181 is a miR-181 precursor or a mature miR-181, preferably said miR-181 is selected from the group consisting of miR-181a-1, miR-181a-2, miR-181b-1, miR-181b-2, miR-181c, and miR-181d or a combination thereof.

3. The inhibitor of miR-181 for use according to claim 1 or 2, wherein said inhibitor comprises a sequence complementary to ACAUUCA (SEQ ID NO. 5) and has at least 80% sequence identity with a sequence complementary to SEQ ID No. 1 or SEQ ID No. 2 or SEQ ID No. 3 or SEQ ID No. 4.

4. The inhibitor of miR-181 for use according to any one of previous claims wherein said inhibitor is from 18 to 21 nucleotide long.

5. The inhibitor of miR-181 for use according to any one of previous claims, wherein the sequence further comprises one or more locked nucleic acid (LNA) nucleotides and one or more non-locked nucleotides, wherein at least one of the non-locked nucleotides comprises a chemical modification, preferably the locked nucleic acid (LNA) nucleotides has a 2' to 4' methylene bridge, preferably the chemical modification is a 2' O-alkyl or 2' halo modification.

6. The inhibitor of miR-181 for use according to any one of previous claims wherein said inhibitor consists of a sequence selected from: SEQ ID No. 6, SEQ ID No. 7, SEQ ID No. 8, SEQ ID No. 9, SEQ ID No. 10, SEQ ID No. 11, SEQ ID No. 12 or SEQ ID No. 13.

7. The inhibitor of miR-181 for use according to any one of previous claims wherein said inhibitor of miR-181 is used with at least one additional agent, preferably said at least one additional agent is an inhibitor of miR-181 as defined in any one of claims 1 to 6, preferably said at least one additional agent is selected from the group consisting of: an agent that increases mitochondrial biogenesis, an agent that modulates autophagy, an agent that inhibits apoptosis, a ROS scavenger, an agent that shifts heteroplasmy, a scavenger of toxic compounds, a supplementation of nucleotides, an agent that replaces the missing gene, preferably said inhibitor of miR-181 and said at least one additional agent are administered sequentially or simultaneously.

8. A pharmaceutical composition comprising at least one inhibitor of miR-181 as defined in any one of previous claims and a pharmaceutically acceptable carrier or diluent for use in the treatment and/or prevention of a mitochondrial disorder selected from the group consisting of: Leigh syndrome, Leber Hereditary optic Neuropathy (LHON), Mitochondrial Myopathy, Encephalopathy, Lactic Acidosis and Stroke Syndrome (MELAS), Myoclonic Epilepsy associated with Ragged-Red fibers (MERRF), Progressive External Ophthalmoplegia (PEO), Pearson's syndrome, Maternally inherited Myopathy and cardiomyopathy (MIMyCA), Kjer's optic neuropathy, Neuropathy ataxia and retinitis pigmentosa (NARP), Autosomal dominant optic atrophy, Kearns-Sayre syndrome, Friedreich's ataxia, Hereditary Spastic paraplegia, Charcot-Marie-Tooth disease, sporadic age-related neurodegenerative disease, Parkinson's Disease (PD), Alzheimer's Disease (AD), Age-related Macular degeneration (AMD), Diabetic Retinopathy, Glaucoma, familiar neurodegenerative disease, familiar PD, familiar AD, Huntington's disease, Retinitis Pigmentosa (RP), and Stargardt's disease, preferably the composition further comprises at least one additional agent, preferably said at least one additional agent is an inhibitor of miR-181 as defined in any one of claims 1 to 6, preferably said at least one additional agent is selected from the group consisting of: an agent that increases mitochondrial biogenesis, an agent that modulates autophagy, an agent that inhibits apoptosis, a ROS scavenger, an agent that shifts heteroplasmy, a scavenger of toxic compounds, a supplementation of nucleotides, an agent that replaces the missing gene.

9. A vector for use in the treatment and/or prevention of a mitochondrial disorder selected from the group consisting of: Leigh syndrome, Leber Hereditary optic Neuropathy (LHON), Mitochondrial Myopathy, Encephalopathy, Lactic Acidosis and Stroke Syndrome (MELAS), Myoclonic Epilepsy associated with Ragged-Red fibers (MERRF), Progressive External Ophthalmoplegia (PEO), Pearson's syndrome, Maternally inherited Myopathy and cardiomyopathy (MIMyCA), Kjer's optic neuropathy, Neuropathy ataxia and retinitis pigmentosa (NARP), Autosomal dominant optic atrophy, Kearns-Sayre syndrome, Friedreich's ataxia, Hereditary Spastic paraplegia, Charcot-Marie-Tooth disease, sporadic age-related neurodegenerative disease, Parkinson's Disease (PD), Alzheimer's Disease (AD), Age-related Macular degeneration (AMD), Diabetic Retinopathy, Glaucoma, familiar neurodegenerative disease, familiar PD, familiar AD, Huntington's disease, Retinitis Pigmentosa (RP), and Stargardt's disease, wherein the vector comprises at least one inhibitor of miR-181 as defined in any one of claims 1 to 6 and a promoter sequence, preferably the vector is a viral vector, preferably an adeno-associated viral vector.

10. The vector for use according to claim 9 having SEQ. ID. No. 14.

## Patentansprüche

1. Inhibitor von miR-181 zur Verwendung bei der Behandlung und/oder Prävention einer mitochondrialen Störung ausgewählt aus der Gruppe bestehend aus: Leigh-Syndrom, hereditärer Leber-Optikusneuropathie (LHON), mitochondrialer Myopathie, Enzephalopathie, Milchsäure- und Schlaganfallsyndrom (MELAS), myoklonischer Epilepsie assoziiert mit Ragged-Red-Fasern (MERRF), progressiver externer Ophthalmoplegie (PEO), Pearson-Syndrom, mütterlich vererbter Myopathie und Kardiomyopathie (MIMyCA), Kjer-Optikusneuropathie, Neuropathie-Ataxie und Retinitis pigmentosa (NARP), autosomal-dominanter Optikusatrophie, Kearns-Sayre-Syndrom, Friedreich-Ataxie, hereditärer spastischer Paraplegie, Charcot-Marie-Tooth-Erkrankung, sporadischer altersbedingter neurodegenerativer Erkrankung, Parkinson-Erkrankung (PD), Alzheimer-Erkrankung (AD), altersbedingter Makuladegeneration (AMD), diabetischer Retinopathie, Glaukom, familiärer neurodegenerativer Erkrankung, familiärer PD, familiärer AD, Huntington-Erkrankung, Retinitis pigmentosa (RP) und Stargardt-Erkrankung, wobei der Inhibitor ein inhibitorisches Nukleinsäuremittel ist, das zumindest eine Sequenz komplementär zu miR-181 umfasst, wobei der Inhibitor eine Sequenz komplementär zu ACAUUCA (SEQ ID NO. 5) umfasst.

2. Inhibitor von miR-181 zur Verwendung nach Anspruch 1, wobei der miR-181 ein miR-181-Vorläufer oder ein reifer miR-181 ist, wobei bevorzugt der miR-181 ausgewählt ist aus der Gruppe bestehend aus miR-181a-1, miR-181a-2, miR-181b-1, miR-181b-2, miR-181c und miR-181d oder einer Kombination davon.

3. Inhibitor von miR-181 zur Verwendung nach Anspruch 1 oder 2, wobei der Inhibitor eine Sequenz komplementär zu ACAUUCA (SEQ ID NO. 5) umfasst und zumindest 80 % Sequenzidentität mit einer Sequenz komplementär zu SEQ ID No. 1 oder SEQ ID No. 2 oder SEQ ID No. 3 oder SEQ ID No. 4 aufweist.

4. Inhibitor von miR-181 zur Verwendung nach einem der vorhergehenden Ansprüche, wobei der Inhibitor 18 bis 21 Nukleotide lang ist.

5. Inhibitor von miR-181 zur Verwendung nach einem der vorhergehenden Ansprüche, wobei die Sequenz ferner ein oder mehrere gesperrte Nukleinsäure(LNA-)Nukleotide und ein oder mehrere nicht gesperrte Nukleotide umfasst, wobei zumindest eines der nicht gesperrten Nukleotide eine chemische Modifikation umfasst, bevorzugt die gesperrten Nukleinsäure(LNA-)Nukleotide eine 2'- bis 4'-Methylenbrücke aufweisen, bevorzugt die chemische Modifikation eine 2'-O-Alkyl- oder 2'-Halo-Modifikation ist.

6. Inhibitor von miR-181 zur Verwendung nach einem der vorhergehenden Ansprüche, wobei der Inhibitor aus einer Sequenz besteht, ausgewählt aus: SEQ ID No. 6, SEQ ID No. 7, SEQ ID No. 8, SEQ ID No. 9, SEQ ID No. 10, SEQ ID No. 11, SEQ ID No. 12 oder SEQ ID No. 13.

7. Inhibitor von miR-181 zur Verwendung nach einem der vorhergehenden Ansprüche, wobei der Inhibitor von miR-181 mit zumindest einem zusätzlichen Mittel verwendet wird, bevorzugt das zumindest eine zusätzliche Mittel ein Inhibitor von miR-181 wie in einem der Ansprüche 1 bis 6 definiert ist, bevorzugt das zumindest eine zusätzliche Mittel ausgewählt ist aus der Gruppe bestehend aus: einem Mittel, das mitochondriale Biogenese erhöht, einem Mittel, das Autophagie moduliert, einem Mittel, das Apoptose inhibiert, einem ROS-Fänger, einem Mittel, das Heteroplasmie verschiebt, einem Fänger von toxischen Verbindungen, einer Ergänzung von Nukleotiden, einem Mittel, welches das fehlende Gen ersetzt, wobei bevorzugt der Inhibitor von miR-181 und das zumindest eine zusätzliche Mittel sequentiell oder gleichzeitig verabreicht werden.

8. Pharmazeutische Zusammensetzung, umfassend zumindest einen Inhibitor von miR-181 wie in einem der vorhergehenden Ansprüche definiert und einen pharmazeutisch annehmbaren Träger oder ein pharmazeutisch annehmbares Verdünnungsmittel zur Verwendung bei der Behandlung und/oder Prävention einer mitochondrialen Störung ausgewählt aus der Gruppe bestehend aus: Leigh-Syndrom, hereditärer Leber-Optikusneuropathie (LHON), mitochondrialer Myopathie, Enzephalopathie, Milchsäure- und Schlaganfallsyndrom (MELAS), myoklonischer Epilepsie assoziiert mit Ragged-Red-Fasern (MERRF), progressiver externer Ophthalmoplegie (PEO), Pearson-Syndrom, mütterlich vererbter Myopathie und Kardiomyopathie (MIMyCA), Kjer-Optikusneuropathie, Neuropathie-Ataxie und Retinitis pigmentosa (NARP), autosomal-dominanter Optikusatrophie, Kearns-Sayre-Syndrom, Friedreich-Ataxie, hereditärer spastischer Paraplegie, Charcot-Marie-Tooth-Erkrankung, sporadischer altersbedingter neurodegenerativer Erkrankung, Parkinson-Erkrankung (PD), Alzheimer-Erkrankung (AD), altersbedingter Makuladegeneration (AMD), diabetischer Retinopathie, Glaukom, familiärer neurodegenerativer Erkrankung, familiärer PD, familiärer AD, Huntington-Erkrankung, Retinitis pigmentosa (RP) und Stargardt-Erkrankung, wobei bevorzugt die Zusammensetzung ferner zumindest ein zusätzliches Mittel umfasst, bevorzugt das zumindest eine zusätzliche Mittel ein Inhibitor von miR-181 wie in einem der Ansprüche 1 bis 6 definiert ist, bevorzugt das zumindest eine zusätzliche Mittel ausgewählt ist aus der Gruppe bestehend aus: einem Mittel, das mitochondriale Biogenese erhöht, einem Mittel, das Autophagie moduliert, einem Mittel, das Apoptose inhibiert, einem ROS-Fänger, einem Mittel, das Heteroplasmie verschiebt, einem Fänger von toxischen Verbindungen, einer Ergänzung von Nukleotiden, einem Mittel, welches das fehlende Gen ersetzt.

9. Vektor zur Verwendung bei der Behandlung und/oder Prävention einer mitochondrialen Störung ausgewählt aus der Gruppe bestehend aus: Leigh-Syndrom, hereditärer Leber-Optikusneuropathie (LHON), mitochondrialer Myopathie, Enzephalopathie, Milchsäure- und Schlaganfallsyndrom (MELAS), myoklonischer Epilepsie assoziiert mit Ragged-Red-Fasern (MERRF), progressiver externer Ophthalmoplegie (PEO), Pearson-Syndrom, mütterlich vererbter Myopathie und Kardiomyopathie (MIMyCA), Kjer-Optikusneuropathie, Neuropathie-Ataxie und Retinitis pigmentosa (NARP), autosomal-dominanter Optikusatrophie, Kearns-Sayre-Syndrom, Friedreich-Ataxie, hereditärer spastischer Paraplegie, Charcot-Marie-Tooth-Erkrankung, sporadischer altersbedingter neurodegenerativer Erkrankung, Parkinson-Erkrankung (PD), Alzheimer-Erkrankung (AD), altersbedingter Makuladegeneration (AMD), diabetischer Retinopathie, Glaukom, familiärer neurodegenerativer Erkrankung, familiärer PD, familiärer AD, Huntington-Erkrankung, Retinitis pigmentosa (RP) und Stargardt-Erkrankung, wobei der Vektor zumindest einen Inhibitor von miR-181 wie in einem der Ansprüche 1 bis 6 und eine Promotorsequenz umfasst, bevorzugt der Vektor ein viraler Vektor ist, bevorzugt ein adenoassoziierter viraler Vektor.

10. Vektor zur Verwendung nach Anspruch 9 mit SEQ. ID. No. 14.

## Revendications

1. Inhibiteur de miR-181 destiné à être utilisé dans le traitement et/ou la prévention d'un trouble mitochondrial choisi dans le groupe constitué par : le syndrome de Leigh, la neuropathie optique héréditaire de Leber (NOHL), le syndrome de myopathie mitochondriale, d'encéphalopathie, d'acidose lactique et d'accident vasculaire cérébral (MELAS), l'épilepsie myoclonique associée aux fibres rouges déchiquetées (MERRF), l'ophtalmoplégie externe progressive (PEO), le syndrome de Pearson, la myopathie et cardiomyopathie héritées de la mère (MIMyCA), la neuropathie optique de Kjer, la neuropathie, ataxie, rétinite pigmentaire (NARP), l'atrophie optique autosomique dominante, le syndrome de Kearns-Sayre, l'ataxie de Friedreich, la paraplégie spastique héréditaire, la maladie de Charcot-Marie-Tooth, les maladies neurodégénératives sporadiques liées à l'âge, la maladie de Parkinson (MP), la maladie d'Alzheimer (MA), la dégénérescence maculaire liée à l'âge (DMLA), la rétinopathie diabétique, le glaucome, les maladies neurodégénératives familiales, la MP familiale, la MA familiale, la maladie de Huntington, la rétinite Pigmentosa (RP) et la maladie de Stargardt, ledit inhibiteur étant un agent acide nucléique inhibiteur comprenant au moins une séquence complémentaire de miR-181, dans lequel ledit inhibiteur comprend une séquence complémentaire de ACAUUCA (SEQ ID N° 5).

2. Inhibiteur de miR-181 destiné à être utilisé selon la revendication 1, dans lequel ledit miR-181 est un précurseur de miR-181 ou un miR-181 mature, de préférence ledit miR-181 est choisi dans le groupe constitué par miR-181a-1, miR-181a-2, miR-181b-1, miR-181b-2, miR-181c et miR-181d ou une combinaison de ceux-ci.

3. Inhibiteur de miR-181 destiné à être utilisé selon la revendication 1 ou 2, dans lequel ledit inhibiteur comprend une séquence complémentaire d'ACAUUCA (SEQ ID N° 5) et comporte au moins 80 % d'identité de séquence avec une séquence complémentaire de SEQ ID N° 1 ou SEQ ID N° 2 ou SEQ ID N° 3 ou SEQ ID N° 4.

4. Inhibiteur de miR-181 destiné à être utilisé selon l'une quelconque des revendications précédentes, dans lequel ledit inhibiteur a une longueur de 18 à 21 nucléotides.

5. Inhibiteur de miR-181 destiné à être utilisé selon l'une quelconque des revendications précédentes, dans lequel la séquence comprend en outre un ou plusieurs nucléotides d'acide nucléique verrouillé (LNA) et un ou plusieurs nucléotides non verrouillés, dans lequel au moins l'un des nucléotides non verrouillés comprend une modification chimique, de préférence les nucléotides d'acide nucléique verrouillé (LNA) comportent un pont méthylène de 2' à 4', de préférence la modification chimique est une modification 2'-O-alkyle ou 2'-halogéno.

6. Inhibiteur de miR-181 destiné à être utilisé selon l'une quelconque des revendications précédentes, dans lequel ledit inhibiteur est constitué d'une séquence choisie parmi : SEQ ID N° 6, SEQ ID N° 7, SEQ ID N° 8, SEQ ID N° 9, SEQ ID N° 10, SEQ ID N° 11, SEQ ID N° 12 ou SEQ ID N° 13.

7. Inhibiteur de miR-181 destiné à être utilisé selon l'une quelconque des revendications précédentes, dans lequel ledit inhibiteur de miR-181 est utilisé avec au moins un agent supplémentaire, de préférence ledit au moins un agent supplémentaire est un inhibiteur de miR-181 tel que défini dans l'une quelconque des revendications 1 à 6, de préférence ledit au moins un agent supplémentaire est choisi dans le groupe constitué par : un agent qui augmente la biogenèse mitochondriale, un agent qui module l'autophagie, un agent qui inhibe l'apoptose, un piégeur de ROS, un agent qui déplace l'hétéroplasmie, un piégeur de composés toxiques, une supplémentation en nucléotides, un agent qui remplace le gène manquant, de préférence ledit inhibiteur de miR-181 et ledit au moins un agent supplémentaire sont administrés séquentiellement ou simultanément.

8. Composition pharmaceutique comprenant au moins un inhibiteur de miR-181 tel que défini dans l'une quelconque des revendications précédentes et un support ou diluant acceptable pharmaceutiquement destinée à être utilisée dans le traitement et/ou la prévention d'un trouble mitochondrial choisi dans le groupe constitué par : le syndrome de Leigh, la neuropathie optique héréditaire de Leber (NOHL), le syndrome de myopathie mitochondriale, d'encéphalopathie, d'acidose lactique et d'accident vasculaire cérébral (MELAS), l'épilepsie myoclonique associée aux fibres rouges déchiquetées (MERRF), l'ophtalmoplégie externe progressive (PEO), le syndrome de Pearson, la myopathie et cardiomyopathie héritées de la mère (MIMyCA), la neuropathie optique de Kjer, la neuropathie, ataxie, rétinite pigmentaire (NARP), l'atrophie optique autosomique dominante, le syndrome de Kearns-Sayre, l'ataxie de Friedreich, la paraplégie spastique héréditaire, la maladie de Charcot-Marie-Tooth, les maladies neurodégénératives sporadiques liées à l'âge, la maladie de Parkinson (MP), la maladie d'Alzheimer (MA), la dégénérescence maculaire liée à l'âge (DMLA), la rétinopathie diabétique, le glaucome, les maladies neurodégénératives familiales, la MP familiale, la MA familiale, la maladie de Huntington, la rétinite Pigmentosa (RP) et la maladie de Stargardt, de préférence la composition comprend en outre au moins un agent supplémentaire, de préférence ledit au moins un agent supplémentaire est un inhibiteur de miR-181 tel que défini dans l'une quelconque des revendications 1 à 6, de préférence ledit au moins un agent supplémentaire est choisi dans le groupe constitué par : un agent qui augmente la biogenèse mitochondriale, un agent qui module l'autophagie, un agent qui inhibe l'apoptose, un piégeur de ROS, un agent qui déplace l'hétéroplasmie, un piégeur de composés toxiques, une supplémentation en nucléotides, un agent qui remplace le gène manquant.

9. Vecteur destiné à être utilisé dans le traitement et/ou la prévention d'un trouble mitochondrial choisi dans le groupe constitué par : le syndrome de Leigh, la neuropathie optique héréditaire de Leber (NOHL), le syndrome de myopathie mitochondriale, d'encéphalopathie, d'acidose lactique et d'accident vasculaire cérébral (MELAS), l'épilepsie myoclonique associée aux fibres rouges déchiquetées (MERRF), l'ophtalmoplégie externe progressive (PEO), le syndrome de Pearson, la myopathie et cardiomyopathie héritées de la mère (MIMyCA), la neuropathie optique de Kjer, la neuropathie, ataxie, rétinite pigmentaire (NARP), l'atrophie optique autosomique dominante, le syndrome de Kearns-Sayre, l'ataxie de Friedreich, la paraplégie spastique héréditaire, la maladie de Charcot-Marie-Tooth, les maladies neurodégénératives sporadiques liées à l'âge, la maladie de Parkinson (MP), la maladie d'Alzheimer (MA), la dégénérescence maculaire liée à l'âge (DMLA), la rétinopathie diabétique, le glaucome, les maladies neurodégénératives familiales, la MP familiale, la MA familiale, la maladie de Huntington, la rétinite Pigmentosa (RP) et la maladie de Stargardt, dans lequel le vecteur comprend au moins un inhibiteur de miR-181 tel que défini dans l'une quelconque des revendications 1 à 6 et une séquence promotrice, de préférence le vecteur est un vecteur viral, de préférence un vecteur viral adéno-associé.

10. Vecteur destiné à être utilisé selon la revendication 9 comportant SEQ ID N° 14.
